# EUROPEAN PATENT APPLICATION

(11) **EP 4 763 946 A1**
(43) Date of publication of application: **24.06.2026**
(21) Application number: 25222368.0
(22) Date of filing: 10.12.2025
(51) Int. Cl.: C09K 11/06, C07D 307/91, H10K 50/15

(54) **LIGHT EMITTING ELEMENT, MONOAMINE COMPOUND FOR THE LIGHT EMITTING ELEMENT, AND ELECTRONIC APPARATUS INCLUDING THE LIGHT EMITTING ELEMENT**

(30) Priority: 17.12.2024 JP 2024220636
(71) Applicant: Samsung Display Co., Ltd., Yongin-si, Gyeonggi-do (KR)
(72) Inventor: SAKAMOTO, Naoya, Yokohama-shi, 220-0011 (JP)
(74) Representative: Marks & Clerk LLP

(57) **Abstract**

A light emitting element including a first electrode, a hole transport region, an emission layer, an electron transport region, and a second electrode. The hole transport region includes a monoamine compound represented by Formula 1 below. Accordingly, the light emitting element of an aspect may exhibit high luminous efficiency and long life.

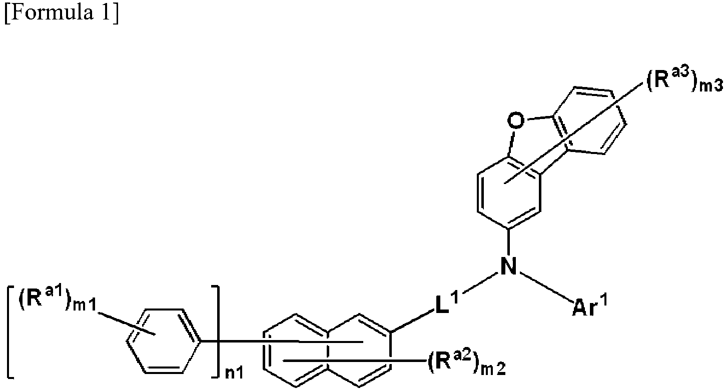

## Description

### TECHNICAL FIELD

The present disclosure herein relates to a light emitting element, a monoamine compound used in the light emitting element, and an electronic apparatus including the light emitting element.

### BACKGROUND

Electronic apparatuses provide images and include display devices. As image display devices, organic electroluminescence display devices and the like have been actively developed lately. The organic electroluminescence display devices and the like are display devices including so-called self-luminescent light emitting elements in which holes and electrons injected from a first electrode and a second electrode recombine in an emission layer, and thus a luminescent material in the emission layer emits light to accomplish display.

For application of light emitting elements to display devices, there is a demand for greater light efficiency and longer service life, and development of materials, for light emitting elements, capable of stably attaining such characteristics is being continuously required.

### SUMMARY

The present disclosure provides a light emitting element exhibiting increased luminous efficiency and lifespan, and an electronic apparatus including the same.

The present disclosure also provides a monoamine compound as a material for a light emitting element, which improves luminous efficiency and lifespan.

An aspect of the present disclosure provides a light emitting element including a first electrode, a hole transport region disposed on the first electrode and including a monoamine compound represented by Formula 1 below, an emission layer disposed on the hole transport region, an electron transport region disposed on the emission layer, and a second electrode disposed on the electron transport region.

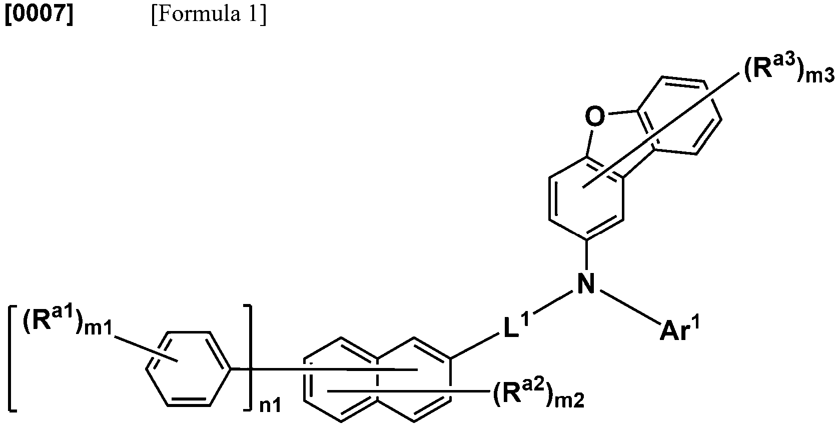

In Formula 1 above, m1 is an integer of 0 to 5, m2 is an integer of 0 to 7, m3 and n1 are each independently an integer of 0 to 7, R^{a1} to R^{a3} are each independently a hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms, L¹ is a direct linkage, a substituted or unsubstituted arylene group having 6 to 30 ring-forming carbon atoms, or a substituted or unsubstituted heteroarylene group having 5 to 30 ring-forming carbon atoms, Ar¹ is represented by any one of Formulas 2-1 to 2-4 below, and when Ar¹ is represented by Formula 2-1 or Formula 2-2, nl is an integer of 1 to 7.

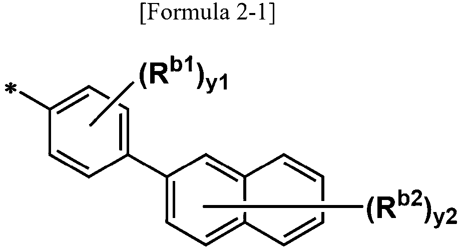

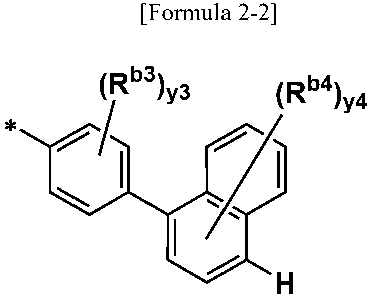

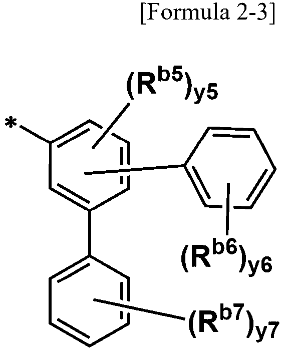

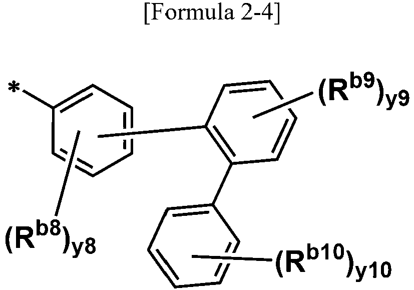

In Formulas 2-1 to 2-4 above, y1, y3, y8, and y9 are each independently an integer of 0 to 4, y2 is an integer of 0 to 7, y4 is an integer of 0 to 6, y5 is an integer of 0 to 3, y6, y7, and y10 are each independently an integer of 0 to 5, R^{b1} to R^{b10} are each independently a hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms, when nl is 0 and Ar¹ is represented by Formula 2-4, L¹ is not a substituted or unsubstituted phenanthryl group, when nl is 0 and Ar¹ is represented by Formula 2-3, y5 is 0, and any one or more hydrogen atoms in the monoamine compound is optionally substituted with a deuterium atom.

In an aspect, Formula 1 above may be represented by any one of Formulas 1-1 to 1-8 below.

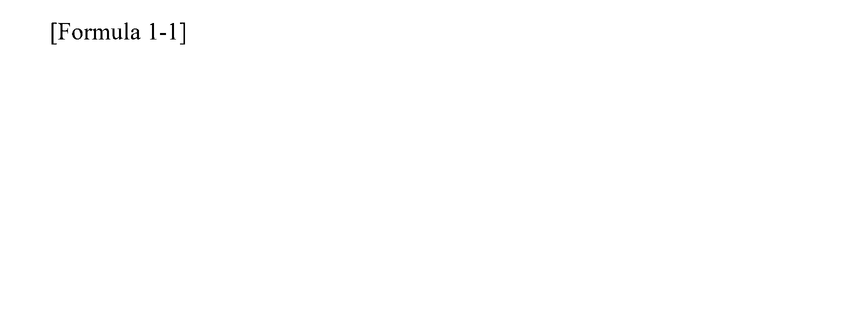

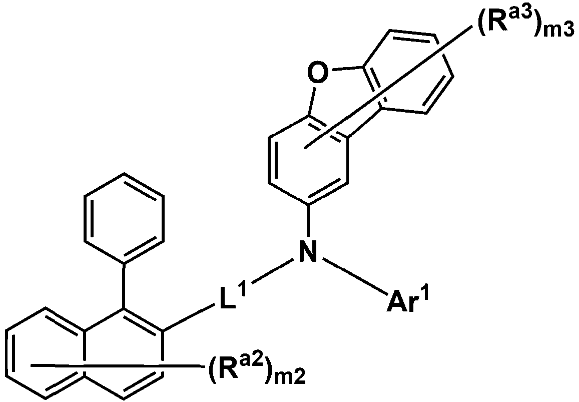

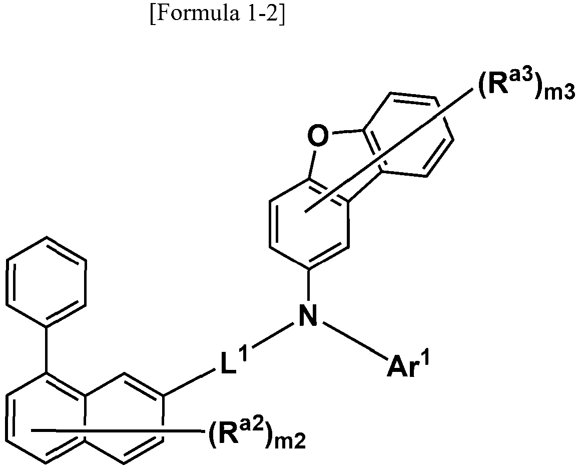

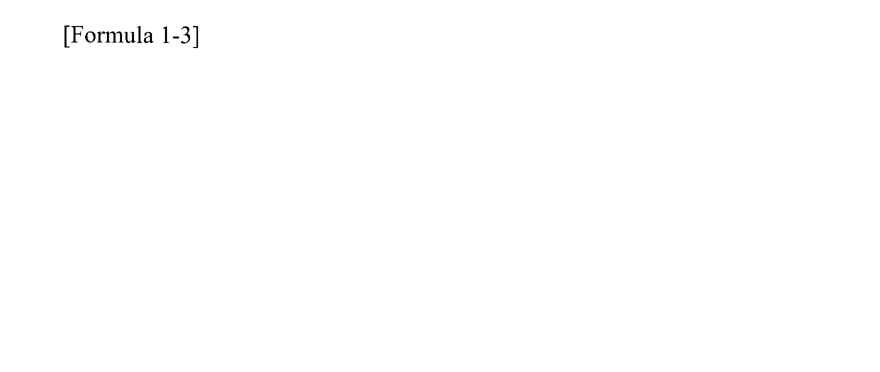

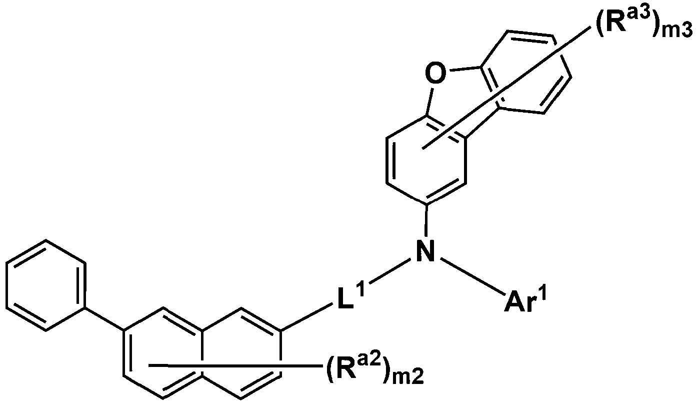

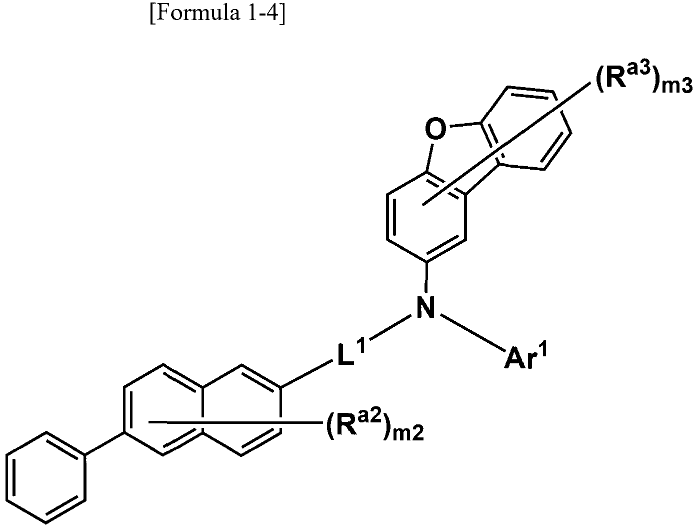

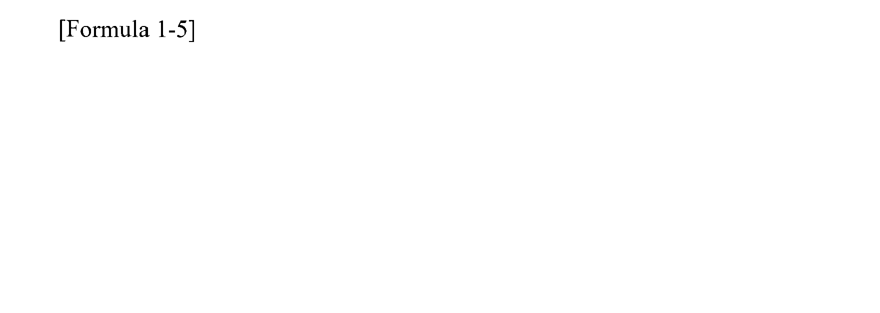

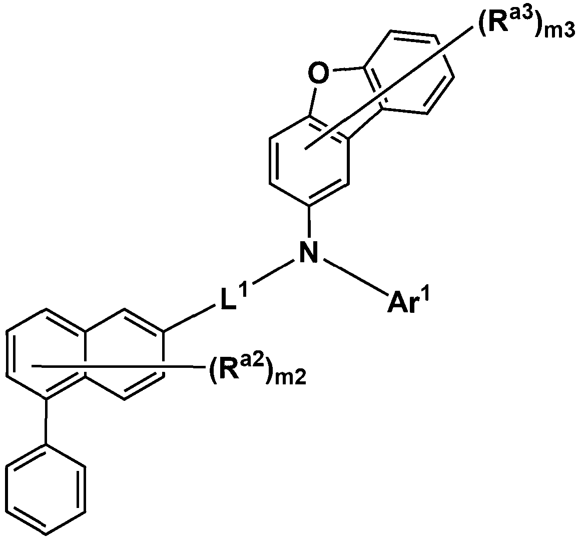

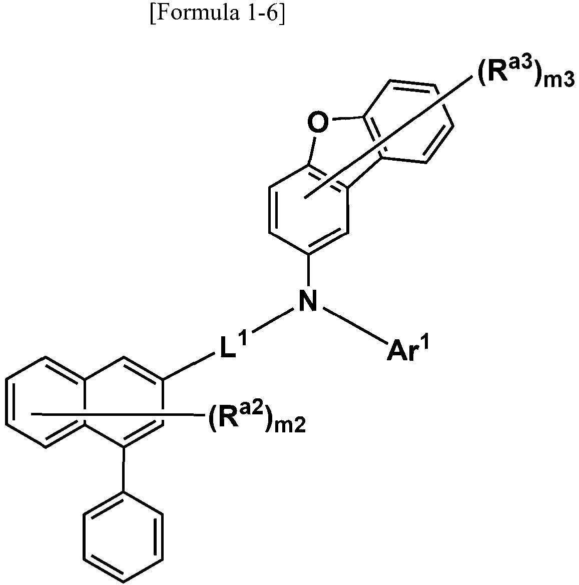

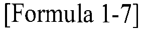

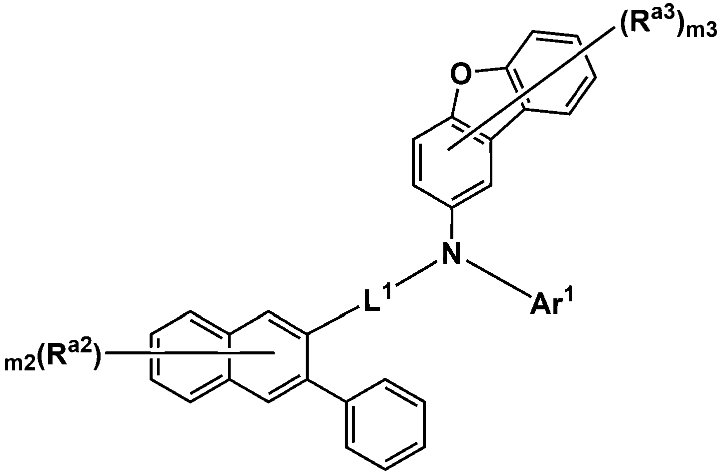

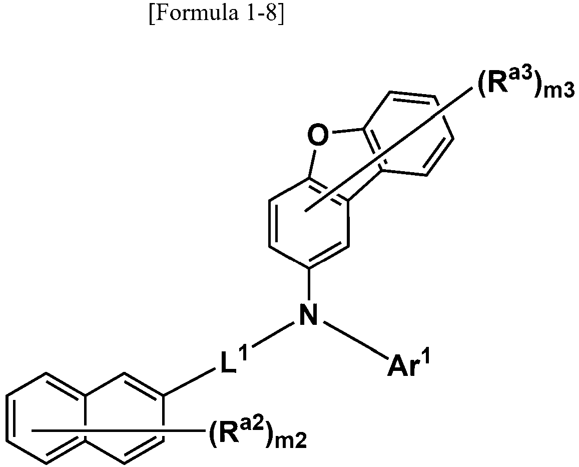

In Formulas 1-1 to 1-8 above, m2, m3, R^{a2}, R^{a3}, L¹, and Ar¹ are the same as defined in Formula 1 above.

In an aspect, Formula 2-1 above may be represented by Formula 2-1A or Formula 2-1B, and Formula 2-2 above may be represented by any one of Formulas 2-2A to 2-2C below.

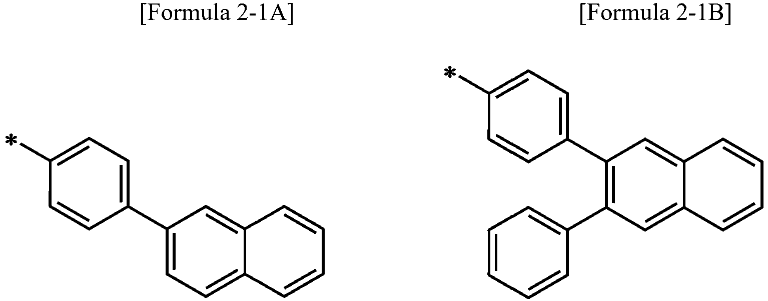

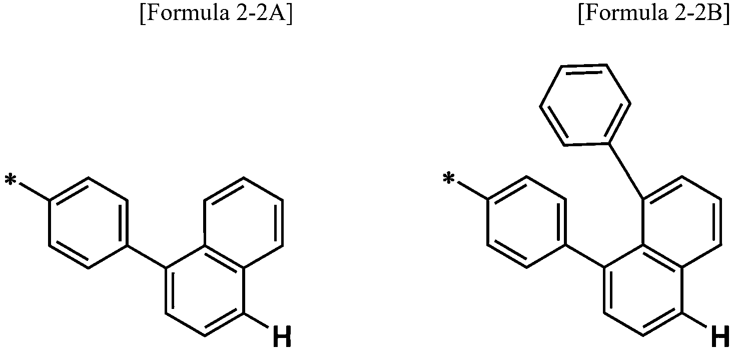

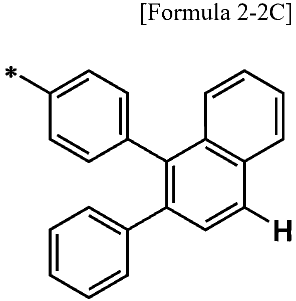

In an aspect, Formula 2-3 above may be represented by any one of Formulas 2-3A to 2-3F, and Formula 2-4 above may be represented by any one of Formulas 2-4A to 2-4D below.

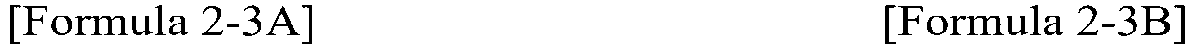

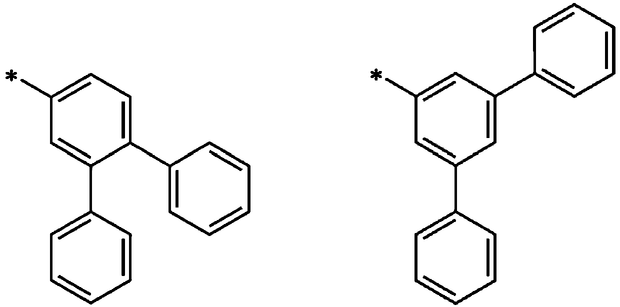

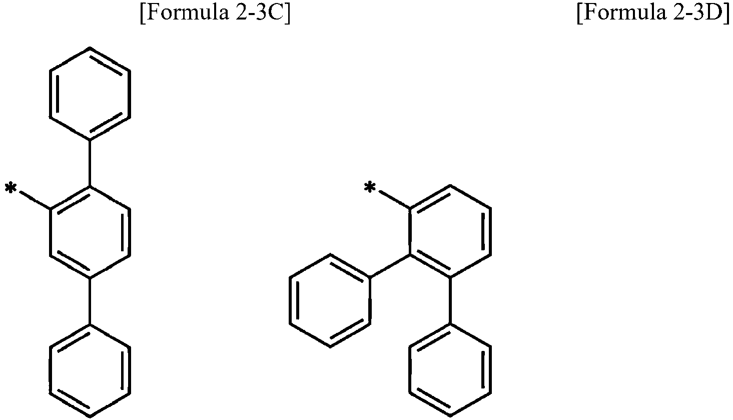

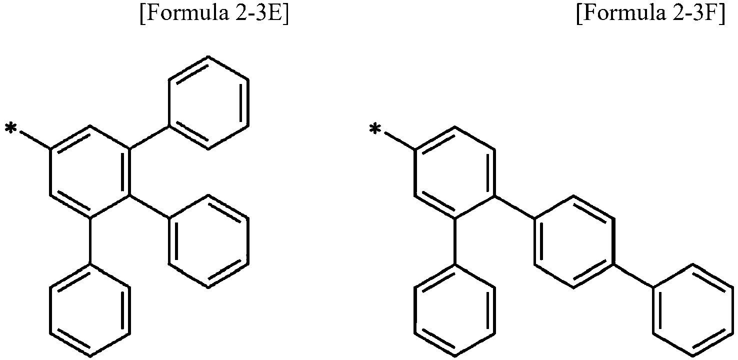

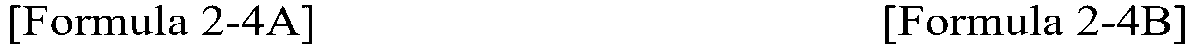

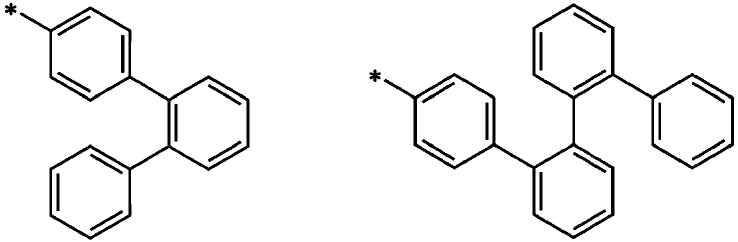

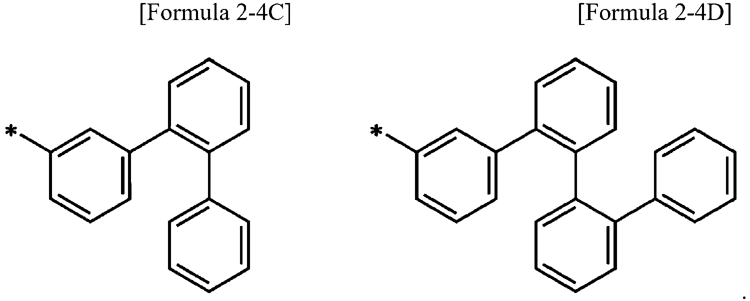

In an aspect, in Formula 1 above, L¹ may be a direct linkage or represented by any one of L1-1 to L1-5 below.

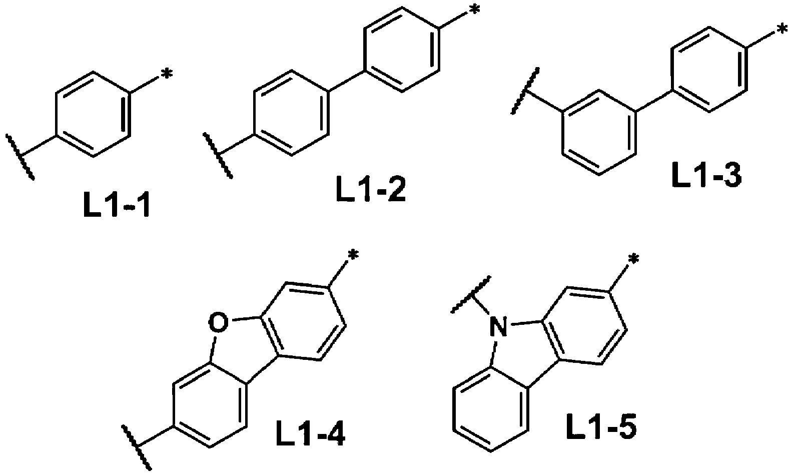

In L1-1 to L1-5 above 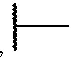 is a position bonded to a naphthalene group containing R^{a2} in Formula 1 above, and -* is a position bonded to a nitrogen atom in Formula 1 above.

In an aspect, in Formula 1 above, L¹ may be a direct linkage, a substituted or unsubstituted phenylene group, a substituted or unsubstituted biphenylene group, a substituted or unsubstituted divalent dibenzofuran group, or a substituted or unsubstituted divalent carbazole group.

In an aspect, in Formula 1 above, R^{a1} to R^{a3} may each independently be a hydrogen atom, a deuterium atom, or a substituted or unsubstituted phenyl group.

In an aspect, the hole transport region may include a hole injection layer, a hole transport layer disposed on the hole injection layer, and an electron blocking layer disposed on the hole transport layer, and at least one of the hole injection layer, the hole transport layer, or the electron blocking layer may include the monoamine compound.

In an aspect of the present disclosure, provided is a monoamine compound represented by Formula 1 above.

In an aspect of the present disclosure, an electronic apparatus provides images and includes a display device, wherein the display device includes a base layer, a circuit layer disposed on the base layer, and a display element layer disposed on the circuit layer and including a light emitting element, the light emitting element includes a first electrode, a hole transport region disposed on the first electrode and including a monoamine compound represented by Formula 1 below, an emission layer disposed on the hole transport region, an electron transport region disposed on the emission layer, and a second electrode disposed on the electron transport region.

In an aspect, the electronic apparatus may further include at least one of a light control layer or a color filter layer, wherein the light control layer may include quantum dots, and the color filter layer may include pigment or dye.

In an aspect, the display device may include at least one of a television, a monitor, an outdoor billboard, a personal computer, a laptop, a personal digital terminal, an in-vehicle display device, a game console, a portable electronic device, or a camera.

At least some of the above and other features of the invention are set out in the claims.

### BRIEF DESCRIPTION OF THE FIGURES

The accompanying drawings are included to provide a further understanding of the present disclosure, and are incorporated in and constitute a part of this specification. The drawings illustrate aspects of the present disclosure and, together with the description, serve to explain principles of the present disclosure. In the drawings:
FIG. 1 is a plan view showing a display device according to an aspect;
FIG. 2 is a cross-sectional view showing a portion corresponding to line I-I' of FIG. 1;
FIG. 3 is a cross-sectional view schematically showing a light emitting element of an aspect;
FIG. 4 is a cross-sectional view schematically showing a light emitting element of an aspect;
FIG. 5 is a cross-sectional view schematically showing a light emitting element of an aspect;
FIG. 6 is a cross-sectional view schematically showing a light emitting element of an aspect;
FIG. 7 is a cross-sectional view showing a display device according to an aspect;
FIG. 8 is a cross-sectional view showing a display device according to an aspect;
FIG. 9 is a cross-sectional view showing a display device according to an aspect;
FIG. 10 is a cross-sectional view showing a display device according to an aspect;
FIG. 11 is a view showing the inside of a vehicle in which a display device of an aspect is disposed;
FIG. 12 is a perspective view showing an electronic apparatus of an aspect;
FIG. 13 is an exploded perspective view showing an electronic apparatus of an aspect;
FIG. 14 is a block diagram of an electronic apparatus according to an aspect; and
FIG. 15 is a view showing an electronic apparatus according to various aspects.

### DETAILED DESCRIPTION

The present disclosure may be modified in many alternate forms, and thus specific aspects will be exemplified in the drawings and described in detail. It should be understood, however, that it is not intended to limit the present disclosure to the particular forms disclosed, but rather, is intended to cover all modifications, equivalents, and alternatives falling within the scope of the present disclosure.

In this specification, it will be understood that when an element (or a region, a layer, a portion, or the like) is referred to as being "on", "connected to" or "coupled to" another element, it may be directly disposed on, connected to, or coupled to the other element, or other elements may be disposed therebetween.

Like reference numerals or symbols refer to like elements throughout. In the drawings, the thickness, ratio, and size of the elements are exaggerated for effectively describing the technical contents. The term "and/or" includes any and all combinations of one or more of the associated listed elements.

It will be understood that, although the terms "first", "second", etc. may be used herein to describe various elements, the elements are not to be limited by these terms. These terms are only used to distinguish one element from another element. For instance, a first element could be termed a second element without departing from the scope of the present disclosure. Similarly, a second element could be termed a first element. The singular expressions "a", "an" and "the" are intended to include the plural forms as well, unless the context clearly indicates otherwise.

In addition, the terms "below", "under", "on the lower side", "above", "over", "on the upper side", or the like may be used to describe the relationships between the elements illustrated in the drawings. These terms are relative concepts and are described on the basis of the directions indicated in the drawings.

It will be further understood that the terms "comprises", "includes", "has" and/or "comprising", "including", "having", when used in this specification, specify the presence of stated features, numbers, steps, operations, elements, components or combinations thereof, but do not preclude the possibility of the presence or addition of one or more other features, numbers, steps, operations, elements, components, and/or combinations thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Herein, the term "substituted or unsubstituted" may indicate that one is substituted or unsubstituted with at least one substituent selected from the group consisting of a deuterium atom, a halogen atom, a cyano group, a nitro group, an amine group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In addition, each of the substituents presented as an example above may be substituted or unsubstituted. For example, a biphenyl group may be interpreted as an aryl group or as a phenyl group substituted with a phenyl group.

Herein, the term "bonded to an adjacent group to form a ring" may indicate that one is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocyclic or polycyclic. In addition, the rings formed by being bonded to each other may be linked to another ring to form a spiro structure.

Herein, the term "adjacent group" may indicate a substituent substituted for an atom which is directly linked to an atom substituted with a corresponding substituent, another substituent substituted for an atom which is substituted with a corresponding substituent, or a substituent sterically closest to a corresponding substituent. For example, two methyl groups in 1,2-dimethylbenzene may be interpreted as mutually "adjacent groups" and two ethyl groups in 1,1-diethylcyclopentane may be interpreted as mutually "adjacent groups". In addition, two methyl groups in 4,5-dimethylphenanthrene may be interpreted as mutually "adjacent groups".

Herein, examples of a halogen atom may include a fluorine atom, a chlorine atom, a bromine atom, or an iodine atom.

Herein, an alkyl group may be linear or branched. The number of carbon atoms in the alkyl group is 1 to 60, 1 to 50, 1 to 30, 1 to 20, 1 to 10, or 1 to 6. Examples of the alkyl group may include a methyl group, an ethyl group, an n-propyl group, an isopropyl group, an n-butyl group, a s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-icosyl group, a 2-ethylicosyl group, a 2-butylicosyl group, a 2-hexylicosyl group, a 2-octylicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, and the like, but are not limited thereto.

Herein, a cycloalkyl group may indicate a cyclic alkyl group. The number of carbon atoms in the cycloalkyl group is 3 to 60, 3 to 50, 3 to 30, 3 to 20, or 3 to 10. Examples of the cycloalkyl group include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, and the like, but are not limited thereto.

Herein, an alkenyl group indicates a hydrocarbon group including at least one carbon double bond in the middle or end of an alkyl group having 2 or more carbon atoms. The alkenyl group may be linear or branched. The number of carbon atoms is not particularly limited, but is 2 to 60, 2 to 30, 2 to 20, or 2 to 10. Examples of the alkenyl group include a vinyl group, a 1-butenyl group, a 1-pentenyl group, a 1,3-butadienyl group, a styrenyl group, a styryl vinyl group, and the like, but are not limited thereto.

Herein, an alkynyl group indicates a hydrocarbon group including at least one carbon triple bond in the middle or end of an alkyl group having 2 or more carbon atoms. The alkynyl group may be linear or branched. The number of carbon atoms is not particularly limited, but is 2 to 30, 2 to 20, or 2 to 10. Specific examples of the alkynyl group may include an ethynyl group, a propynyl group, and the like, but are not limited thereto.

Herein, a hydrocarbon ring group indicates any functional group or substituent derived from an aliphatic hydrocarbon ring or an aromatic hydrocarbon ring. The hydrocarbon ring group may be a saturated hydrocarbon ring group having 6 to 60, 6 to 30, 5 to 30, or 5 to 20 ring-forming carbon atoms.

Herein, an aryl group indicates any functional group or substituent derived from an aromatic hydrocarbon ring. The aryl group may be a monocyclic aryl group or a polycyclic aryl group. The number of ring-forming carbon atoms in the aryl group may be 6 to 60, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, and the like, but are not limited thereto.

Herein, a fluorenyl group may be substituted, and two substituents may be bonded to each other to form a spiro structure. An example that the fluorenyl group is substituted is as follows. However, an aspect is not limited thereto.

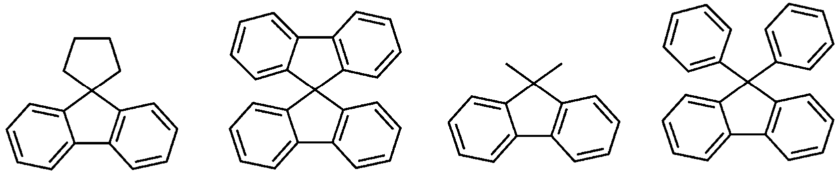

Herein, a heterocyclic group indicates any functional group or substituent derived from a ring containing at least one of B, O, N, P, Si, or S as a hetero atom. The heterocyclic group includes an aliphatic heterocyclic group and an aromatic heterocyclic group. The aromatic heterocyclic group may be a heteroaryl group. The aliphatic heterocyclic group and the aromatic heterocyclic group may be monocyclic or polycyclic.

Herein, the heterocyclic group may contain at least one of B, O, N, P, Si, or S as a hetero atom. When the heterocyclic group contains two or more hetero atoms, the two or more hetero atoms may be the same as or different from each other. The heterocyclic group may be a monocyclic heterocyclic group or a polycyclic heterocyclic group, and include a heteroaryl group. The number of ring-forming carbon atoms in the heterocyclic group may be 2 to 60, 2 to 30, 5 to 30, 2 to 20, or 2 to 10.

Herein, the aliphatic heterocyclic group may contain at least one of B, O, N, P, Si, or S as a hetero atom. The number of ring-forming carbon atoms in the aliphatic heterocyclic group may be 2 to 60, 2 to 30, 2 to 20, or 2 to 10. Examples of the aliphatic heterocyclic group include an oxirane group, a thiirane group, a pyrrolidine group, a piperidine group, a tetrahydrofuran group, a tetrahydrothiophene group, a thiane group, a tetrahydropyran group, a 1,4-dioxane group, and the like, but are not limited to thereto.

Herein, a heteroaryl group may contain at least one of B, O, N, P, Si, or S as a hetero atom. When the heteroaryl group contains two or more hetero atoms, the two or more hetero atoms may be the same as or different from each other. The heteroaryl group may be a monocyclic heteroaryl group or a polycyclic heteroaryl group. The number of ring-forming carbon atoms in the heteroaryl group may be 2 to 60, 5 to 30, 2 to 30, 2 to 20, or 2 to 10. Examples of the heteroaryl group include a thiophene group, a furan group, a pyrrole group, an imidazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzoimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, and the like, but are not limited thereto.

Herein, the above description of the aryl group may also apply to an arylene group, except that the arylene group is a divalent group. The above description of the heteroaryl group may also apply to a heteroarylene group, except that the heteroarylene group is a divalent group.

Herein, a silyl group includes an alkyl silyl group and an aryl silyl group. Examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

Herein, the number of carbon atoms in a carbonyl group is not particularly limited, but may be 1 to 40, 1 to 30, or 1 to 20. For example, the carbonyl group may have the following structure, but is not limited thereto.

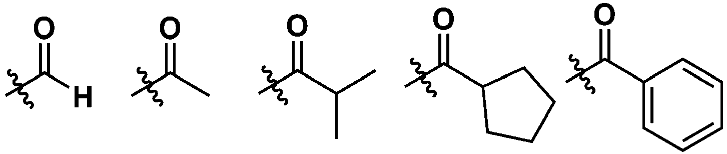

Herein, the number of carbon atoms in a sulfinyl group and a sulfonyl group is not particularly limited, but may be 1 to 30. The sulfinyl group may include an alkyl sulfinyl group and an aryl sulfinyl group. The sulfonyl group may include an alkyl sulfonyl group and an aryl sulfonyl group.

Herein, a thio group may include an alkyl thio group and an aryl thio group. The thio group may indicate the one that a sulfur atom is bonded to the alkyl group or the aryl group as defined above. Examples of the thio group include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, and the like, but are not limited to thereto.

Herein, an oxy group may indicate the one that an oxygen atom is bonded to the alkyl group or the aryl group as defined above. The oxy group may include an alkoxy group and an aryl oxy group. The alkoxy group may be linear, branched, or cyclic. The number of carbon atoms in the alkoxy group is not particularly limited, but may be, for example, 1 to 20, or 1 to 10. Examples of the oxy group include methoxy, ethoxy, n-propoxy, isopropoxy, butoxy, pentyloxy, hexyloxy, octyloxy, nonyloxy, decyloxy, benzyloxy, and the like, but are not limited thereto.

Herein, a boron group may indicate the one that a boron atom is bonded to the alkyl group or the aryl group as defined above. The boron group includes an alkyl boron group and an aryl boron group. Examples of the boron group include a dimethylboron group, a diethylboron group, a t-butylmethylboron group, a diphenylboron group, a phenylboron group, and the like, but are not limited thereto.

Herein, the number of carbon atoms in an amine group is not particularly limited, but may be 1 to 50, 1 to 30, or 1 to 20. The amine group may include an alkyl amine group and an aryl amine group. Examples of the amine group include a methylamine group, a dimethylamine group, a phenylamine group, a diphenylamine group, a naphthylamine group, a 9-methyl-anthracenylamine group, and the like, but are not limited thereto.

Herein, the above-described examples of the alkyl group also apply to an alkyl group from an alkylthio group, an alkyl sulfoxy group, an alkylaryl group, an alkylamino group, an alkyl boron group, an alkyl silyl group, and an alkyl amine group.

Herein, the above-described examples of the aryl group also apply to an aryl group from an aryloxy group, an arylthio group, an aryl sulfoxy group, an arylamino group, an aryl boron group, an aryl silyl group, and an aryl amine group.

Herein, the phosphine oxide group may be substituted with, for example, at least one of the alkyl groups or aryl groups described above.

Herein, the phosphine sulfide group may be substituted with, for example, at least one of the alkyl groups or aryl groups described above.

Herein, a direct linkage may indicate a single bond. Herein, 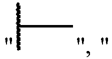 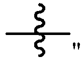 and " -*" indicate positions to be connected.

Hereinafter, aspects of the present disclosure will be described with reference to the accompanying drawings.

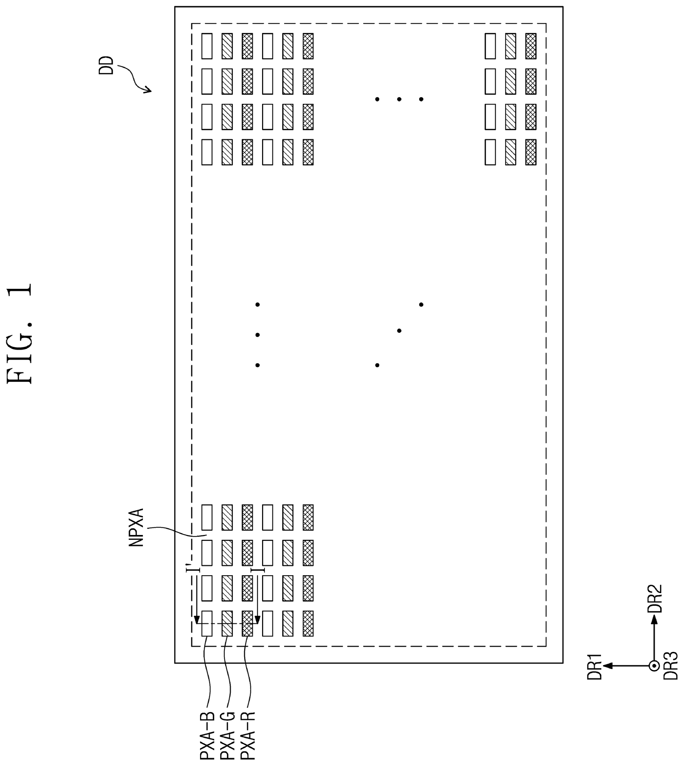

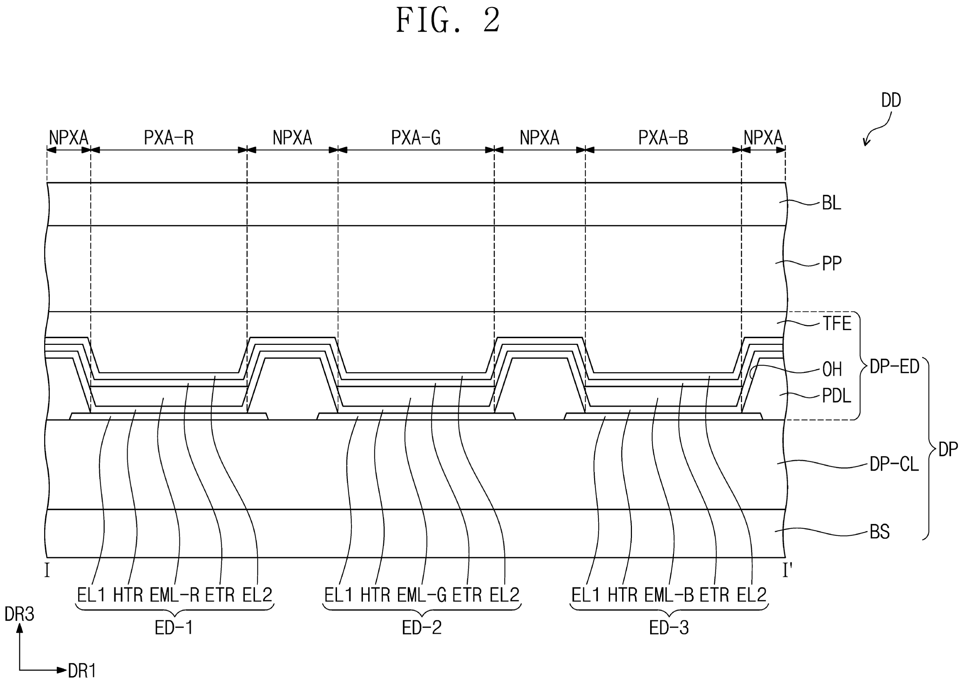

FIG. 1 is a plan view showing an aspect of a display device DD. FIG. 2 is a cross-sectional view showing a display device DD of an aspect. FIG. 2 is a cross-sectional view showing a portion corresponding to line I-I' of FIG. 1.

The display device DD may include a display panel DP and an optical layer PP disposed on the display panel DP. The display panel DP includes light emitting elements ED-1, ED-2, and ED-3. The display device DD may include a plurality of light emitting elements ED-1, ED-2, and ED-3. The optical layer PP may be disposed on the display panel DP to control reflected light in the display panel DP due to external light. The optical layer PP may include, for example, a polarizing layer or a color filter layer. Meanwhile, unlike what is shown in the drawings, the optical layer PP may not be provided in the display device DD of an aspect.

A base substrate BL may be disposed on the optical layer PP. The base substrate BL may be a member providing a base surface on which the optical layer PP is disposed. The base substrate BL may be a glass substrate, a metal substrate, a plastic substrate, and the like. However, an aspect is not limited thereto, and the base substrate BL may be an inorganic layer, an organic layer, or a composite material layer. In addition, unlike what is shown, the base substrate BL may not be provided in an aspect.

The display device DD according to an aspect may further include a filling layer (not shown). The filling layer (not shown) may be disposed between a display element layer DP-ED and the base substrate BL. The filling layer (not shown) may be an organic material layer. The filling layer (not shown) may include at least one of an acrylic resin, a silicone-based resin, or an epoxy-based resin.

The display panel DP may include a base layer BS, a circuit layer DP-CL provided on the base layer BS, and a display element layer DP-ED. The display element layer DP-ED may include pixel defining film PDL, a plurality of light emitting elements ED-1, ED-2, and ED-3 disposed between the pixel defining film PDL, and an encapsulation layer TFE disposed on the plurality of light emitting elements ED-1, ED-2, and ED-3.

The base layer BS may be a member providing a base surface on which the display element layer DP-ED is disposed. The base layer BS may be a glass substrate, a metal substrate, a plastic substrate, and the like. However, an aspect is not limited thereto, and the base layer BS may be an inorganic layer, an organic layer, or a composite material layer.

In an aspect, the circuit layer DP-CL may be disposed on the base layer BS, and the circuit layer DP-CL may include a plurality of transistors (not shown). The transistors (not shown) may each include a control electrode, an input electrode, and an output electrode. For example, the circuit layer DP-CL may include a switching transistor and a driving transistor for driving the light emitting elements ED-1, ED-2 and ED-3 of the display element layer DP-ED.

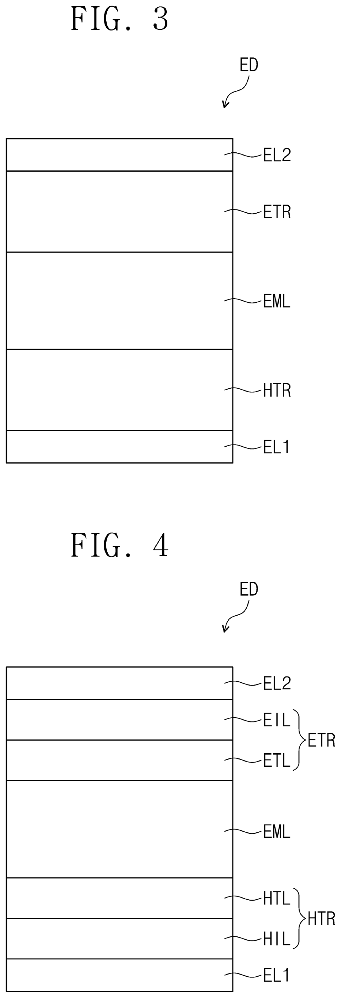

The light emitting elements ED-1, ED-2, and ED-3 may each have a structure of a light emitting element ED of an aspect from FIGS. 3 to 6, which will be described later. The light emitting elements ED-1, ED-2, and ED-3 may each include a first electrode EL1, a hole transport region HTR, emission layers EML-R, EML-G, and EML-B, an electron transport region ETR, and a second electrode EL2.

FIG. 2 shows an aspect in which the emission layers EML-R, EML-G, and EML-B of the light emitting elements ED-1, ED-2, and ED-3 are disposed in openings OH defined in the pixel defining film PDL, and the hole transport region HTR, the electron transport region ETR, and the second electrode EL2 are provided as a common layer throughout the light emitting elements ED-1, ED-2, and ED-3. However, an aspect is not limited thereto, and unlike what is shown in FIG. 2, in an aspect, the hole transport region HTR and the electron transport region ETR may be provided to be patterned inside the openings OH defined in the pixel defining film PDL. For example, in an aspect, the hole transport region HTR, the emission layers EML-R, EML-G, and EML-B, and the electron transport region ETR of the light emitting elements ED-1, ED-2, and ED-3 may be patterned and provided through an inkjet printing method.

The encapsulation layer TFE may cover the light emitting elements ED-1, ED-2 and ED-3. The encapsulation layer TFE may seal the light emitting elements ED-1 ED-2, and ED-3 of the display element layer DP-ED. The encapsulation layer TFE may be a thin film encapsulation layer. The encapsulation layer TFE may be a single layer or a stack layer of a plurality of layers. The encapsulation layer TFE includes at least one insulating layer. The encapsulation layer TFE according to an aspect may include at least one inorganic film (hereinafter, an encapsulation inorganic film). In addition, the encapsulation layer TFE according to an aspect may include at least one organic film (hereinafter, an encapsulation organic film) and at least one encapsulation inorganic film.

The encapsulation inorganic film protects the display element layer DP-ED from moisture/oxygen, and the encapsulation organic film protects the display element layer DP-ED from foreign substances such as dust particles. The encapsulation inorganic film may include silicon nitride, silicon oxy nitride, silicon oxide, titanium oxide, aluminium oxide, and the like, but is not particularly limited thereto. The encapsulation organic film may include an acrylic compound, an epoxy-based compound, and the like. The encapsulation organic film may include a photopolymerizable organic material, and is not particularly limited.

The encapsulation layer TFE may be disposed on the second electrode EL2, and may be disposed to fill the openings OH.

Referring to FIGS. 1 and 2, the display device DD may include non-light emitting regions NPXA and light emitting regions PXA-R, PXA-G, and PXA-B. The light emitting regions PXA-R, PXA-G, and PXA-B may each be a region emitting light generated from each of the light emitting elements ED-1, ED-2, and ED-3. The light emitting regions PXA-R, PXA-G, and PXA-B may be spaced apart from each other when viewed on a plane.

The light emitting regions PXA-R, PXA-G, and PXA-B may each be a region separated by the pixel defining film PDL. The non-light emitting regions NPXA may be regions between neighboring light emitting regions PXA-R, PXA-G, and PXA-B, and may correspond to the pixel defining film PDL. Meanwhile, herein, the light emitting regions PXA-R, PXA-G, and PXA-B may each correspond to a pixel. The pixel defining film PDL may separate the light emitting elements ED-1, ED-2 and ED-3. The emission layers EML-R, EML-G, and EML-B of the light emitting elements ED-1, ED-2 and ED-3 may be disposed in the openings OH defined by the pixel defining film PDL and thus be separated.

The light emitting regions PXA-R, PXA-G, and PXA-B may be divided into a plurality of groups according to the color of light generated from the light emitting elements ED-1, ED-2, and ED-3. In the display device DD of an aspect shown in FIGS. 1 and 2, three light emitting regions PXA-R, PXA-G, and PXA-B which emit red light, green light, and blue light, are shown as an example. For example, the display device DD of an aspect may include a red light emitting region PXA-R, a green light emitting region PXA-G, and a blue light emitting region PXA-B, which are distinct from one another.

In the display device DD according to an aspect, the plurality of light emitting elements ED-1, ED-2, and ED-3 may emit light having different wavelength ranges. For example, in an aspect, the display device DD may include a first light emitting element ED-1 emitting red light, a second light emitting element ED-2 emitting green light, and a third light emitting element ED-3 emitting blue light. That is, the red light emitting region PXA-R, the green light emitting region PXA-G, and the blue light emitting region PXA-B of the display device DD may correspond to the first light emitting element ED-1, the second light emitting element ED-2, and the third light emitting element ED-3, respectively.

However, an aspect is not limited thereto, and the first to third light emitting elements ED-1, ED-2 and ED-3 may emit light in the same wavelength range or emit light in at least one different wavelength range. For example, the first to third light emitting elements ED-1, ED-2, and ED-3 may all emit blue light.

The light emitting regions PXA-R, PXA-G, and PXA-B in the display device DD according to an aspect may be arranged in the form of a stripe. Referring to FIG. 1, a plurality of red light emitting regions PXA-R, a plurality of green light emitting regions PXA-G, and a plurality of blue light emitting regions PXA-B may each be arranged along a second directional axis DR2. Alternatively, the red light emitting region PXA-R, the green light emitting region PXA-G, and the blue light emitting region PXA-B may be alternately arranged in that order along a first directional axis DR1.

FIGS. 1 and 2 show that the light emitting regions PXA-R, PXA-G, and PXA-B are all similar in size, but an aspect is not limited thereto, and the light emitting regions PXA-R, PXA-G, and PXA-B may be different in size from each other according to wavelength range of emitted light. Meanwhile, the areas of the light emitting regions PXA-R, PXA-G, and PXA-B may indicate areas when viewed on a plane defined by the first directional axis DR1 and the second directional axis DR2. A third directional axis DR3 may be perpendicular to a plane defined by the first directional axis DR1 and the second directional axis DR2.

Meanwhile, the arrangement of the light emitting regions PXA-R, PXA-G, and PXA-B is not limited to what is shown in FIG. 1, and the order in which the red light emitting region PXA-R, the green light emitting region PXA-G, and the blue light emitting region PXA-B are arranged comes with varied combination according to display quality characteristics required for the display device DD. For example, the light emitting regions PXA-R, PXA-G, and PXA-B may be arranged in the form of a pentile (PENTILE^{™}) or a diamond (Diamond Pixel^{™}).

In addition, areas of each of the light emitting regions PXA-R, PXA-G, and PXA-B may be different in size from one another. For example, in an aspect, the green light emitting region PXA-G may be smaller than the blue light emitting region PXA-B in size, but an aspect is not limited thereto.

Hereinafter, FIGS. 3 to 6 are cross-sectional views schematically showing a light emitting element according to an aspect. The light emitting element ED according to an aspect may include a first electrode EL1, a hole transport region HTR, an emission layer EML, an electron transport region ETR, and a second electrode EL2, which are sequentially stacked.

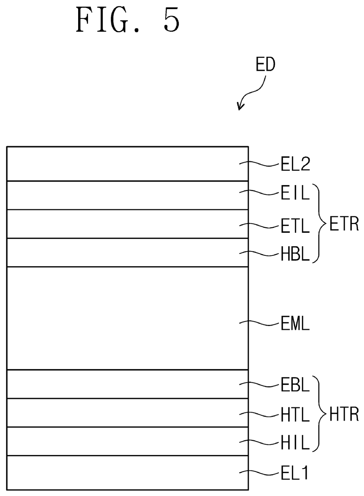

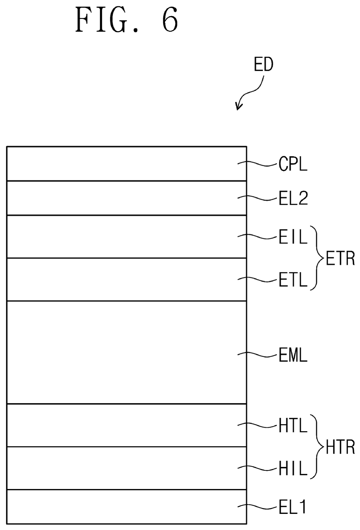

FIG. 4 shows, compared with FIG. 3, a cross-sectional view of a light emitting element ED of an aspect in which the hole transport region HTR includes a hole injection layer HIL and a hole transport layer HTL, and the electron transport region ETR includes an electron injection layer EIL and an electron transport layer ETL. In addition, FIG. 5 shows, compared with FIG. 3, a cross-sectional view of a light emitting element ED of an aspect, in which the hole transport region HTR includes a hole injection layer HIL, a hole transport layer HTL, and an electron blocking layer EBL, and the electron transport region ETR includes an electron injection layer EIL, an electron transport layer ETL, and a hole blocking layer HBL. FIG.6 shows, compared with FIG. 4, a cross-sectional view of a light emitting element ED of an aspect, in which a capping layer CPL disposed on the second electrode EL2 is provided.

The first electrode EL1 has conductivity. The first electrode EL1 may be formed of a metal material, a metal alloy, or a conductive compound. The first electrode EL1 may be an anode or a cathode. However, an aspect is not limited thereto. In addition, the first electrode EL1 may be a pixel electrode. The first electrode EL1 may be a transmissive electrode, a transflective electrode, or a reflective electrode. The first electrode EL1 may be a transmissive electrode, a transflective electrode, or a reflective electrode. The first electrode EL1 may include at least one selected from Ag, Mg, Cu, Al, Pt, Pd, Au, Ni, Nd, Ir, Cr, Li, Ca, LiF, Mo, Ti, W, In, Sn, or Zn, at least two compounds selected therefrom, two or more mixtures selected therefrom, or an oxide thereof.

When the first electrode EL1 is a transmissive electrode, the first electrode EL1 may include a transparent metal oxide such as indium tin oxide (ITO), indium zinc oxide (IZO), zinc oxide (ZnO), and indium tin zinc oxide (ITZO). When the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stack structure of LiF and Ca), LiF/Al (a stack structure of LiF and Al), Mo, Ti, W, or a compound thereof or a mixture thereof (e.g., a mixture of Ag and Mg). Alternatively, the first electrode EL1 may have a multilayer structure including a reflective film or a transflective film formed of the above-described materials, and a transparent conductive film formed of indium tin oxide (ITO), indium zinc oxide (IZO), zinc oxide (ZnO), indium tin zinc oxide (ITZO), and the like. For example, the first electrode EL1 may have a three-layer structure of ITO/Ag/ITO, but is not limited thereto. In addition, an aspect is not limited thereto, and the first electrode EL1 may include the above-described metal materials, a combination of two or more metal materials selected from the above-described metal materials, or oxides of the above-described metal materials. The first electrode EL1 may have a thickness of about 700 Å to about 10000 Å. For example, the first electrode EL1 may have a thickness of about 1000 Å to about 3000 Å.

The light emitting element ED of an aspect includes a monoamine compound of an aspect. In an aspect, the hole transport region HTR includes the monoamine compound of an aspect. At least one of the hole injection layer HIL, the hole transport layer HTL, or the electron blocking layer EBL may include the monoamine compound of an aspect. For example, the hole transport layer HTL may include a monoamine compound of an aspect.

The monoamine compound of an aspect may include only one amine group. Herein, substituents (e.g., aryl groups) bonded to amine groups are bonded to form a ring (e.g., carbazole groups), and these are not included in the amine groups, but these (e.g., carbazole groups) are included in heteroaryl groups. A ring group containing a nitrogen atom as a ring-forming atom (e.g., a pyridine group, a pyrimidine group, a triazine group, a carbazole group, and the like) is not included in an amine group and is included in a heterocyclic group.

The monoamine compound of an aspect includes first to third substituents directly or indirectly bonded to an amine group. The first substituent is a 2-dibenzofuranyl group, and the second substituent is a phenylnaphthalenyl group or an unsubstituted naphthalenyl group. The phenylnaphthalenyl group may be a naphthalenyl group substituted with a phenyl group. The third substituent is a substituted phenyl group, and the substituent of the phenyl group may be a phenyl group, a biphenyl group, a terphenyl group, and/or a naphthalenyl group. Accordingly, the monoamine compound of an aspect may exhibit excellent hole transport properties and excellent material stability, and the light emitting element ED of an aspect including the monoamine compound may exhibit low driving voltage, high luminous efficiency, and/or long life characteristics.

The light emitting element ED includes a monoamine compound of an aspect. The monoamine compound of an aspect is represented by Formula 1 below.

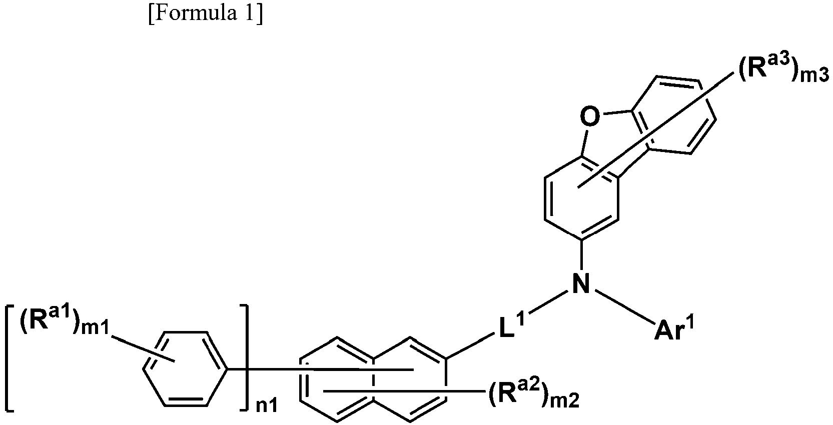

In Formula 1, the 2-dibenzofuranyl group containing R^{a3} may correspond to the first substituent described above, and Ar¹ may correspond to the third substituent described above. The naphthalenyl group containing R^{a2} may correspond to the second substituent described above.

In Formula 1, m1 is an integer of 0 to 5, m2 is an integer of 0 to 7, and m3 and nl are each independently integers of 0 to 7. R^{a1} to R^{a3} are each independently a hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms.

When m1 is an integer of 2 or greater, a plurality of R^{a1}'s may all be the same or at least one may be different from the others. When m1 is 0, the case may be the same as a case in which m1 is 5 and five R^{a1}'s are hydrogen atoms. When m2 is an integer of 2 or greater, a plurality of R^{a2}'s may all be the same or at least one may be different from the others. When m2 is 0, the case may be the same as a case in which m2 is 7 and seven R^{a2}'s are hydrogen atoms. When m3 is an integer of 2 or greater, a plurality of R^{a3}'s may all be the same or at least one may be different from the others. When m3 is 0, the case may be the same as a case in which m3 is 7 and seven R^{a3}'s are hydrogen atoms.

For example, R^{a1} to R^{a3} may each independently be a hydrogen atom, a deuterium atom, or a substituted or unsubstituted phenyl group. When R^{a3} is a phenyl group, the dibenzofuranyl group containing R^{a3} may be represented by any one of DF-1 to DF-3.

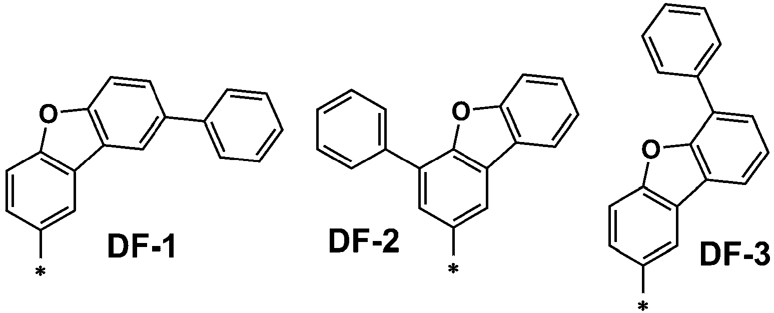

When nl is an integer of 2 or greater, a phenyl group containing R^{a1} is provided in plurality, and the plurality of phenyl groups may be the same or at least one may be different. The plurality of phenyl groups being different may indicate that R^{a1}, the substituent bonded to each of the plurality of phenyl groups, is different. The case where nl is 0 indicates that no phenyl group containing R^{a1} is present. For example, nl may be 0 or 1.

In Formula 1, L¹ is a direct linkage, a substituted or unsubstituted arylene group having 6 to 30 ring-forming carbon atoms, or a substituted or unsubstituted heteroarylene group having 5 to 30 ring-forming carbon atoms. For example, L¹ may be a direct linkage, a substituted or unsubstituted phenylene group, a substituted or unsubstituted biphenylene group, a substituted or unsubstituted divalent dibenzofuran group, or a substituted or unsubstituted divalent carbazole group.

L¹ may be a direct linkage or represented by any one of L1-1 to L1-5 below. In L1-1 to L1-5 below, 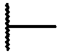 is a position bonded to a naphthalene group containing R^{a2} in Formula 1, and -* is a position bonded to a nitrogen atom in Formula 1.

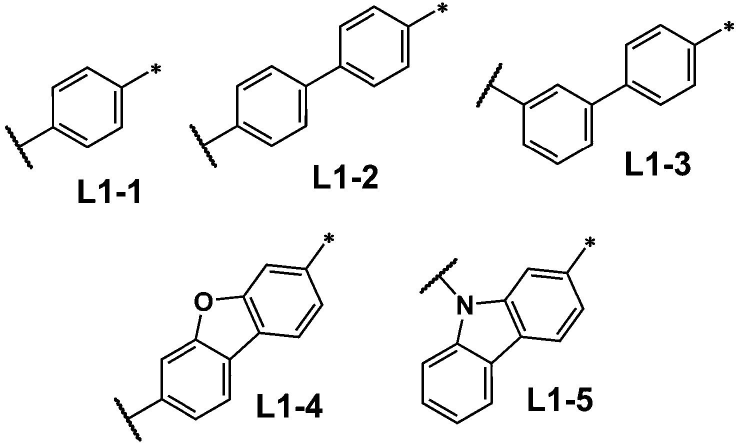

Ar¹ is represented by any one of Formulas 2-1 to 2-4 below. In Formula 2-2 below, H is a hydrogen atom. When Ar¹ is represented by Formula 2-1 or Formula 2-2, nl is an integer of 1 to 7, That is, when Ar¹ is represented by Formula 2-1 or Formula 2-2, the naphthalenyl group containing R^{a2} is substituted with a phenyl group containing R^{a1}.

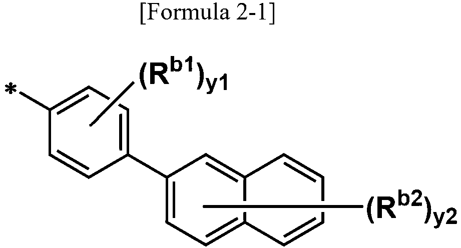

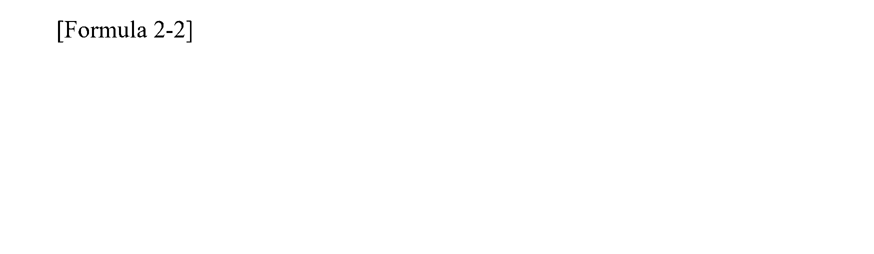

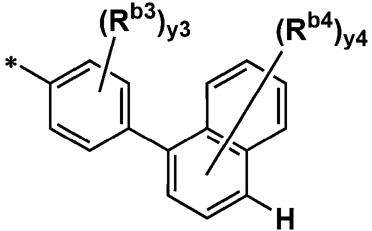

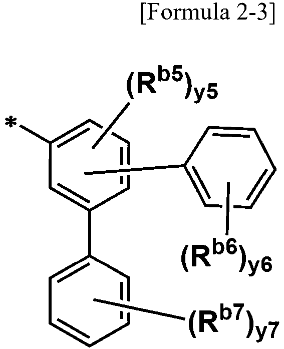

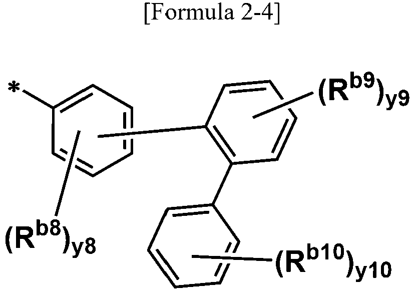

In Formulas 2-1 to 2-4, y1, y3, y8, and y9 are each independently an integer of 0 to 4, y2 is an integer of 0 to 7, and y4 is an integer of 0 to 6. y5 is an integer of 0 to 3, and y6, y7, and y10 are each independently an integer of 0 to 5. R^{b1} to R^{b10} are each independently hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms. For example, R^{b1} to R^{b10} may each independently be a hydrogen atom, a deuterium atom, or a substituted or unsubstituted phenyl group.

When y1 is an integer of 2 or greater, a plurality of R^{b1}'s may all be the same or at least one may be different from the others. The case where y1 is 0 may be the same as a case where y1 is 4 and four R^{b1}'s are hydrogen atoms. When y2 is an integer of 2 or greater, a plurality of R^{b2}'s may all be the same or at least one may be different from the others. When y2 is 0, the case may be the same as a case in which y2 is 7 and seven R^{b2}'s are hydrogen atoms.

When y3 is an integer of 2 or greater, a plurality of R^{b3}'s may all be the same or at least one may be different from the others. The case where y3 is 0 may be the same as a case where y3 is 4 and four R^{b3}'s are hydrogen atoms. When y4 is an integer of 2 or greater, a plurality of R^{b4}'s may all be the same or at least one may be different from the others. When y4 is 0, the case may be the same as a case in which y4 is 6 and six R^{b4}'s are hydrogen atoms.

When y5 is an integer of 2 or greater, a plurality of R^{b5}'s may all be the same or at least one may be different from the others. When y5 is 0, the case may be the same as a case in which y5 is 3 and three R^{b5}'s are hydrogen atoms. When y6 is an integer of 2 or greater, a plurality of R^{b6}'s may all be the same or at least one may be different from the others. The case where y6 is 0 may be the same as a case where y6 is 5 and five R^{b6}'s are hydrogen atoms.

When y7 is an integer of 2 or greater, a plurality of R^{b7}'s may all be the same or at least one may be different from the others. The case where y7 is 0 may be the same as a case where y7 is 5 and five R^{b7}'s are hydrogen atoms. When y8 is an integer of 2 or greater, a plurality of R^{b8}'s may all be the same or at least one may be different from the others. The case where y8 is 0 may be the same as a case where y8 is 4 and four R^{b8}'s are hydrogen atoms.

When y9 is an integer of 2 or greater, a plurality of R^{b9}'s may all be the same or at least one may be different from the others. The case where y9 is 0 may be the same as a case where y9 is 4 and four R^{b9}'s are hydrogen atoms. When y10 is an integer of 2 or greater, a plurality of R^{b10}'s may all be the same or at least one may be different from the others. The case where y10 is 0 may be the same as a case where y10 is 5 and five R^{b10}'s are hydrogen atoms.

In Formula 1, when nl is 0 and Ar¹ is represented by Formula 2-4, L¹ is not a substituted or unsubstituted phenanthryl group. A compound in which n1 is 0, Ar¹ is represented by Formula 2-4, and L¹ is a substituted or unsubstituted phenanthryl group has a large steric hindrance, which reduces material stability and causes an increase in deposition temperature during deposition of the compound for forming a light emitting element, resulting in thermal decomposition. In contrast, the monoamine compound of an aspect in which L¹ is not a substituted or unsubstituted phenanthryl group when n1 is 0 and Ar¹ is represented by Formula 2-4 may exhibit excellent material stability. For example, when n1 is 0 and Ar¹ is represented by Formula 2-4, L¹ may be a substituted or unsubstituted phenylene group or a substituted or unsubstituted biphenylene group.

In Formula 1, when n1 is 0 and Ar¹ is represented by Formula 2-3, y5 is 0. That is, when nl is 0 and Ar¹ is represented by Formula 2-3, the phenyl group containing R^{b5} in Formula 2-3 may not be directly substituted with any other substituent except the phenyl group containing R^{b6} and the phenyl group containing R^{b7}. The fact that the first substituent is directly substituted onto the second substituent indicates that the first substituent is directly bonded to the second substituent, and that the first substituent is not bonded to the second substituent via a linker.

In an aspect, the monoamine compound represented by Formula 1 may include a chemical structure in which any hydrogen atom is substituted with a deuterium atom. An any one or more hydrogen atoms in the monoamine compound of an aspect may be optionally substituted with a deuterium atom. For example, in Formula 1, R^{a3} may be a deuterium atom, and in this case, the monoamine compound of an aspect may include a dibenzofuranyl group substituted with a deuterium atom. In Formula 1, L¹ may be a phenylene group substituted with a deuterium atom, and in this case, the monoamine compound of an aspect may include a phenylene group substituted with a deuterium atom. Alternatively, in Formula 1, Ar¹ may be represented by Formula 2-1, and R^{b1} may be a deuterium atom in Formula 2-1, and in this case, the monoamine compound of an aspect may include a phenylene group substituted with a deuterium atom. However, this is presented as an example, and the present disclosure is not limited thereto.

In an aspect, Formula 2-1 may be represented by Formula 2-1A or Formula 2-1B below. Formula 2-1A may indicate a case where R^{b1} and R^{b2} are hydrogen atoms in Formula 2-1. Formula 2-1B may indicate a case where R^{b1} is a hydrogen atom and R^{b2} is a phenyl group in Formula 2-1.

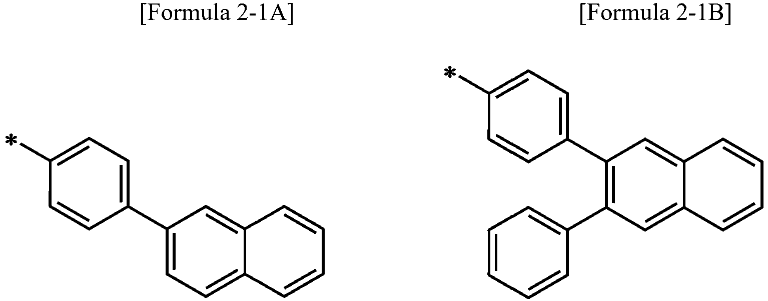

In an aspect, Formula 2-2 may be represented by any one of Formulas 2-2A to 2-2C below. Formula 2-2A may indicate a case where R^{b3} and R^{b4} are hydrogen atoms in Formula 2-2. Formula 2-2B and Formula 2-2C may each indicate a case where R^{b3} is a hydrogen atom and R^{b4} is a phenyl group in Formula 2-2.

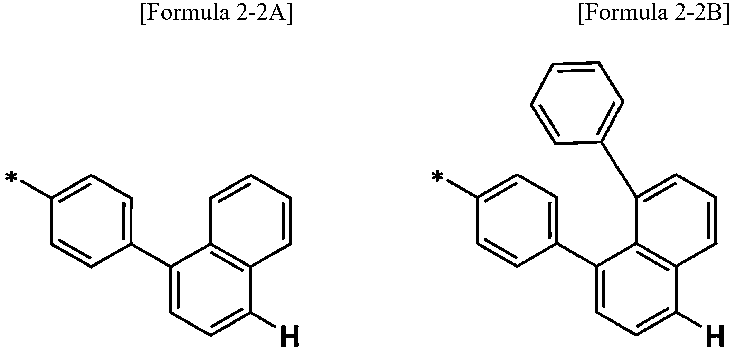

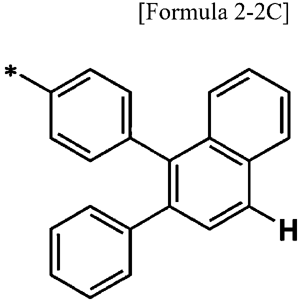

In an aspect, Formula 2-3 may be represented by any one of Formulas 2-3A to 2-3F below. Formulas 2-3A to 2-3F may specifically indicate that a position at which a phenyl group containing R^{b6} is bonded to a phenyl group containing R^{b5} in Formula 2-3 is specified. In addition, Formulas 2-3A to 2-3D may indicate a case where R^{b5} to R^{b7} are hydrogen atoms in Formula 2-3. Formula 2-3E may indicate a case where R^{b5} is a phenyl group in Formula 2-3. Formula 2-3F may indicate a case where R^{b6} is a phenyl group in Formula 2-3.

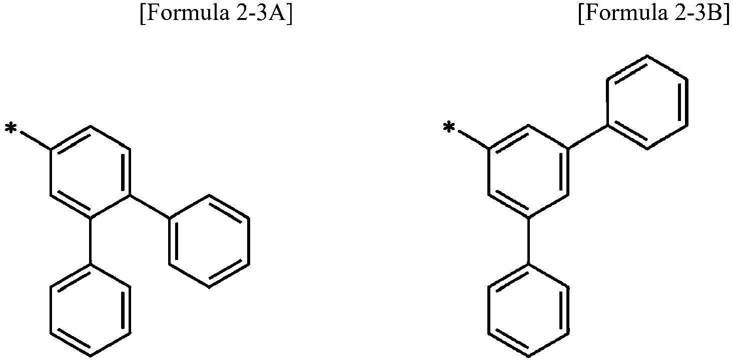

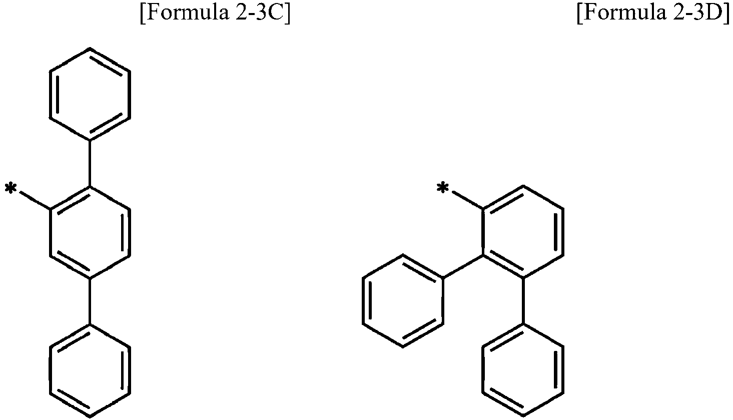

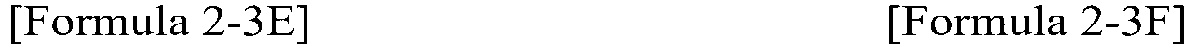

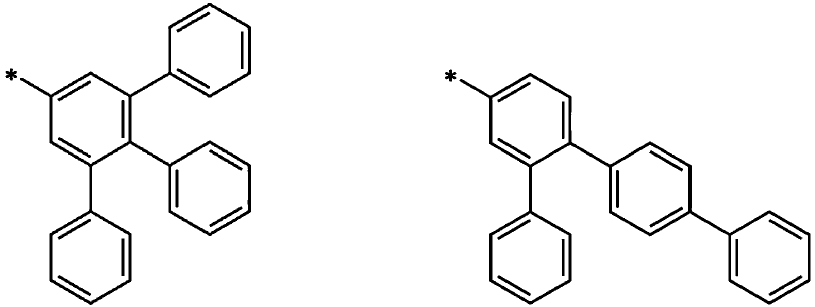

In an aspect, Formula 2-4 may be represented by any one of Formulas 2-4A to 2-4D below. Formulas 2-4A to 2-4D may specifically indicate that a position at which a phenyl group containing R^{b9} is bonded to a phenyl group containing R^{b8} in Formula 2-4 is specified. In addition, Formulas 2-4A and 2-4C may indicate a case where R^{b8} to R^{b10} are hydrogen atoms in Formula 2-4. Formulas 2-4B and 2-4D may indicate a case where R^{b8} and R^{b9} are hydrogen atoms and R^{b10} is a phenyl group in Formula 2-4.

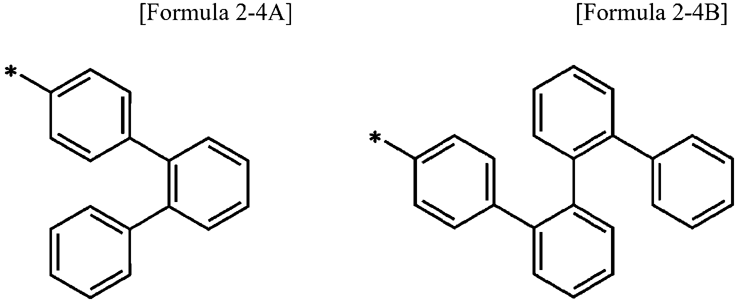

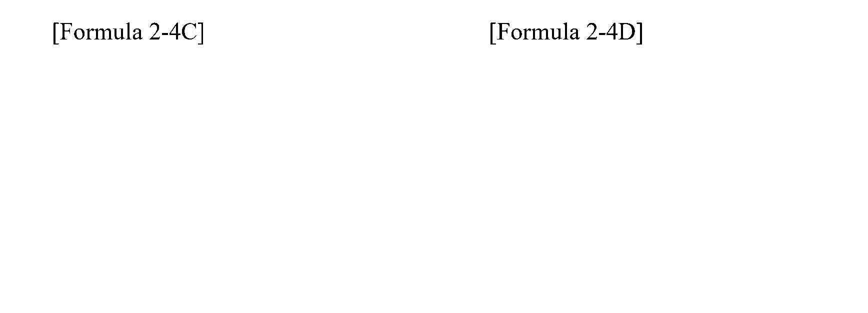

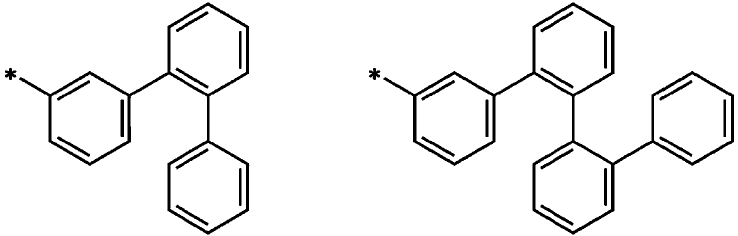

In an aspect, Formula 1 may be represented by any one of Formulas 1-1 to 1-8 below. Formulas 1-1 to 1-7 may indicate a case where nl is 1 and R^{a1} is a hydrogen atom in Formula 1. Formula 1-8 may indicate a case where n1 is 0 in Formula 1. In Formulas 1-1 to 1-8, the description of Formula 1 also applies to m2, m3, R^{a2}, R^{a3}, L¹, and Ar¹.

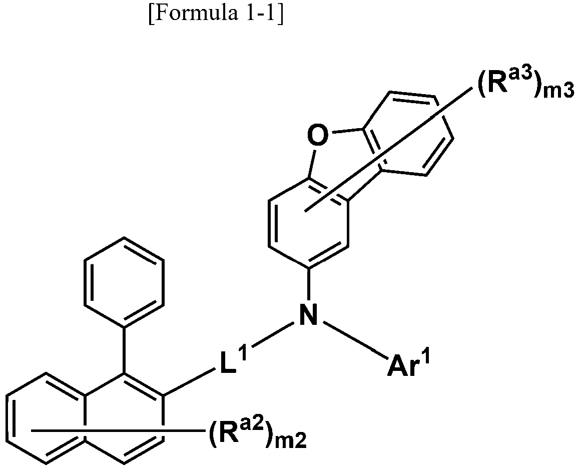

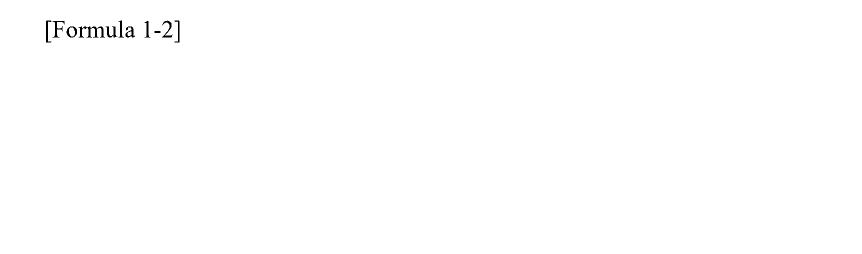

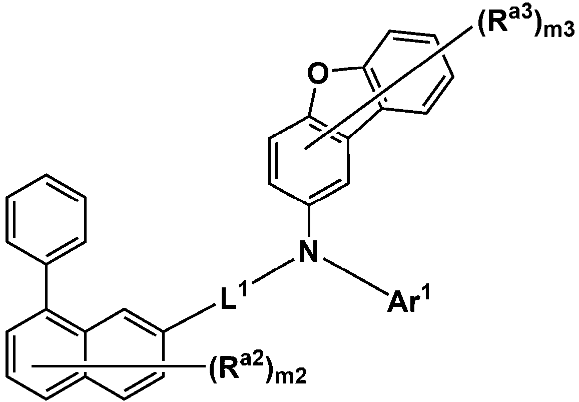

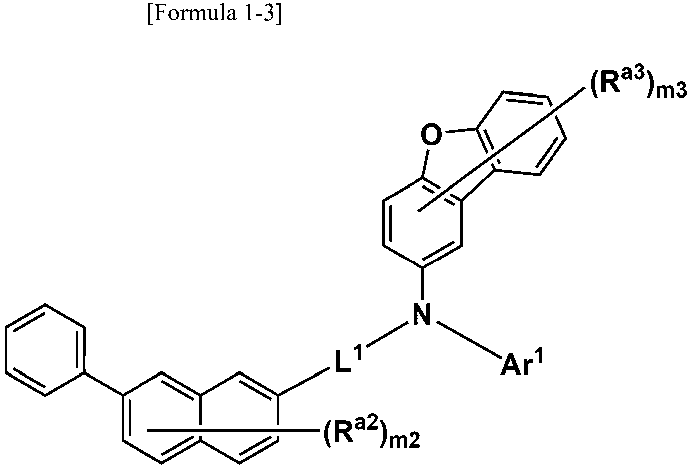

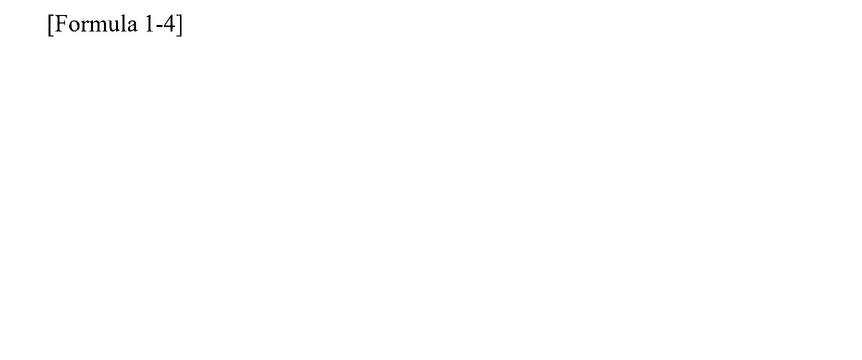

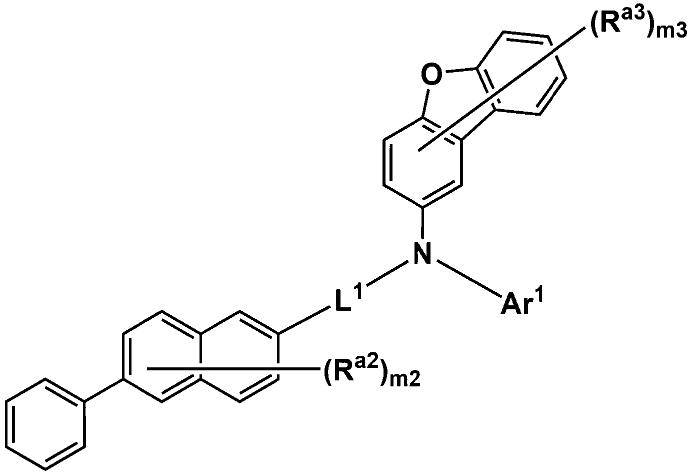

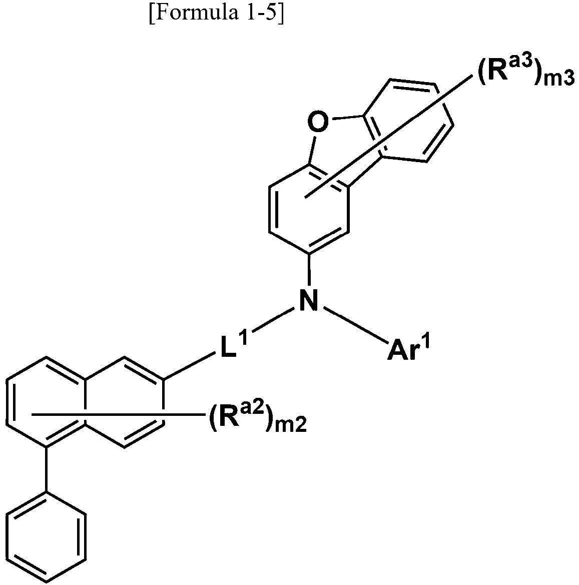

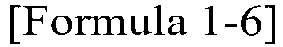

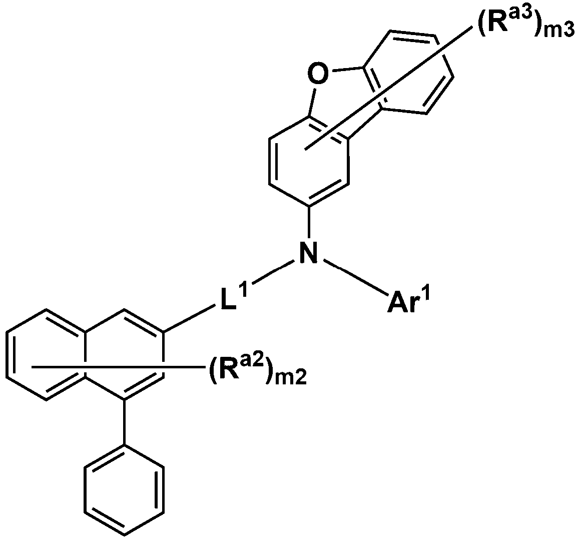

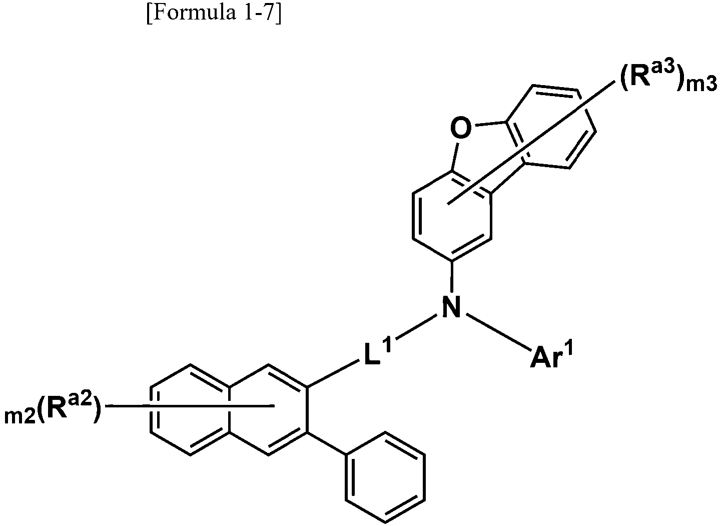

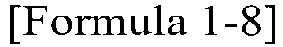

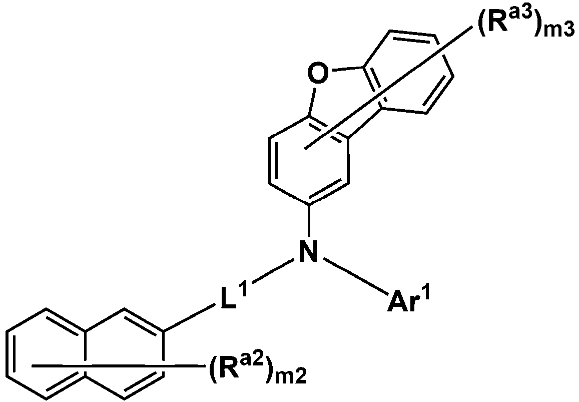

Formula 1 may be represented by any one of the compounds from Compound Group 1 below. The monoamine compound of an aspect may be represented by any one of the compounds from Compound Group 1 below. The light emitting element ED of an aspect may include at least one of the compounds from Compound Group 1 below. In Compound Group 1 below, D is a deuterium atom.

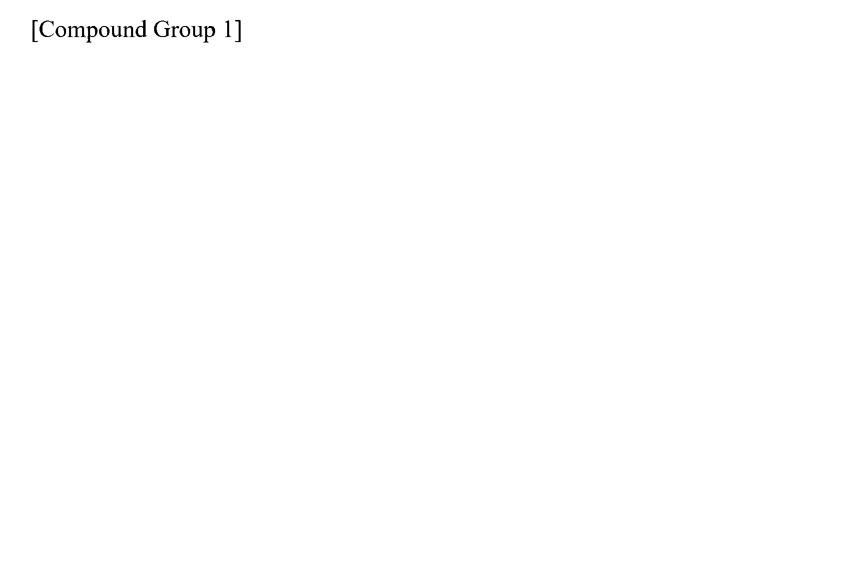

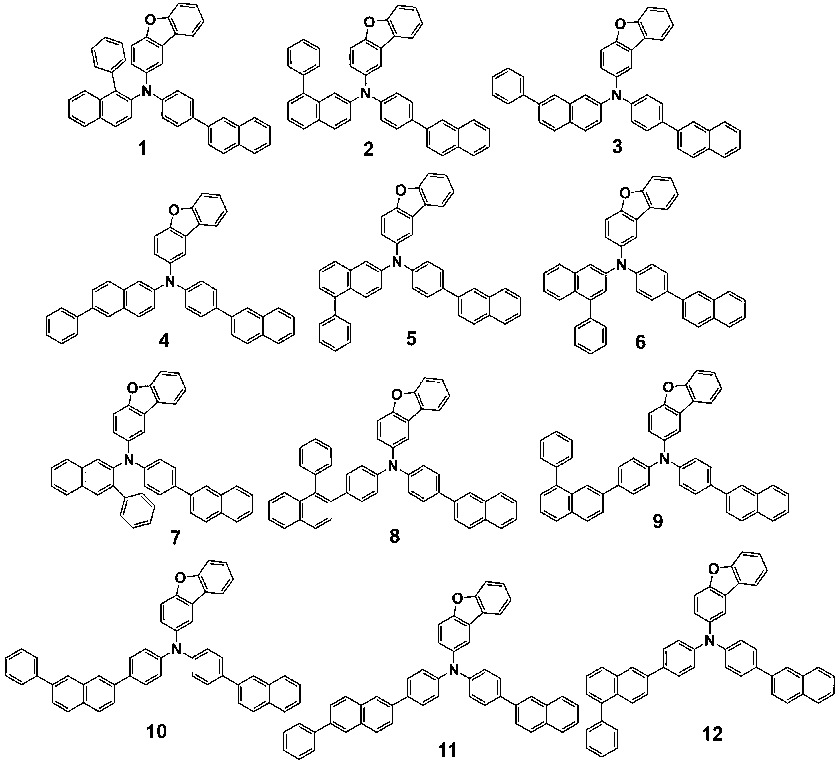

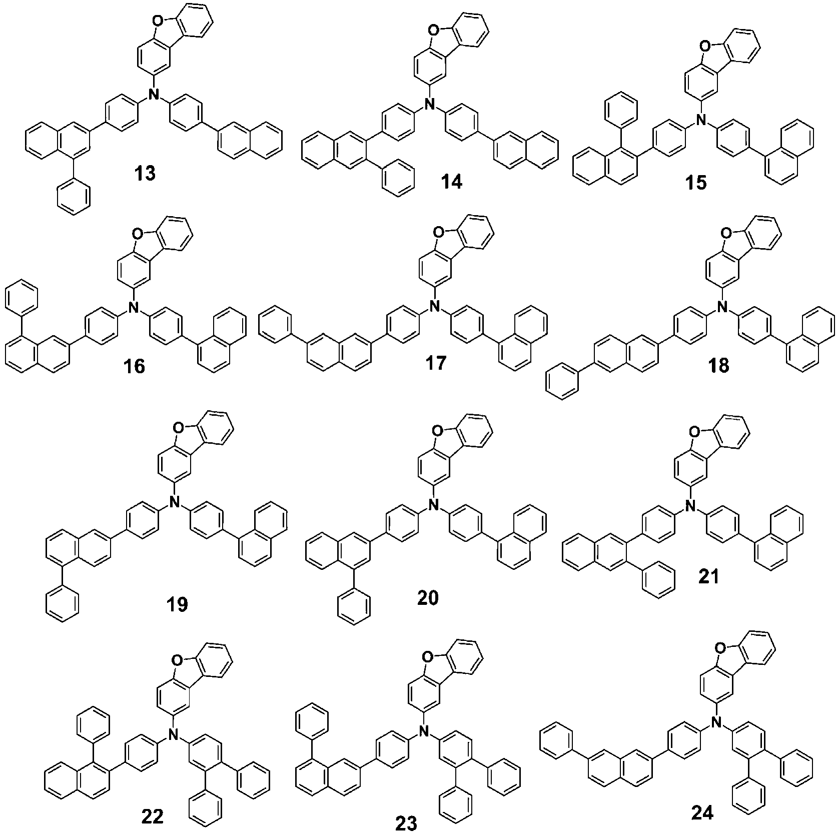

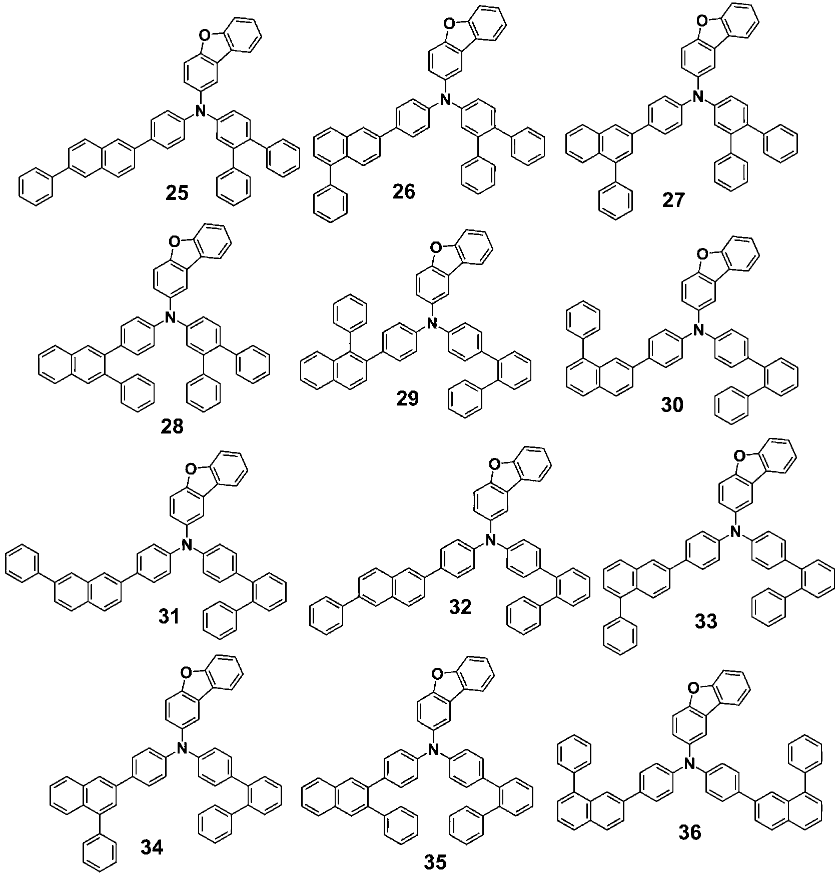

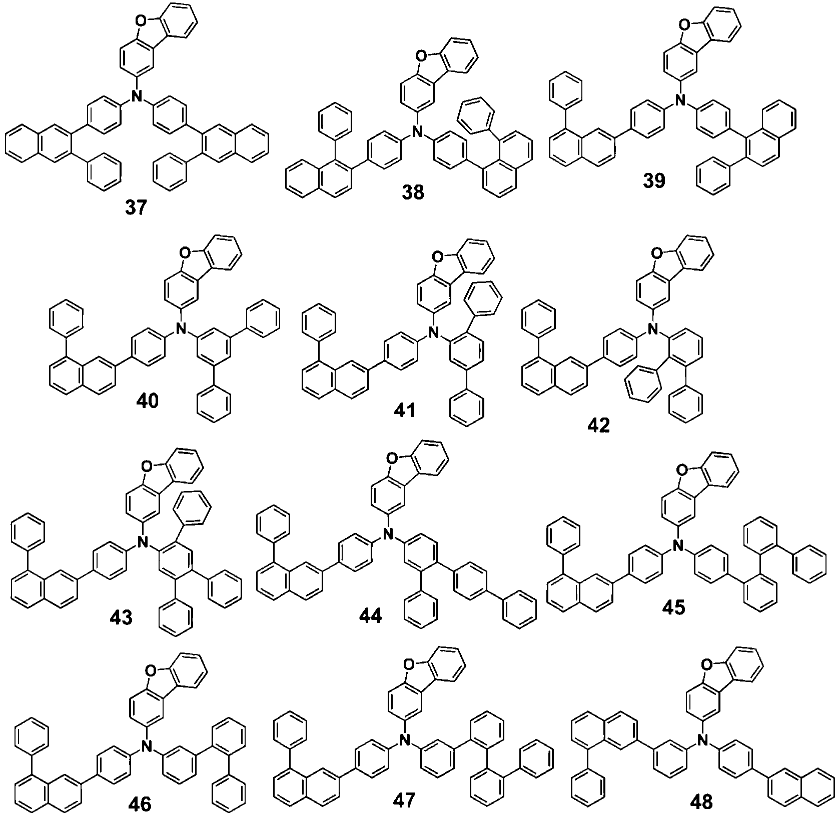

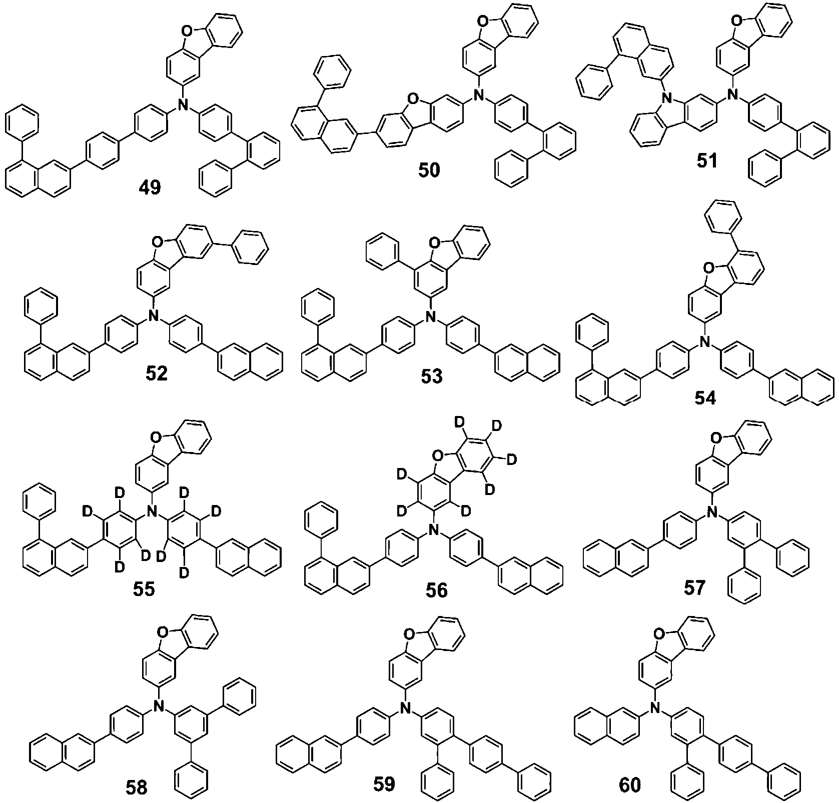

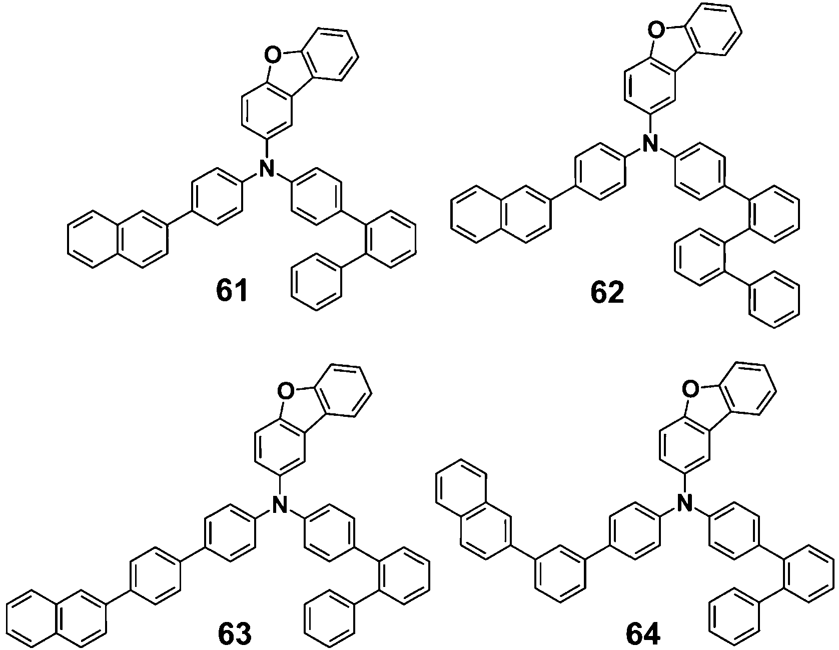

The monoamine compound of an aspect includes first to third substituents directly or indirectly bonded to an amine group. The first substituent is a 2-dibenzofuranyl group, and the second substituent is a phenylnaphthalenyl group or an unsubstituted naphthalenyl group. The phenylnaphthalenyl group may be a naphthalenyl group substituted with a phenyl group. The third substituent is a substituted phenyl group, and the substituent of the phenyl group may be a phenyl group, a biphenyl group, a terphenyl group, and/or a naphthalenyl group.

In Formula 1 described above, the 2-dibenzofuranyl group containing R^{a3} corresponds to the first substituent, and Ar¹ corresponds to the third substituent. That is, the third substituent is represented by any one of Formulas 2-1 to 2-4 described above. In Formula 1 described above, the naphthalenyl group containing R^{a2} corresponds to the second substituent. In Formula 1 described above, when n1 is 0 and R^{a2} is a hydrogen atom, the second substituent is an unsubstituted naphthalenyl group. In Formula 1 described above, when nl is 1 or greater, the second substituent may be a phenylnaphthalenyl group.

The monoamine compound of an aspect including the first to third substituents may prevent an increase in deposition temperature during deposition of the compound for forming a functional layer (e.g., a hole transport region HTR) of the light emitting element ED, and may thus exhibit no thermal decomposition, resulting in excellent material stability. The monoamine compound of an aspect including the first substituent (i.e., a 2-dibenzofuranyl group) and the second substituent (i.e., a phenylnaphthalenyl group or a naphthalenyl group) may have hole transport properties appropriately controlled to improve charge balance and enhance the hole transport properties. The monoamine compound of an aspect including the first and second substituents may exhibit excellent hole transport properties and contribute to reducing the driving voltage of the light emitting element ED. In addition, the monoamine compound of an aspect may contribute to improving luminous efficiency and lifespan of the light emitting element ED. The light emitting element ED of an aspect including the monoamine compound of an aspect may exhibit low driving voltage, high luminous efficiency, and/or long life characteristics.

The hole transport region HTR may include at least one among a hole injection layer HIL, a hole transport layer HTL, a buffer layer, a light emitting auxiliary layer (not shown), and an electron blocking layer EBL. The hole transport region HTR may have, for example, a thickness of about 50 Å to about 15000 Å.

The hole transport region HTR may have a single layer formed of a single material, a single layer formed of a plurality of different materials, or a multilayer structure having a plurality of layers formed of a plurality of different materials.

For example, the hole transport region HTR may have a single-layer structure formed of the hole injection layer HIL or the hole transport layer HTL, or a single-layer structure formed of a hole injection material or a hole transport material. For example, the hole transport region HTR may have a single-layer structure formed of a plurality of different materials, or a structure in which a hole injection layer HIL/hole transport layer HTL, a hole injection layer HIL/hole transport layer HTL/light emitting auxiliary layer (not shown), a hole injection layer HIL/light emitting auxiliary layer (not shown), a hole transport layer HTL/light emitting auxiliary layer (not shown), or a hole injection layer HIL/hole transport layer HTL/electron blocking layer EBL are stacked in order from the first electrode EL1, but an aspect is not limited thereto.

The hole transport region HTR may be formed using various methods such as a vacuum deposition method, a spin coating method, a cast method, a Langmuir-Blodgett (LB) method, an inkjet printing method, a laser printing method, and a laser induced thermal imaging (LITI) method.

The hole transport region HTR may further include compounds, which will be described later, in addition to the monoamine compound of an aspect.

The hole transport region HTR may include a compound represented by Formula H-1 below.

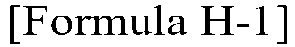

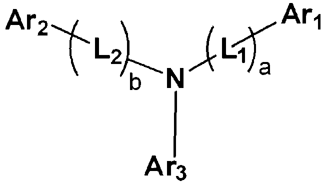

In Formula H-1 above, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. Meanwhile, when a or b is an integer of 2 or greater, a plurality of L₁'s and L₂'s may each independently be a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. In addition, in Formula H-1, Ar₃ may be a substituted or unsubstituted aryl group having 6 to 30 ring-forming carbon atoms, or a substituted or unsubstituted heteroaryl group having 2 to 30 ring-forming carbon atoms.

The compound represented by Formula H-1 above may be a monoamine compound. Alternatively, the compound represented by Formula H-1 above may be a diamine compound in which at least one of Ar₁ to Ar₃ includes an amine group as a substituent. In addition, the compound represented by Formula H-1 above may be a carbazole-based compound including a substituted or unsubstituted carbazole group in at least one of Ar₁ or Ar₂ or a substituted or unsubstituted fluorene-based group including a substituted or unsubstituted fluorene group in at least one of Ar₁ or Ar₂.

The compound represented by Formula H-1 may be represented by any one of compounds from Compound Group H below. However, the compounds listed in Compound Group H below are presented as an example, and the compound represented by Formula H-1 is not limited to the those listed in Compound Group H below.

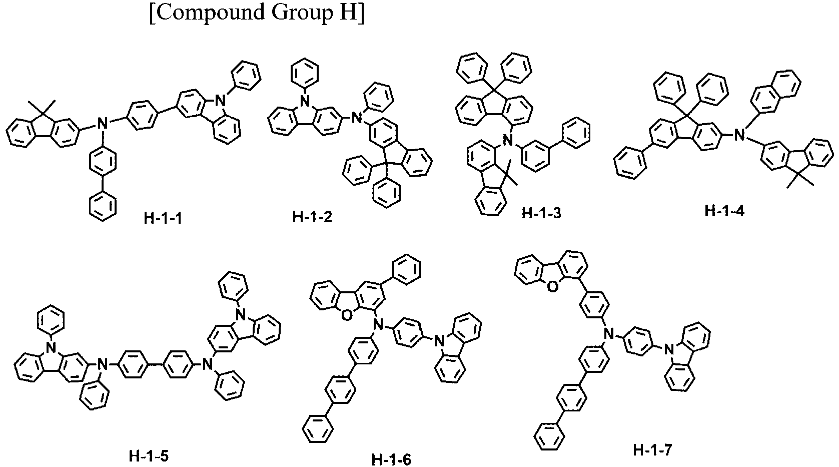

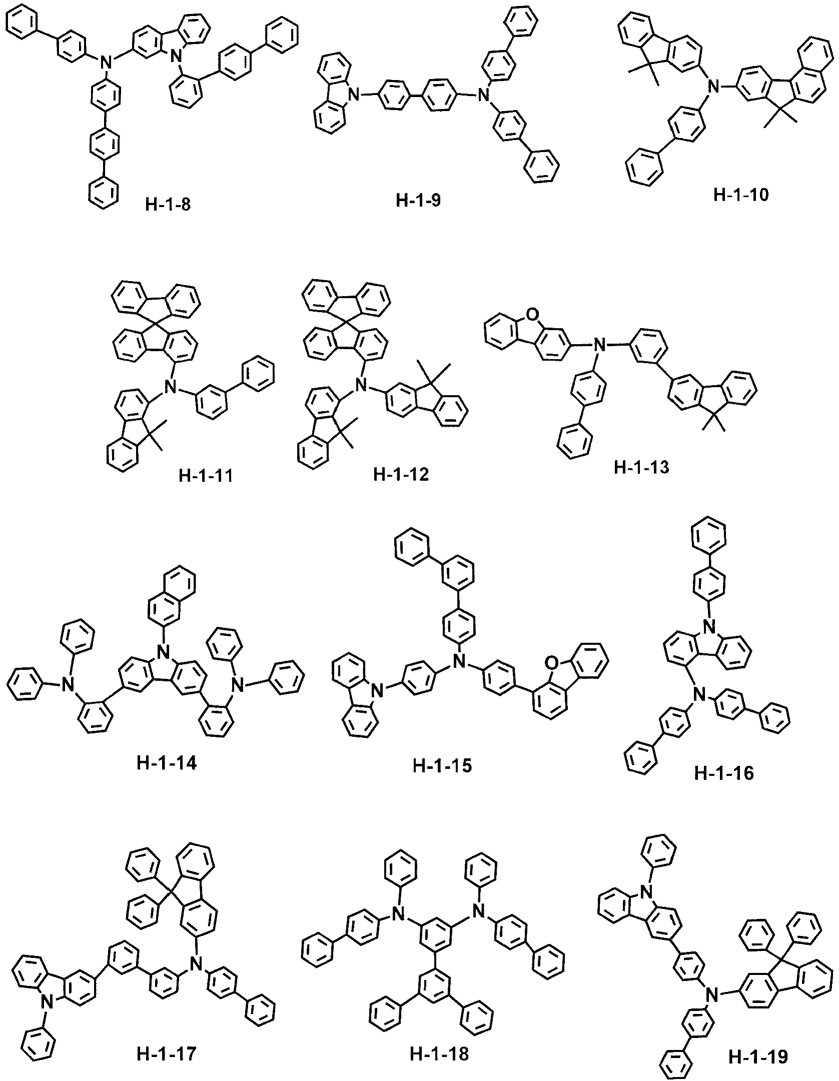

The hole transport region HTR may include a phthalocyanine compound such as copper phthalocyanine, N¹,N^{1'}-([1,1'-biphenyl]-4,4'-diyl)bis(N¹-phenyl-N⁴,N⁴-dim-tolylbenzene-1,4-diamine) (DNTPD), 4,4',4"-[tris(3-methylphenyl)phenylamino] triphenylamine (m-MTDATA), 4,4'4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonicacid (PANI/CSA), polyaniline/poly(4-styrenesulfonate) (PANI/PSS), N,N'-di(naphthalen-1-yl)-N,N'-diphenyl-benzidine (NPB), triphenylamine-containing polyetherketone (TPAPEK), 4-isopropyl-4'-methyldiphenyliodonium tetrakis(pentafluorophenyl)borate, dipyrazino[2,3-f: 2',3'-h]quinoxaline-2,3,6,7,10,11-hexacarbonitrile (HATCN), and the like.

The hole transport region HTR may include carbazole-based derivatives such as N-phenyl carbazole and polyvinyl carbazole, fluorene-based derivatives, N,N'-bis(3-methylphenyl)-N,N'-diphenyl-[1,1-biphenyl]-4,4'-diamine (TPD), triphenylamine-based derivatives such as 4,4',4"-tris(N-carbazolyl)triphenylamine (TCTA), N,N'-di(naphthalen-1-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), and 1,3-bis(N-carbazolyl)benzene (mCP), and the like.

In addition, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol- 9-yl)benzene (mDCP), and the like.

The hole transport region HTR may include the compounds of the hole transport region described above in at least one among the hole injection layer HIL, the hole transport layer HTL, and the electron blocking layer EBL.

The hole transport region HTR may have a thickness of about 100 Å to about 10000 Å, for example, about 100 Å to about 5000 Å. When the hole transport region HTR includes the hole injection layer HIL, the hole injection layer HIL may have a thickness of, for example, about 30 Å to about 1000 Å. When the hole transport region HTR includes the hole transport layer HTL, the hole transport layer HTL may have a thickness of about 30 Å to about 1000 Å. When the hole transport region HTR includes the electron blocking layer EBL, the electron blocking layer EBL may have a thickness of, for example, about 10 Å to about 1000 Å. When the thicknesses of the hole transport region HTR, the hole injection layer HIL, the hole transport layer HTL, and the electron blocking layer EBL satisfy the above-described ranges, satisfactory hole transport properties may be obtained without a substantial increase in driving voltage.

The hole transport region HTR may further include, in addition to the above-described materials, a charge generation material to increase conductivity. The charge generation material may be uniformly or non-uniformly dispersed in the hole transport region HTR. The charge generation material may be, for example, a p-dopant. The p-dopant may include at least one of halogenated metal compounds, quinone derivatives, metal oxides, or cyano group-containing compounds, but is not limited thereto. For example, the p-dopant may include halogenated metal compounds such as CuI and RbI, quinone derivatives such as tetracyanoquinodimethane (TCNQ) and 2,3,5,6-tetrafluoro-7,7',8,8-tetracyanoquinodimethane (F4-TCNQ), metal oxides such as tungsten oxides and molybdenum oxides, cyano group-containing compounds such as dipyrazino[2,3-f: 2',3'-h] quinoxaline-2,3,6,7,10,11-hexacarbonitrile (HATCN) and 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), and the like, but an aspect is not limited thereto.

As described above, the hole transport region HTR may further include at least one of a buffer layer (not shown) or an electron blocking layer EBL in addition to the hole injection layer HIL and the hole transport layer HTL. The buffer layer (not shown) may compensate a resonance distance according to wavelengths of light emitted from an emission layer EML, and may thus increase light emitting efficiency. Materials which may be included in the hole transport region HTR may be used as materials included in the buffer layer (not shown). The electron blocking layer EBL is a layer that serves to prevent electrons from being injected from the electron transport region ETR to the hole transport region HTR.

The emission layer EML is provided on the hole transport region HTR. The emission layer EML may have, for example, a thickness of about 100 Å to about 1000 Å or about 100 Å to about 300 Å. The emission layer EML may have a single layer formed of a single material, a single layer formed of a plurality of different materials, or a multilayer structure having a plurality of layers formed of a plurality of different materials.

In the light emitting element ED according to an aspect, the emission layer EML may include an anthracene derivative, a pyrene derivative, a fluoranthene derivative, a chrysene derivative, a dihydrobenzanthracene derivative, or a triphenylene derivative. To be specific, the emission layer EML may include an anthracene derivative or a pyrene derivative.

In the light emitting element ED of an aspect shown in FIGS. 3 to 6, the emission layer EML may include a host and a dopant, and the emission layer EML may include a compound represented by Formula E-1 below. The compound represented by Formula E-1 below may be used as a fluorescent host material.

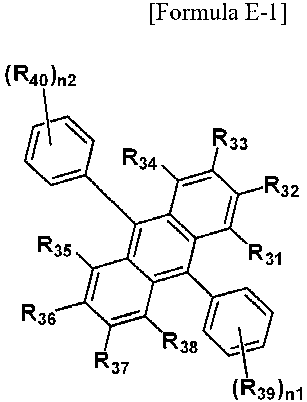

In Formula E-1, n1 and n2 may each independently be an integer of 0 to 5. When n1 is an integer of 2 or greater, a plurality of R₃₉'s may all be the same or at least one may be different from the others. When n1 is 0, the case may be the same as a case in which n1 is 5 and five R₃₉'s are hydrogen atoms. When n2 is an integer of 2 or greater, a plurality of R₄₀'s may all be the same or at least one may be different from the others. When n2 is 0, the case may be the same as a case in which n2 is 5 and five R₄₀'s are hydrogen atoms.

In Formula E-1, R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring.

Formula E-1 may be represented by any one of compounds E1 to E21 below.

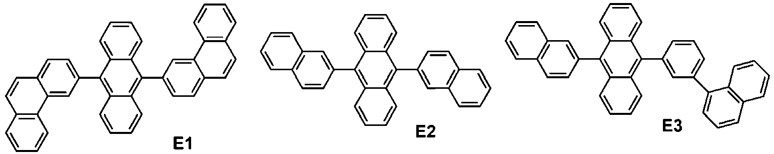

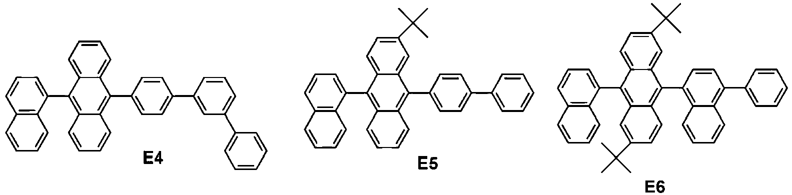

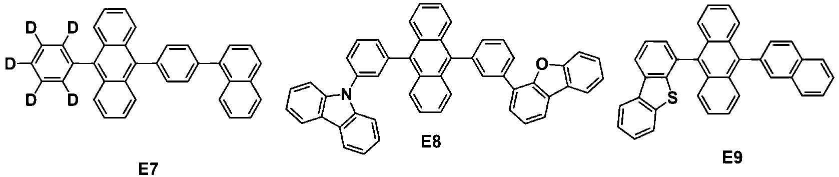

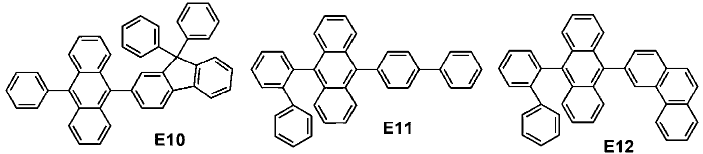

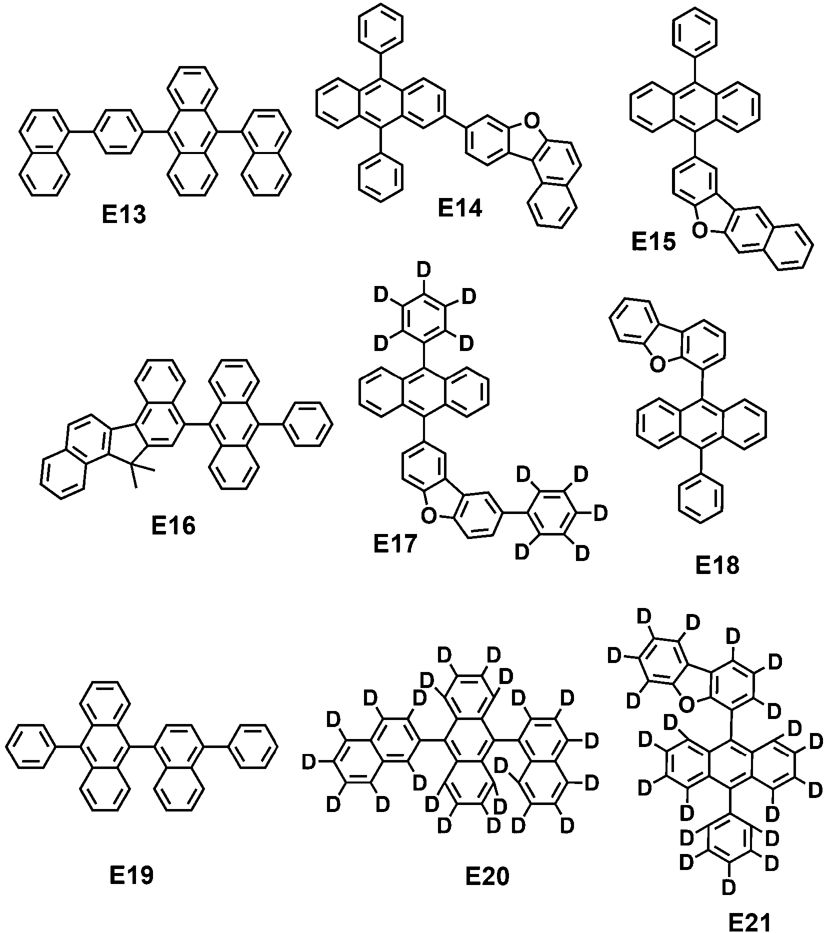

In an aspect, the emission layer EML may include a compound represented by Formula E-2a or Formula E-2b below. The compound represented by Formula E-2a or Formula E-2b may be used as a host material for a phosphorescent element.

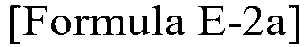

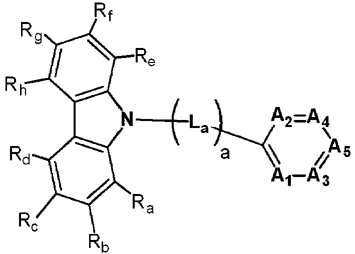

In Formula E-2a, a may be an integer of 0 to 10, and Lₐ may be a direct linkage, a substituted or unsubstituted arylene group having 6 to 30 ring-forming carbon atoms, or a substituted or unsubstituted heteroarylene group having 2 to 30 ring-forming carbon atoms. Meanwhile, when a is an integer of 2 or greater, a plurality of Lₐ's may each independently be a substituted or unsubstituted arylene group having 6 to 30 ring-forming carbon atoms, or a substituted or unsubstituted heteroarylene group having 2 to 30 ring-forming carbon atoms.

In addition, in Formula E-2a, A₁ to A₅ may each independently be N or CRᵢ. Rₐ to Rᵢ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. Rₐ to Rᵢ may be bonded to an adjacent group to form a hydrocarbon ring or a heterocycle containing N, O, S, and the like as a ring-forming atom.

Meanwhile, in Formula E-2a, two or three selected from A₁ to A₅ may be N, and the others may be CRᵢ.

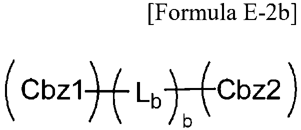

In Formula E-2b, Cbz1 and Cbz2 may each independently be an unsubstituted carbazole group or an aryl-substituted carbazole group having 6 to 30 ring-forming carbon atoms. L_{b} may be a direct linkage, a substituted or unsubstituted arylene group having 6 to 30 ring-forming carbon atoms, or a substituted or unsubstituted heteroarylene group having 2 to 30 ring-forming carbon atoms, b may be an integer of 0 to 10, and when b is an integer of 2 or greater, a plurality of L_{b}'s may each independently be a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one of compounds from Compound Group E-2 below. However, the compounds listed in Compound Group E-2 below are presented as an example, and the compound represented by Formula E-2a or Formula E-2b is not limited to those listed in Compound Group E-2 below.

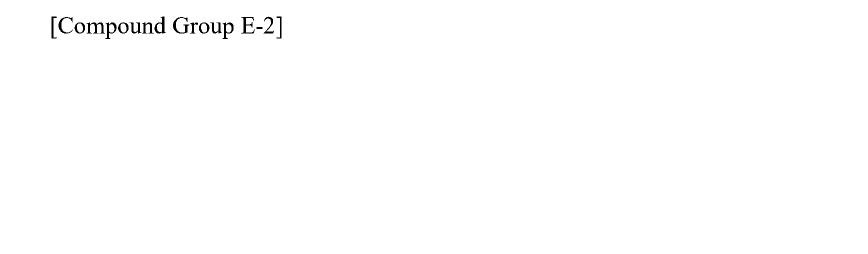

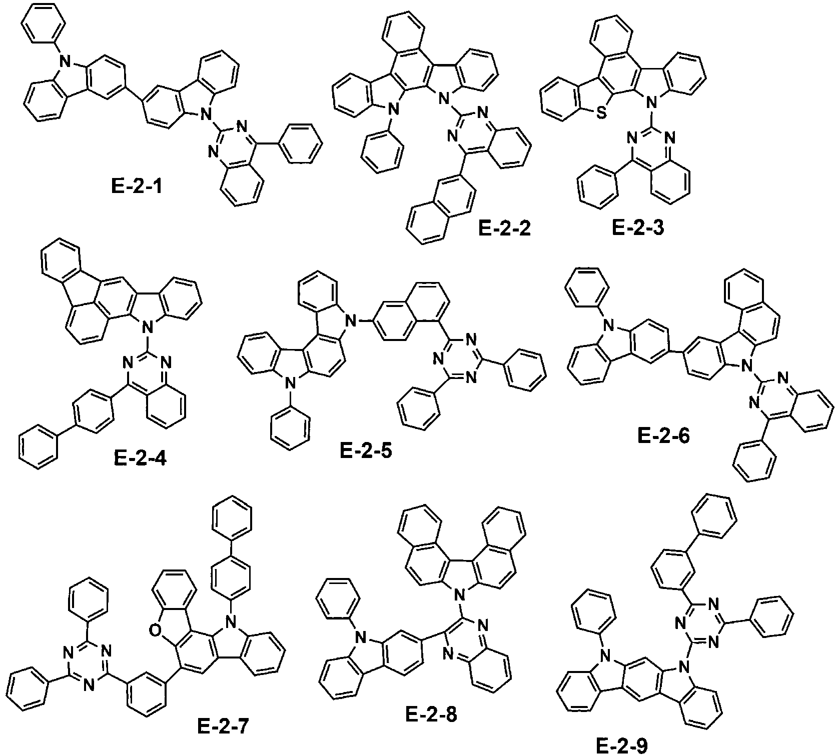

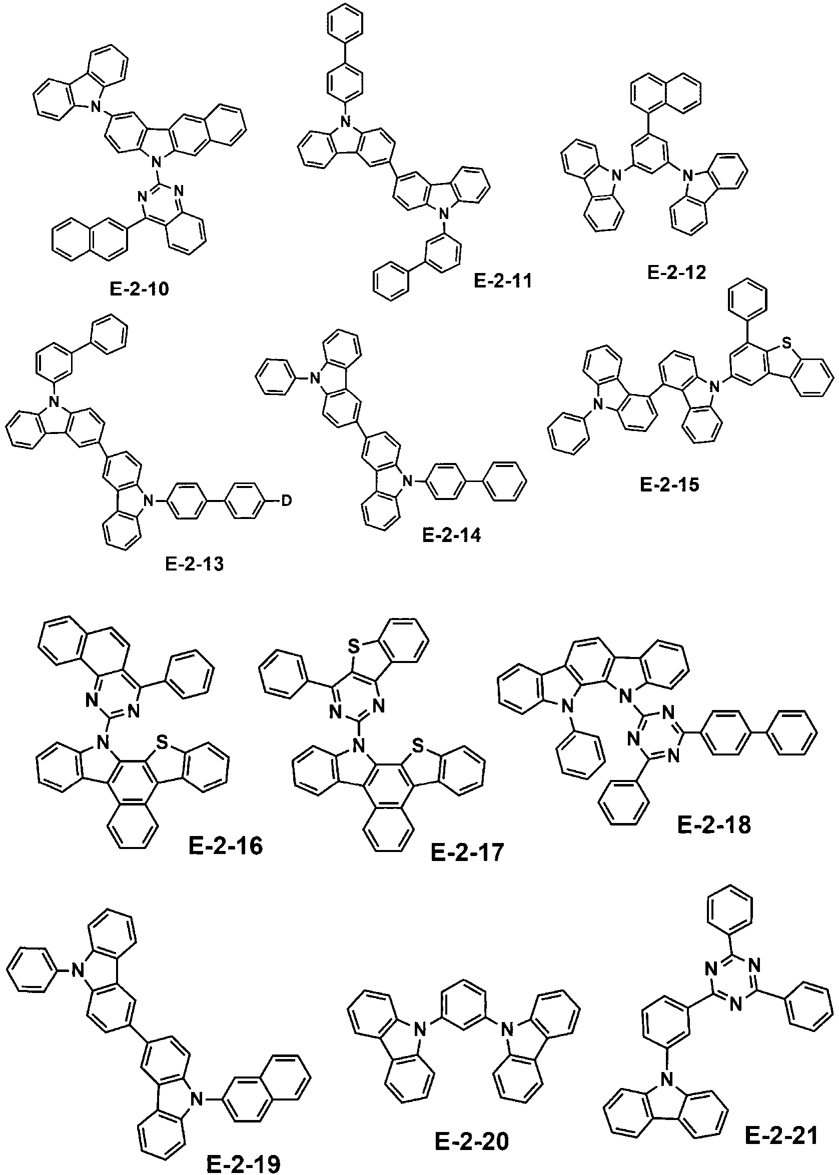

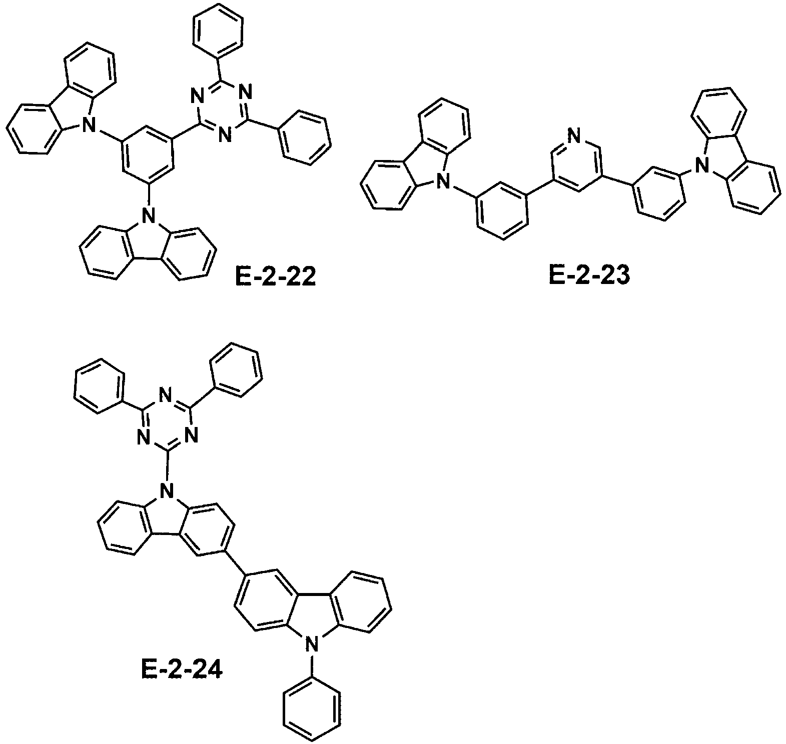

The emission layer EML may further include a general material known in the art as a host material. For example, the emission layer EML may include, as a host material, at least one among bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazolyl-9-yl)benzene (mCP), 2,8-bis(diphenylphosphoryl)dibenzofuran (PPF), 4,4',4"-tris(carbazol-9-yl)-triphenylamine (TCTA), and 1,3,5-tris(1-phenyl-1H-benzo[d]imidazol-2-yl)benzene (TPBi). However, an aspect is not limited thereto, and for example, tris(8-hydroxyquinolinato)aluminium (Alq₃), 9,10-di(naphthalen-2-yl)anthracene (ADN), 3-tert-butyl-9,10-di(naphth-2-yl)anthracene (TBADN), distyrylarylene (DSA), 4,4'-bis(9-carbazolyl)-2,2'-dimethylbiphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), and the like may be used as a host material.

The emission layer EML may include a compound represented by Formula M-a or Formula M-b below. The compound represented by Formula M-a or Formula M-b below may be used as a phosphorescent dopant material.

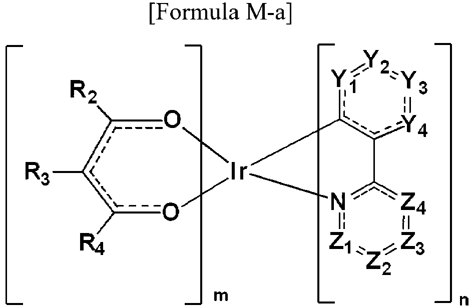

In Formula M-a above, Y₁ to Y₄ and Z₁ to Z₄ may each independently be CR₁ or N, and R₁ to R₄ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. In Formula M-a, m is 0 or 1, and n is 2 or 3. In Formula M-a, when m is 0, n is 3, and when m is 1, n is 2.

The compound represented by Formula M-a may be used as a phosphorescent dopant.

The compound represented by Formula M-a may be represented by any one of compounds M-a1 to M-a25 below. However, the compounds M-a1 to M-a25 below are presented as an example, and the compound represented by Formula M-a is not limited to those represented by the compounds M-a1 to M-a25 below.

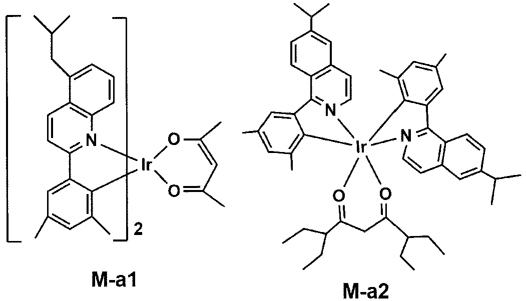

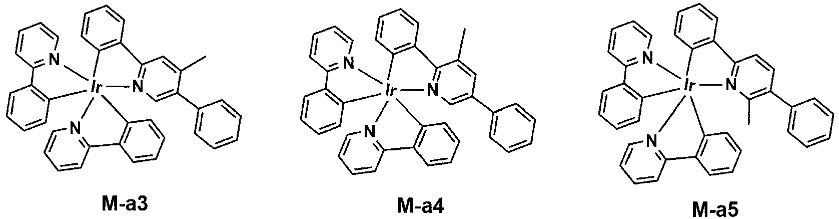

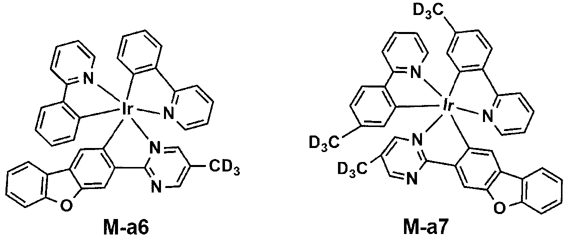

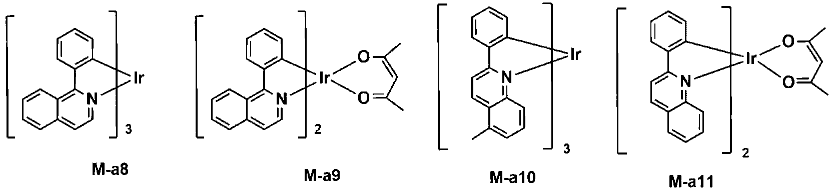

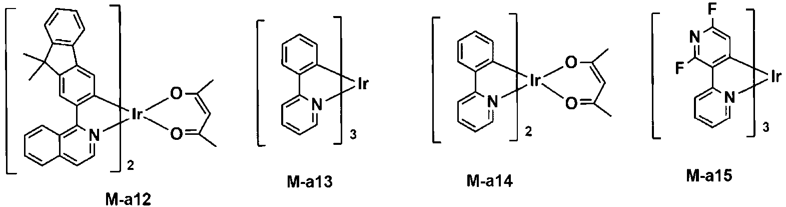

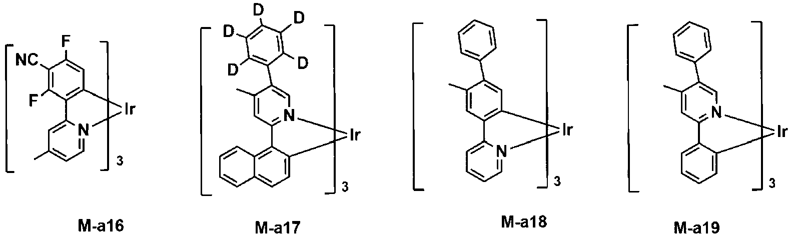

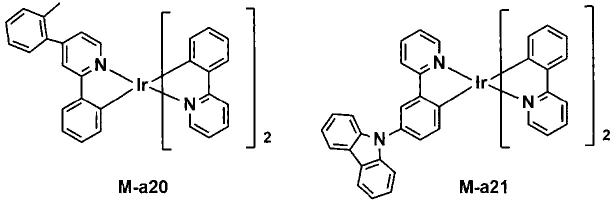

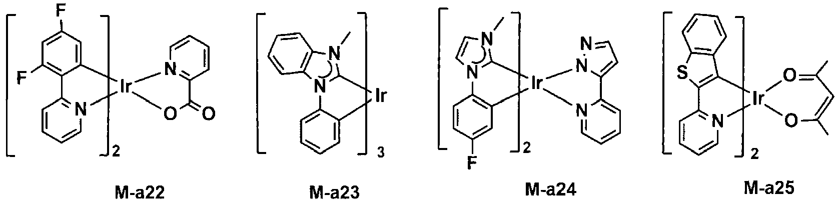

The compounds M-a1 and M-a2 may be used as a red dopant material, and the compounds M-a3 to M-a5 may be used as a green dopant material.

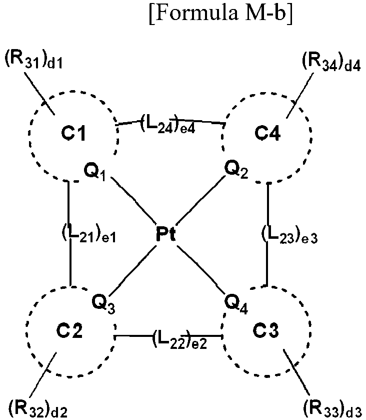

In Formula M-b, Q₁ to Q₄ may each independently be C or N. C1 to C4 may each independently be a substituted or unsubstituted hydrocarbon ring having 5 to 30 ring-forming carbon atoms, a substituted or unsubstituted aryl group having 6 to 30 ring-forming carbon atoms, or a substituted or unsubstituted heterocycle having 2 to 30 ring-forming carbon atoms.

L₂₁ to L₂₄ may each independently be a direct linkage, *-O-*, 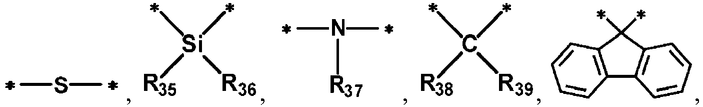 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms. e1 to e4 may each independently be 0 or 1.

R₃₁ to R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring. d1 to d4 may each independently be an integer of 0 to 4. When d1 is an integer of 2 or greater, a plurality of R₃₁'s may all be the same or at least one may be different from the others. When d2 is an integer of 2 or greater, a plurality of R₃₂'s may all be the same or at least one may be different from the others. When d3 is an integer of 2 or greater, a plurality of R₃₃'s may all be the same or at least one may be different from the others. When d4 is an integer of 2 or greater, a plurality of R₃₄'s may all be the same or at least one may be different from the others.

A compound represented by Formula M-b may be used as a blue phosphorescent dopant or a green phosphorescent dopant. The compound represented by Formula M-b may be represented by any one of compounds below. However, the compounds below are presented as an example, and the compound represented by Formula M-b is not limited to those represented by the compounds below. In compounds AD-01 to AD-53, D indicates a deuterium atom.

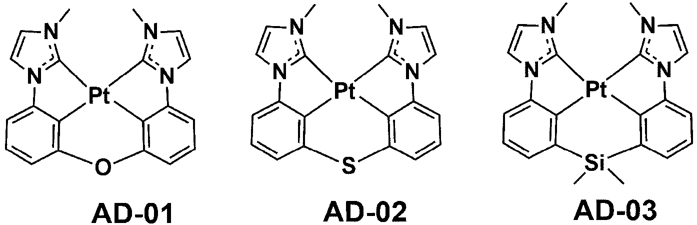

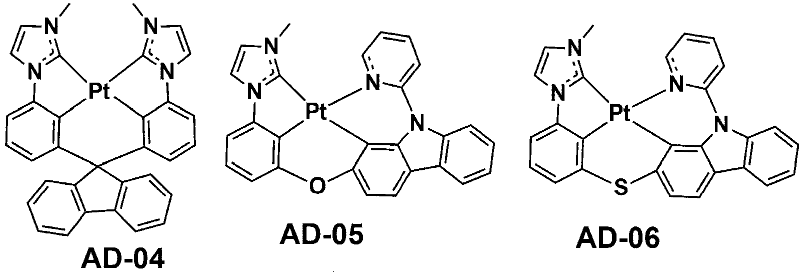

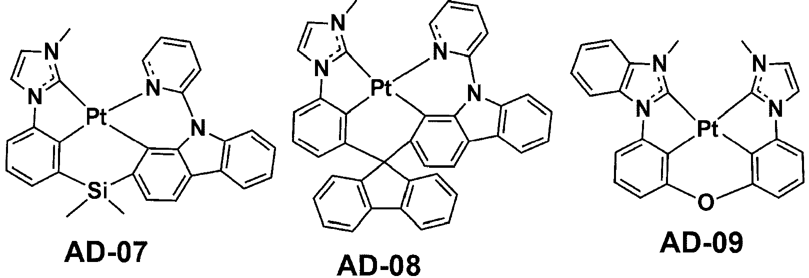

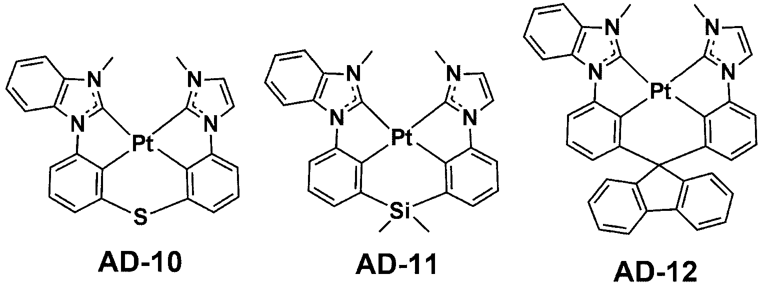

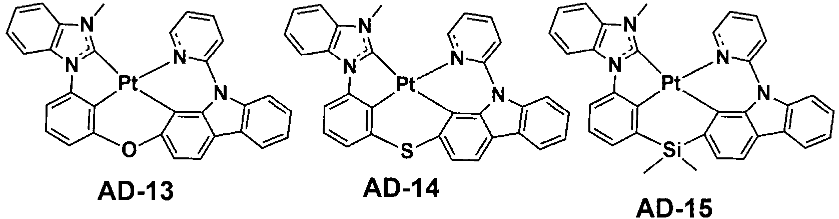

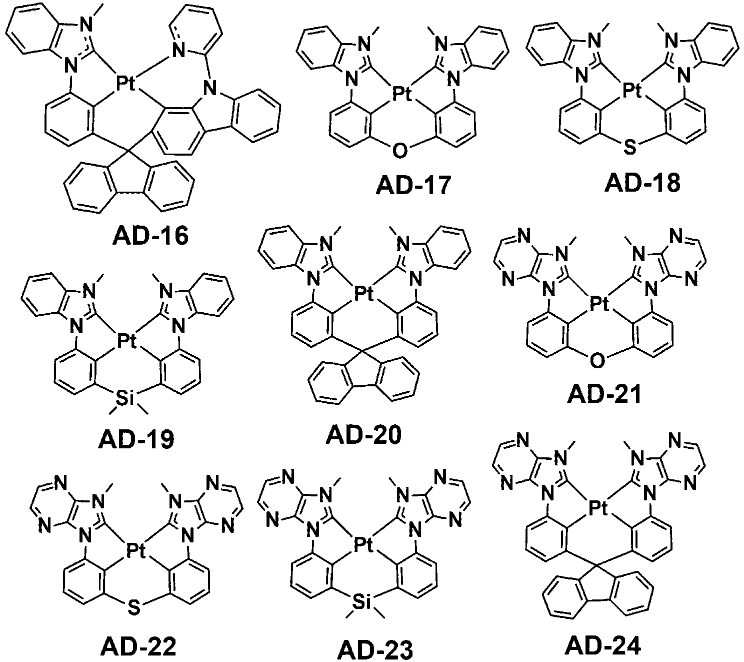

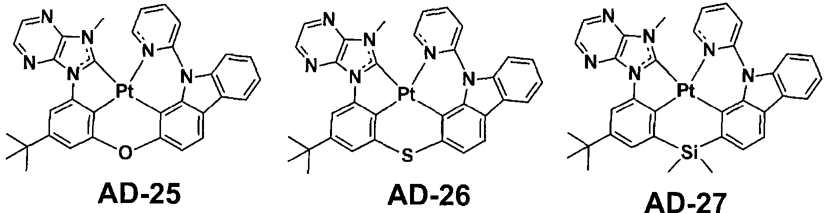

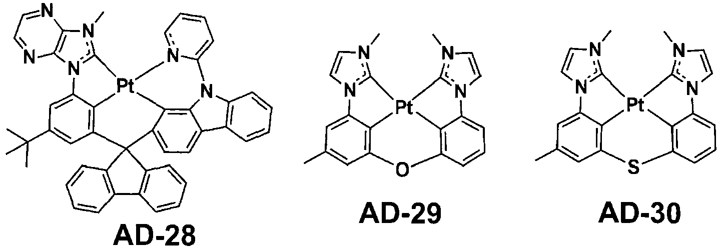

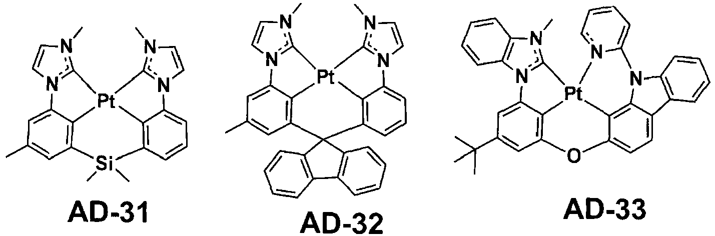

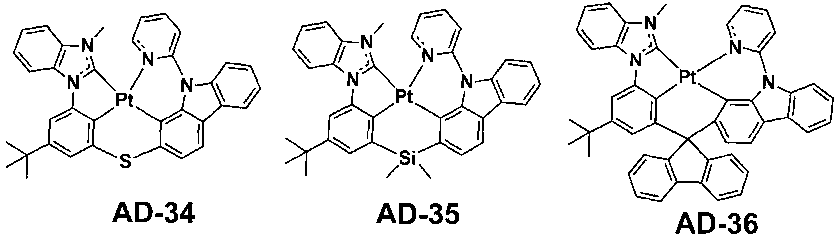

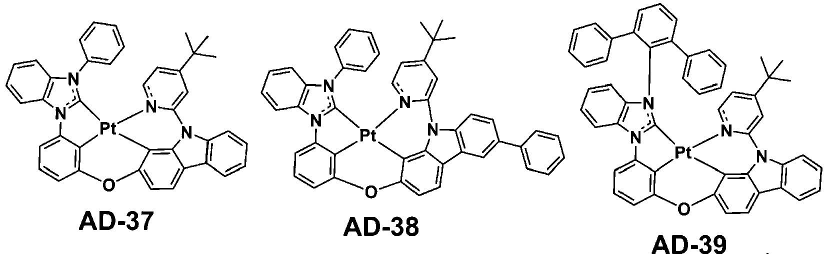

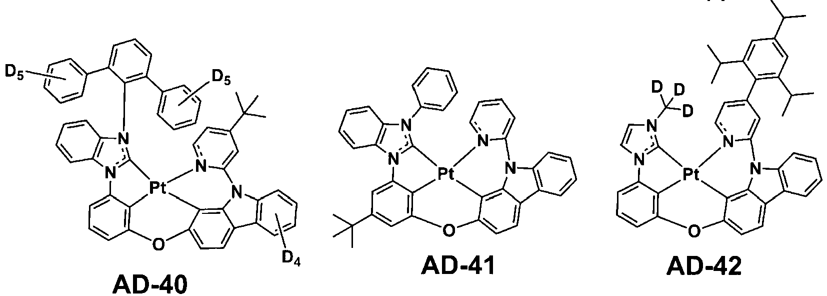

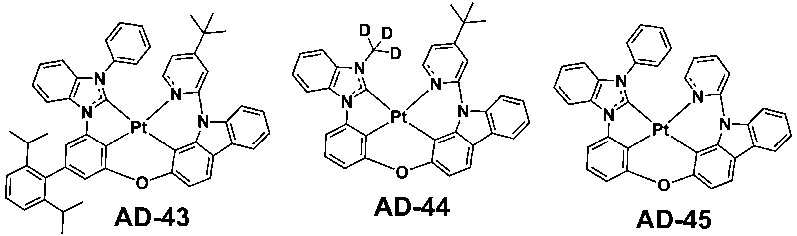

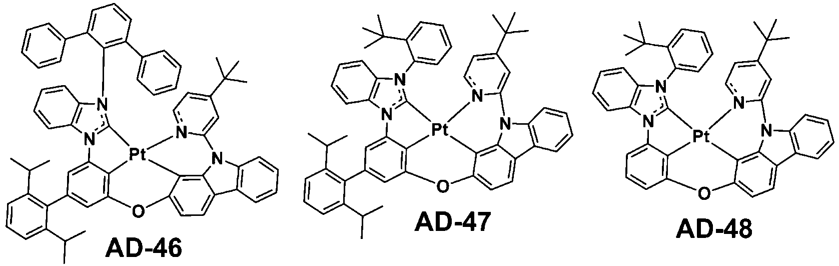

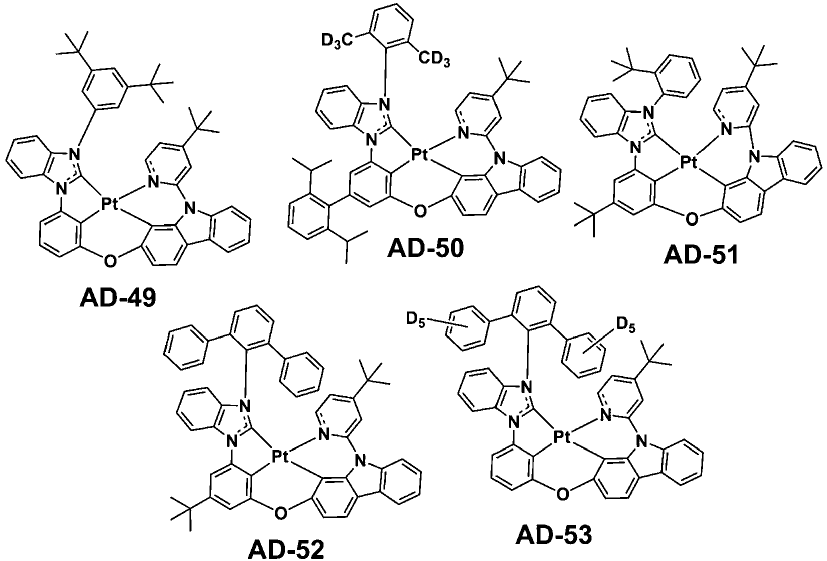

The emission layer EML may include a compound represented by any one of Formulas F-a to F-c below. The compounds represented by Formulas F-a to F-c below may be used as a fluorescence dopant material.

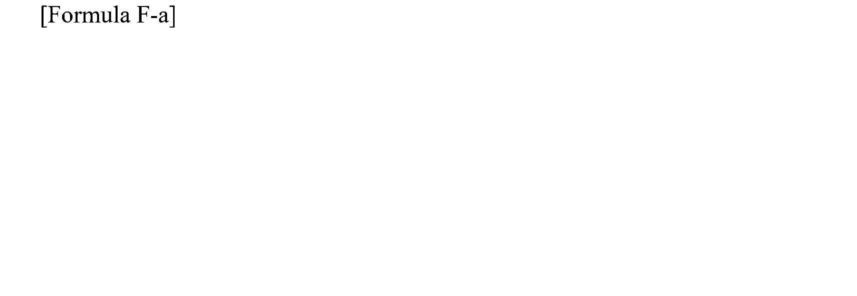

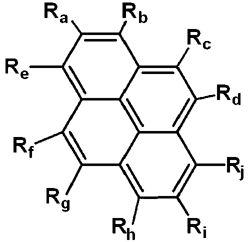

In Formula F-a above, two selected from Rₐ to Rⱼ may each independently be substituted with *-NAr₁Ar₂. The others among Rₐ to Rⱼ which are not substituted with *-NAr₁Ar₂ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In *-NAr₁Ar₂, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. For example, at least one of Ar₁ or Ar₂ may be a heteroaryl group containing O or S as a ring-forming atom.

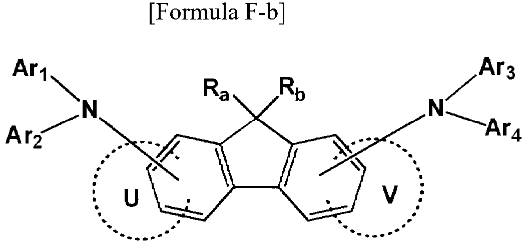

In Formula F-b above, Rₐ and R_{b} may each independently be a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. Ar₁ to Ar₄ may each independently be a substituted or unsubstituted aryl group having 6 to 30 ring-forming carbon atoms, or a substituted or unsubstituted heteroaryl group having 2 to 30 ring-forming carbon atoms.

In Formula F-b, U and V may each independently be a substituted or unsubstituted hydrocarbon ring having 5 to 30 ring-forming carbon atoms, or a substituted or unsubstituted heterocycle having 2 to 30 ring-forming carbon atoms. At least one of Ar₁ to Ar₄ may be a heteroaryl group containing O or S as a ring-forming atom.

In Formula F-b, the number of rings represented by U and V may each independently be 0 or 1. For example, in Formula F-b, when the number of U or V is 1, one ring forms a fused ring in a portion indicated by U or V, and when the number of U or V is 0, it means that no ring indicated by U or V is present. To be specific, when the number of U is 0 and the number of V is 1, or when the number of U is 1 and the number of V is 0, a fused ring having a fluorene core of Formula F-b may be a cyclic compound having four rings. In addition, when the number of U and V are both 0, the fused ring of Formula F-b may be a cyclic compound having three rings. In addition, when the number of U and V are both 1, the fused ring having a fluorene core of Formula F-b may be a cyclic compound having five rings.

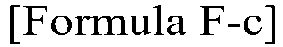

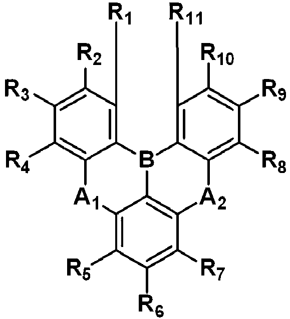

In Formula F-c, A₁ and A₂ may each independently be O, S, Se, or NRₘ, and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ are each independently a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

In Formula F-c, A₁ and A₂ may each independently be bonded to substituents of neighboring rings to form a fused ring. For example, when A₁ and A₂ are each independently NRₘ, A₁ may be bonded to R₄ or R₅ to form a ring. In addition, A₂ may be bonded to R₇ or R₈ to form a ring.

The emission layer EML may include, as a known dopant material, styryl derivatives (e.g., 1,4-bis[2-(3-N-ethylcarbazoryl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N-diphenylamino)phenyl)vinyl]biphenyl (DPAVBi), perylene and derivatives thereof (e.g., 2,5,8,11-tetra-t-butylperylene (TBP)), pyrene and derivatives thereof (e.g., 1,1'-dipyrene, 1,4-dipyrenylbenzene, 1,4-bis(N,N'-diphenylamino)pyrene), and the like.

The emission layer EML may include a known phosphorescent dopant material. For example, as a phosphorescent dopant, a metal complex including iridium (Ir), platinum (Pt), osmium (Os), gold (Au), titanium (Ti), zirconium (Zr), hafnium (Hf), europium (Eu), and terbium (Tb), or thulium (Tm) may be used. To be specific, iridium(III) bis(4,6-difluorophenylpyridinato-N,C2')picolinate (FIrpic), bis(2,4-difluorophenylpyridinato)-tetrakis(1-pyrazolyl)borate iridium(III) (Fir6), platinum octaethyl porphyrin (PtOEP), and the like may be used as a phosphorescent dopant. However, an aspect is not limited thereto.

The emission layer EML may include a quantum dot material. The core of a quantum dot may be selected from a Group II-VI compound, a Group I-II-VI compound, a Group II-IV-VI compound, a Group I-II-IV-VI compound, a Group II-IV-V compound, a Group III-VI compound, a Group I-III-VI compound, a Group III-V compound, a Group III-II-V compound, a Group IV-VI compound, a Group IV element, a Group IV compound, and a combination thereof.

The Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and a mixture thereof.

Meanwhile, the Group II-VI compound may further include a Group I metal and/or a Group IV element. The Group I-II-VI compound may be selected from CuSnS or CuZnS, and the Group II-IV-VI compound may be selected from ZnSnS and the like. The Group I-II-IV-VI compound may be selected from quaternary compounds selected from the group consisting of Cu₂ZnSnS₂, Cu₂ZnSnS₄, Cu₂ZnSnSe₄, Ag₂ZnSnS₂, and a mixture thereof.

The Group II-IV-V compound may be selected from a ternary compound selected from the group consisting of ZnSnP, ZnSnP₂, ZnSnAs₂, ZnGeP₂, ZnGeAs₂, CdSnP₂, and CdGeP₂ and a mixture thereof.

The Group III-VI compound may include a binary compound such as GaS, Ga₂S₃, GaSe, Ga₂Se₃, GaTe, InTe, InS, InSe, In₂S₃, and In₂Se₃, a ternary compound such as InGaS₃ and InGaSe₃, or any combination thereof.

The Group I-III-VI compound may be selected from a ternary compound selected from the group consisting of AgInS, AgInS₂, CuInS, CuInS₂, AgGaS₂, CuGaS₂ CuGaO₂, AgGaO₂, AgAlO₂, or a mixture thereof, or a quaternary compound such as AgInGaS₂ and CuInGaS₂.

The Group III-V compound may be selected from the group consisting of a binary compound selected from the group consisting of GaN, GaP, GaAs, GaSb, AlN, AlP, AlAs, AlSb, InN, InP, InAs, InSb, and a mixture thereof, a ternary compound selected from the group consisting of GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and a mixture thereof, and a quaternary compound selected from the group consisting of GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and a mixture thereof. Meanwhile, the Group III-V compound may further include a Group II metal. For example, InZnP and the like may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

Each element included in the multi-element compound such as the binary compound, ternary compound, and quaternary compound may be present in particles at a uniform concentration or a non-uniform concentration. That is, Formula above indicates the types of elements included in a compound, and element ratios in the compound may be different. For example, AgInGaS₂ may indicate AgInₓGa₁₋ₓS₂ (x is a real number between 0 and 1).

Meanwhile, the quantum dot may have a single structure having a uniform concentration of each element included in the corresponding quantum dot or a dual structure of core-shell. For example, materials included in the core may be different from materials included in the shell.

The shell of the quantum dot may serve as a protection layer to prevent the chemical deformation of the core so as to keep semiconductor properties, and/or a charging layer to impart electrophoresis properties to the quantum dot. The shell may be single-layered or multi-layered. An interface between the core and the shell may have a concentration gradient in which the concentration of an element present in the shell becomes lower towards the center.

In some aspects, the quantum dot may have the above-described core/shell structure including a core having nano-crystals and a shell surrounding the core. Examples of the shell of the quantum dot may be a metal or non-metal oxide, a semiconductor compound, or a combination thereof.

For example, the metal or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, or a ternary compound such as MgAl₂O₄, CoFe₂O₄, NiFe₂O₄, and CoMn₂O₄, but the present disclosure is not limited thereto.

In addition, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and the like, but the present disclosure is not limited thereto.

Each element included in the multi-element compound such as the binary compound and the ternary compound may be present in particles at a uniform concentration or a non-uniform concentration. That is, Formula above indicates the types of elements included in a compound, and element ratios in the compound may be different.

The quantum dot may have, in a light emitting wavelength spectrum, a full width of half maximum (FWHM) of about 45 nm or less, preferably about 40 nm or less, and more preferably about 30 nm or less, and in this range, color purity or color reproducibility may be improved. In addition, light emitted through the quantum dot is emitted in all directions, and thus a wide viewing angle may be improved.

In addition, the form of the quantum dot is not particularly limited as long as it is a form commonly used in the art, but more specifically, a quantum dot in the form of spherical, pyramidal, multi-arm, or cubic nanoparticles, nanotubes, nanowires, nanofibers, nanoplatelets, and the like may be used.

As the size of the quantum dot or the ratio of elements in the quantum dot compound is regulated, the energy band gap may be accordingly controlled to obtain light of various wavelengths from the quantum dot emission layer. Therefore, by using the quantum dots as described above (using quantum dots of different sizes or having different element ratios in the quantum dot compound), a light emitting element emitting light of various wavelengths may be obtained. Specifically, the size of the quantum dot or the ratio of elements in the quantum dot compound may be regulated to emit red, green, and/or blue light. In addition, the quantum dots may be configured to emit white light by combining light of various colors.

In the light emitting element ED according to an aspect shown in FIGS. 3 to 6, an electron transport region ETR is provided on the emission layer EML. The electron transport region ETR may include at least one among a hole blocking layer HBL, an electron transport layer ETL, and an electron injection layer EIL, but an aspect is not limited thereto.

The electron transport region ETR may have a single layer formed of a single material, a single layer formed of a plurality of different materials, or a multilayer structure having a plurality of layers formed of a plurality of different materials.

For example, the electron transport region ETR may have a single layer structure of an electron injection layer EIL or an electron transport layer ETL, and may have a single layer structure formed of an electron injection material and an electron transport material. In addition, the electron transport region ETR may have a single layer structure formed of a plurality of different materials, or may have a structure in which an electron transport layer ETL/electron injection layer EIL, or a hole blocking layer HBL/electron transport layer ETL/electron injection layer EIL are stacked in order from the emission layer EML, but is not limited thereto. The electron transport region ETR may have a thickness of, for example, about 100 Å to about 1500 Å.

The electron transport region ETR may be formed using various methods such as a vacuum deposition method, a spin coating method, a cast method, a Langmuir-Blodgett (LB) method, an inkjet printing method, a laser printing method, and a laser induced thermal imaging (LITI) method.

The electron transport region ETR may include a compound represented by Formula ET-2 below.

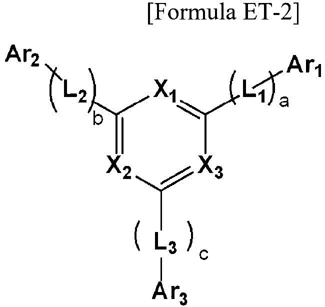

In Formula ET-2, at least one of X₁ to X₃ may be N and the rest are CRₐ. Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a to c may each independently be an integer of 0 to 10. In Formula ET-2, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. Meanwhile, when a to c are an integer of 2 or greater, L₁ to L₃ may each independently be a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, an aspect is not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(biphenyl-4-yl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), berylliumbis(benzoquinolin-10-olate (Bebq₂), 9,10-di(naphthalen-2-yl)anthracene (ADN), 1,3-bis[3,5-di(pyridin-3-yl)phenyl]benzene (BmPyPhB), or a mixture thereof.

The electron transport region ETR may include at least one of compounds ET1 to ET36 below.

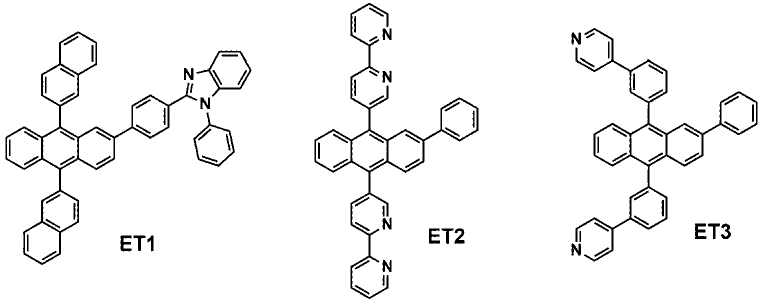

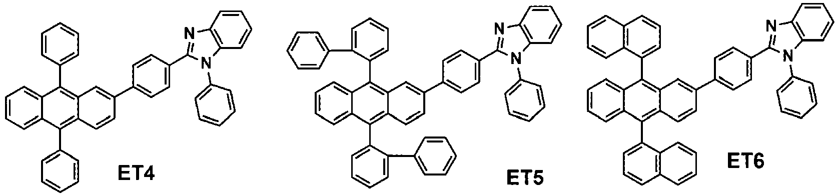

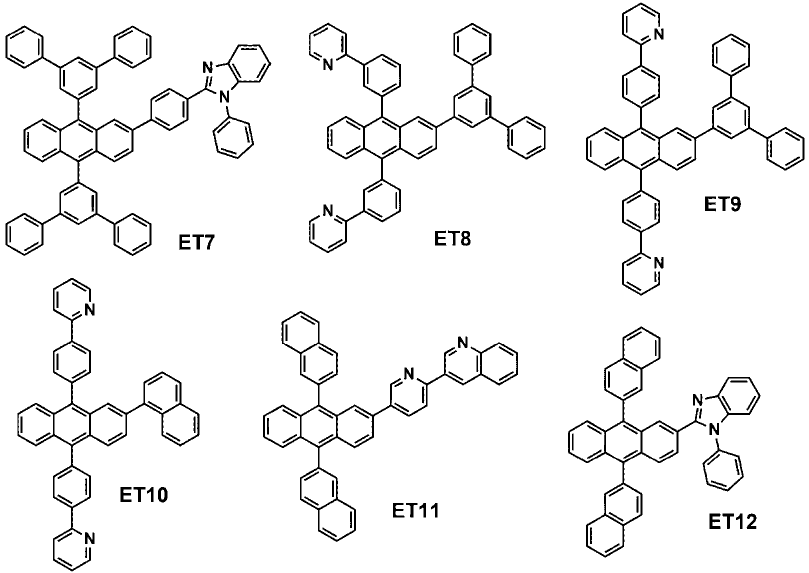

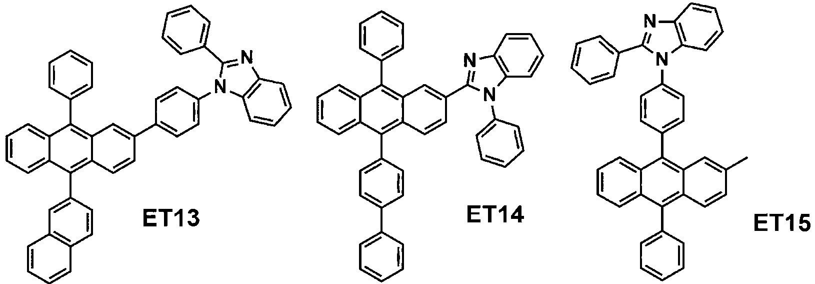

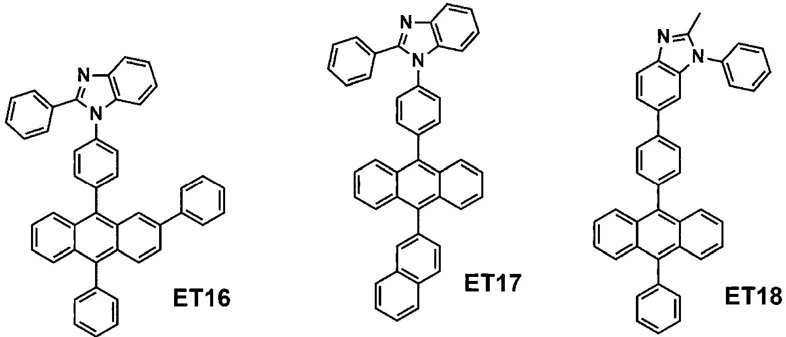

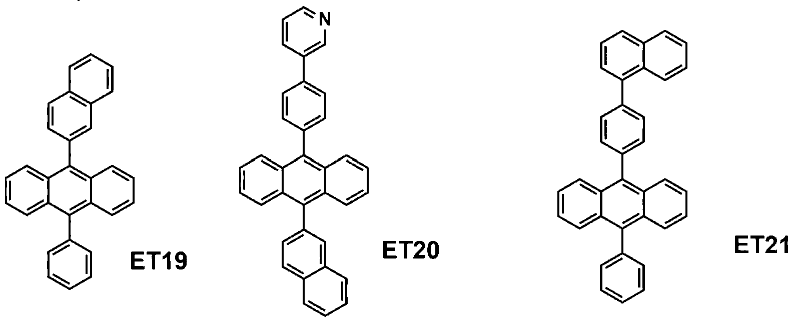

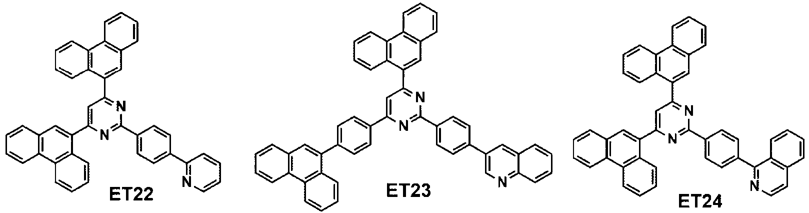

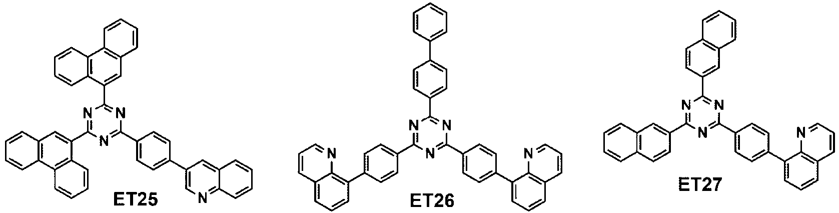

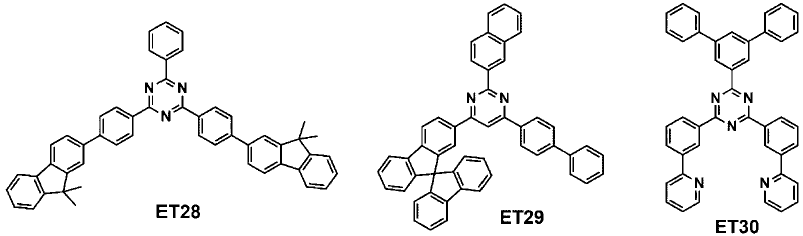

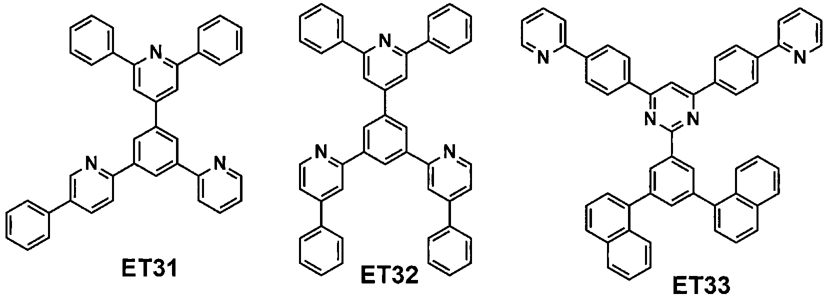

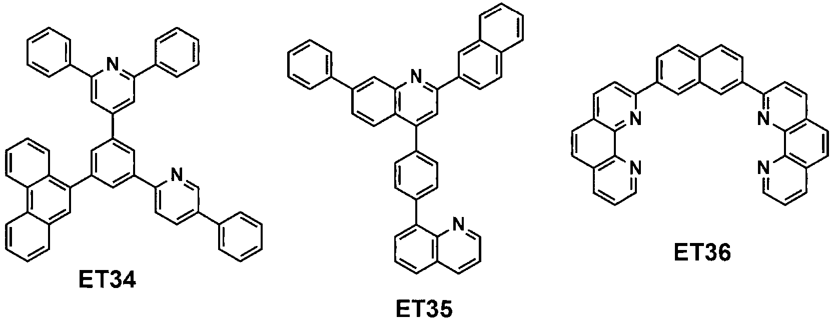

In addition, the electron transport region ETR may include halogenated metals such as LiF, NaCl, CsF, RbCl, RbI, Cul, and KI, lanthanide metals such as Yb, or co-deposition materials of a halogenated metal and a lanthanide metal. For example, the electron transport region ETR may include KI:Yb, RbI:Yb, LiF:Yb, and the like as a co-deposition material. Meanwhile, for the electron transport region ETR, a metal oxide such as Li₂O and BaO, 8-hydroxyl-lithium quinolate (Liq), or the like may be used, but an aspect is not limited thereto. The electron transport region ETR may also be formed of a mixture material of an electron transport material and an insulating organo-metal salt. The organo metal salt may be a material having an energy band gap of about 4 eV or greater. Specifically, the organo-metal salt may include, for example, metal acetates, metal benzoates, metal acetoacetates, metal acetylacetonates, or metal stearates.

The electron transport region ETR may further include, for example, at least one of 2,9-dimethyl-4,7-diphenyl-1,10-phenanthroline (BCP), diphenyl(4-(triphenylsilyl)phenyl)phosphine oxide (TSPO1), or 4,7-diphenyl-1,10-phenanthroline (Bphen) in addition to the materials described above, but an aspect is not limited thereto.

The electron transport region ETR may include the compounds of the electron transport region described above in at least one among the electron injection layer EIL, the electron transport layer ETL, and the hole blocking layer HBL.

When the electron transport region ETR includes the electron transport layer ETL, the electron transport layer ETL may have a thickness of about 100 Å to about 1000 Å, for example, about 150 Å to about 500 Å. When the thickness of the electron transport layer ETL satisfies the above-described range, satisfactory electron transport properties may be obtained without a substantial increase in driving voltage. When the electron transport region ETR includes the electron injection layer EIL, the electron injection layer EIL may have a thickness of about 1 Å to about 100 Å, for example, about 3 Å to about 90 Å. When the thickness of the electron injection layer EIL satisfies the above-described ranges, satisfactory electron injection properties may be obtained without a substantial increase in driving voltage.

The second electrode EL2 is provided on the electron transport region ETR. The second electrode EL2 may be a common electrode. The second electrode EL2 may be a cathode or an anode but an aspect is not limited thereto. For example, when the first electrode EL1 is an anode, the second electrode EL2 may be a cathode, and when the first electrode EL1 is a cathode, the second electrode EL2 may be an anode.

The second electrode EL2 may be a transmissive electrode, a transflective electrode, or a reflective electrode. When the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), and the like.

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, Na, a compound thereof, or a mixture thereof (e.g., AgMg, AgYb, MgYb, AgLi, or AgNa). Alternatively, the second electrode EL2 may have a multilayer structure including a reflective film or a transflective film formed of the above-described materials, and a transparent conductive film formed of indium tin oxide (ITO), indium zinc oxide (IZO), zinc oxide (ZnO), indium tin zinc oxide (ITZO), and the like. For example, the second electrode EL2 may include the above-described metal materials, a combination of two or more metal materials selected from the above-described metal materials, or oxides of the above-described metal materials.

Although not shown, the second electrode EL2 may be connected with an auxiliary electrode. When the second electrode EL2 is connected with the auxiliary electrode, the resistance of the second electrode EL2 may decrease.

Meanwhile, a capping layer CPL may be further disposed on the second electrode EL2 of the light emitting element ED of an aspect. The capping layer CPL may include a multi-layer or a single layer.

In an aspect, the capping layer CPL may be an organic layer or an inorganic layer. For example, when the capping layer CPL includes an inorganic material, the inorganic material may include an alkali metal compound such as LiF, an alkaline earth metal compound such as MgF₂, SiON, SiNₓ, SiO_{y}, and the like.

For example, when the capping layer CPL includes an organic material, the organic material may include α-NPD, NPB, TPD, m-MTDATA, Alq₃ CuPc, N4,N4,N4',N4'-tetra(biphenyl-4-yl) biphenyl-4,4'-diamine (TPD15), 4,4',4"-tris(carbazol-9-yl) triphenylamine (TCTA), and the like or may include epoxy resins or acrylates such as methacrylates. However, an aspect is not limited thereto, and the capping layer CPL may include at least one of compounds P1 to P5 below.

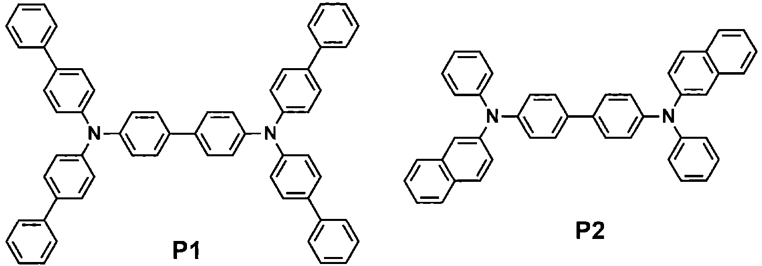

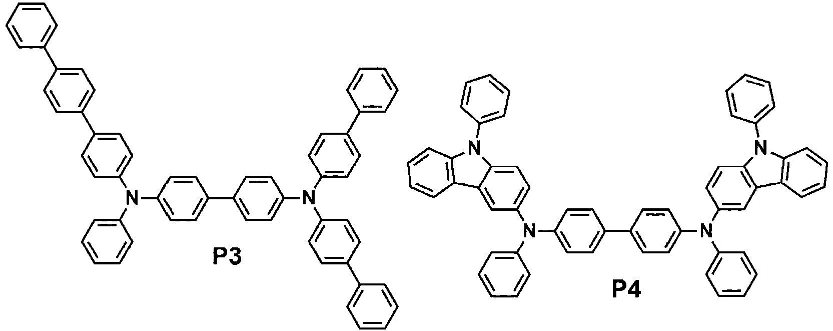

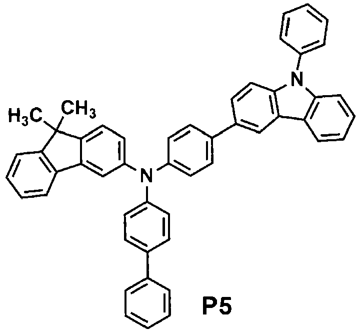

Meanwhile, the capping layer CPL may have a refractive index of about 1.6 or greater. To be specific, the capping layer CPL may have a refractive index of about 1.6 or greater in a wavelength range of about 550 nm to about 660 nm.

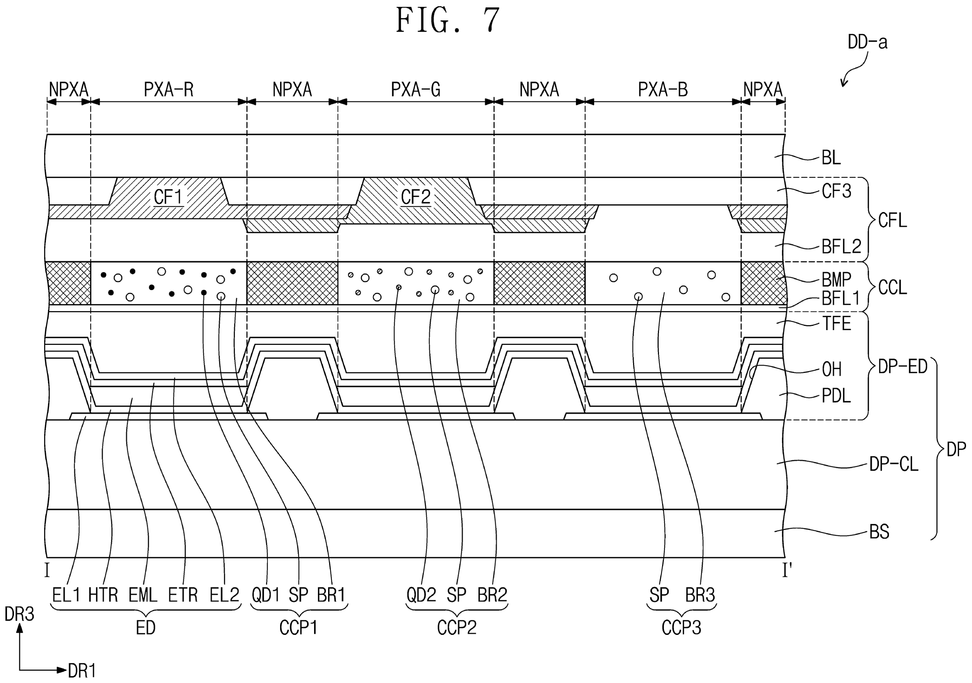

FIGS. 7 to 10 are each a cross-sectional view of a display device according to an aspect. Hereinafter, in the description of the display device according to an aspect with reference to FIGS. 7 to 10, content overlapping the one described above with reference to FIGS. 1 to 6 will not be described again, and the differences will be mainly described.

Referring to FIG. 7, a display device DD-a according to an aspect may include a display panel DP having a display element layer DP-ED, a light control layer CCL disposed on the display panel DP, and a color filter layer CFL. In an aspect shown in FIG. 7, the display panel DP may include a base layer BS, a circuit layer DP-CL provided on the base layer BS, and a display element layer DP-ED, and the display element layer DP-ED may include a light emitting element ED.

The light emitting element ED includes a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. Meanwhile, a structure of the light emitting element ED shown in FIG. 7 may be the same as the structure of the light emitting element ED of FIGS. 3 to 6 described above. The light emitting element ED shown in FIG. 7 includes a monoamine compound of an aspect. The light emitting element ED including the monoamine compound of an aspect may exhibit low driving voltage, high luminous efficacy, and/or long life characteristics.

Referring to FIG. 7, the emission layer EML may be disposed in the openings OH defined in the pixel defining film PDL. For example, the emission layer EML separated by the pixel defining film PDL and provided corresponding to each of light emitting regions PXA-R, PXA-G, and PXA-B may emit light in the same wavelength ranges. In the display device DD-a of an aspect, the emission layer EML may emit blue light. Meanwhile, unlike what is shown, in an aspect, the emission layer EML may be provided as a common layer throughout the light emitting regions PXA-R, PXA-G, and PXA-B.

The light control layer CCL may be disposed on the display panel DP. The light control layer CCL may include a light converter. The light converter may be a quantum dot or a phosphor. The light converter may wavelength-convert the provided light and emit the wavelength-converted light. That is, the light control layer CCL may be a layer containing quantum dots or phosphors.

The light control layer CCL may include a plurality of light control units CCP1, CCP2, and CCP3. The light control units CCP1, CCP2, and CCP3 may be spaced apart from one another.

Referring to FIG. 7, a division pattern BMP may be disposed between the light control units CCP1, CCP2, and CCP3 spaced apart from each other, but an aspect is not limited thereto. In FIG. 7, the division pattern BMP is shown to nonoverlap the light control units CCP1, CCP2, and CCP3, but edges of the light control units CCP1, CCP2, and CCP3 may overlap at least a portion of the division pattern BMP.

The light control layer CCL may include a first light control unit CCP1 including a first quantum dot QD1 for converting first color light provided from the light emitting element ED into second color light, a second light control unit CCP2 including a second quantum dot QD2 for converting the first color light into third color light, and a third light control unit CCP3 transmitting the first color light.

In an aspect, the first light control unit CCP1 may provide red light, which is the second color light, and the second light control unit CCP2 may provide green light, which is the third color light. The third light control unit CCP3 may transmit and provide blue light, which is the first color light provided from the light emitting element ED. For example, the first quantum dot QD1 may be a red quantum dot and the second quantum dot QD2 may be a green quantum dot. The same descriptions above may be applied to the quantum dots QD1 and QD2.

In addition, the light control layer CCL may further include scatterers SP. The first light control unit CCP1 may include the first quantum dot QD1 and the scatterers SP, the second light control unit CCP2 may include the second quantum dot QD2 and the scatterers SP, and the third light control unit CCP3 may not include a quantum dot but may include the scatterers SP.

The scatterers SP may be inorganic particles. For example, the scatterers SP may include at least one among TiO₂, ZnO, Al₂O₃, SiO₂, and hollow silica. The scatterers SP may include any one of TiO₂, ZnO, Al₂O₃, SiO₂, and hollow silica, or may be a mixture of two or more materials selected from TiO₂, ZnO, Al₂O₃, SiO₂, and hollow silica.

The first light control unit CCP1, the second light control unit CCP2, and the third light control unit CCP3 may each include base resins BR1, BR2, and BR3 for dispersing the quantum dots QD1 and QD2 and the scatterers SP. In an aspect, the first light control unit CCP1 may include the first quantum dot QD1 and the scatterers SP dispersed in the first base resin BR1, the second light control unit CCP2 may include the second quantum dot QD2 and the scatterers SP dispersed in the second base resin BR2, and the third light control unit CCP3 may include the scatterers SP dispersed in the third base resin BR3.

The base resins BR1, BR2, and BR3 are a medium in which the quantum dots QD1 and QD2 and the scatterers SP are dispersed, and may be formed of various resin compositions, which may be generally referred to as a binder. For example, the base resins BR1, BR2, and BR3 may be an acrylic-based resin, a urethane-based resin, a silicone-based resin, an epoxy-based resin, and the like. Base resins BR1, BR2, and BR3 may be transparent resins. In an aspect, the first base resin BR1, the second base resin BR2, and the third base resin BR3 may each be the same as or different from each other.

The light control layer CCL may include a barrier layer BFL1. The barrier layer BFL1 may serve to prevent moisture and/or oxygen (hereinafter referred to as "moisture/oxygen") from being introduced. The barrier layer BFL1 may be disposed on the light control units CCP1, CCP2, and CCP3 to prevent the light control units CCP1, CCP2, and CCP3 from being exposed to moisture/oxygen. Meanwhile, the barrier layer BFL1 may cover the light control units CCP1, CCP2, and CCP3. In addition, a barrier layer BFL2 may be provided between the light control units CCP1, CCP2, and CCP3 and the color filter layer CFL.

The barrier layers BFL1 and BFL2 may include at least one inorganic layer. That is, the barrier layers BFL1 and BFL2 may be formed of an inorganic material. For example, the barrier layers BFL1 and BFL2 may be formed by including silicon nitride, aluminium nitride, zirconium nitride, titanium nitride, hafnium nitride, tantalum nitride, silicon oxide, aluminium oxide, titanium oxide, tin oxide, cerium oxide, silicon oxynitride, or a metal thin film in which light transmittance is secured, and the like. Meanwhile, the barrier layers BFL1 and BFL2 may further include an organic film. The barrier layers BFL1 and BFL2 may be formed of a single layer or a plurality of layers.

In the display device DD-a of an aspect, the color filter layer CFL may be disposed on the light control layer CCL. For example, the color filter layer CFL may be directly disposed on the light control layer CCL. In this case, the barrier layer BFL2 may be omitted.

The color filter layer CFL may include filters CF1, CF2, and CF3. That is, the color filter layer CFL may include a first filter CF1 transmitting second color light, a second filter CF2 transmitting third color light, and a third filter CF3 transmitting first color light. For example, the first filter CF1 may be a red filter, the second filter CF2 may be a green filter, and the third filter CF3 may be a blue filter. The filters CF1, CF2, and CF3 may each include a polymer photosensitive resin, a pigment or a dye. The first filter CF1 may include a red pigment or a red dye, the second filter CF2 may include a green pigment or a green dye, and the third filter CF3 may include a blue pigment or a blue dye.

Meanwhile, an aspect is not limited thereto, and the third filter CF3 may not include a pigment or a dye. The third filter CF3 may include a polymer photosensitive resin, but not include a pigment or a dye. The third filter CF3 may be transparent. The third filter CF3 may be formed of a transparent photosensitive resin.

In addition, in an aspect, the first filter CF1 and the second filter CF2 may be yellow filters. The first filter CF1 and the second filter CF2 may not be separated and may be provided as a single body.

Although not shown, the color filter layer CFL may further include a light blocking unit (not shown). The light blocking unit may be a black matrix. The light blocking unit may be formed by including an organic light blocking material or an inorganic light blocking material, both including a black pigment or a black dye. The light blocking unit may prevent light leakage, and separate boundaries between the adjacent filters CF1, CF2, and CF3. In addition, in an aspect, the light blocking unit may be formed with a blue filter.

The first to third filters CF1, CF2, and CF3 may be disposed corresponding to the red light emitting region PXA-R, the green light emitting region PXA-G, and the blue light emitting region PXA-B, respectively.

The base substrate BL may be disposed on the color filter layer CFL. The base substrate BL may be a member providing a base surface on which the color filter layer CFL and the light control layer CCL are disposed. The base substrate BL may be a glass substrate, a metal substrate, a plastic substrate, and the like. However, an aspect is not limited thereto, and the base substrate BL may be an inorganic layer, an organic layer, or a composite material layer. In addition, unlike what is shown, the base substrate BL may be omitted in an aspect.

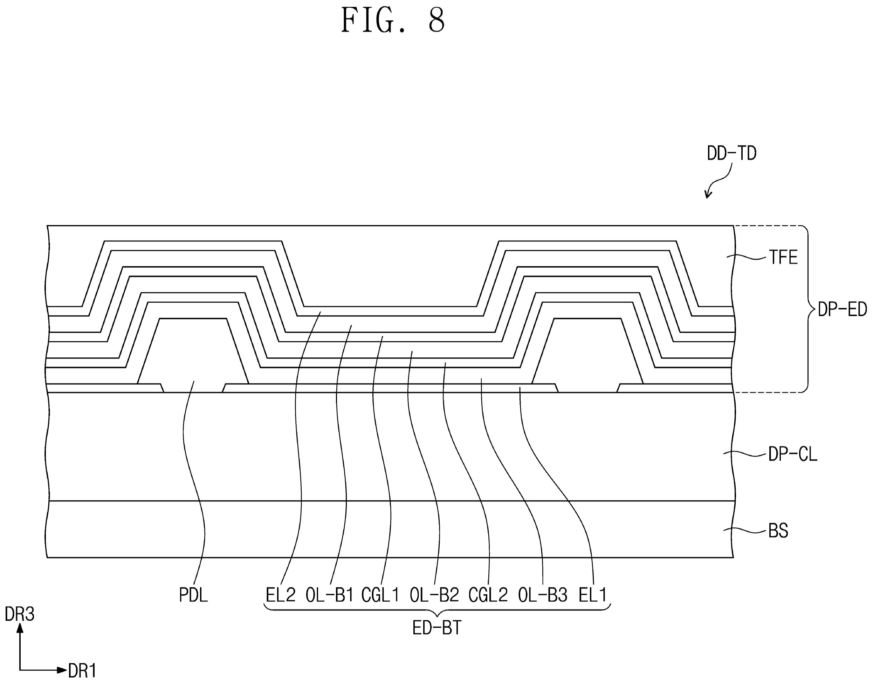

FIG. 8 is a cross-sectional view showing a portion of a display device according to an aspect. In a display device DD-TD of an aspect, a light emitting element ED-BT may include a plurality of light emitting structures OL-B1, OL-B2, and OL-B3. The light emitting element ED-BT may include the first electrode EL1 and the second electrode EL2 facing each other, and the plurality of light emitting structures OL-B1, OL-B2, and OL-B3 provided by being sequentially stacked in a thickness direction between the first electrode EL1 and the second electrode EL2. The light emitting structures OL-B1, OL-B2, and OL-B3 each may include the emission layer EML (FIG. 7), a hole transport region HTR and an electron transport region ETR disposed with the emission layer EML (FIG. 7) therebetween. That is, the light emitting element ED-BT included in the display device DD-TD of an aspect may be a light emitting element having a tandem structure including a plurality of emission layers.

The light emitting element ED-BT shown in FIG. 8 includes a monoamine compound of an aspect. The light emitting element ED-BT including the monoamine compound of an aspect may exhibit low driving voltage, high luminous efficacy, and/or long life characteristics.

In an aspect shown in FIG. 8, light emitted from each of the light emitting structures OL-B1, OL-B2, and OL-B3 may all be blue light. However, an aspect is not limited thereto, and wavelength ranges of light emitted from each of the light emitting structures OL-B1, OL-B2, and OL-B3 may be different from each other. For example, the light emitting element ED-BT including the plurality of light emitting structures OL-B1, OL-B2, and OL-B3 emitting light in different wavelength ranges may emit white light.

Charge generation layers CGL1 and CGL2 may be disposed between neighboring light emitting structures OL-B1, OL-B2, and OL-B3. The charge generation layers CGL1 and CGL2 may include a p-type charge generation layer and/or an n-type charge generation layer.

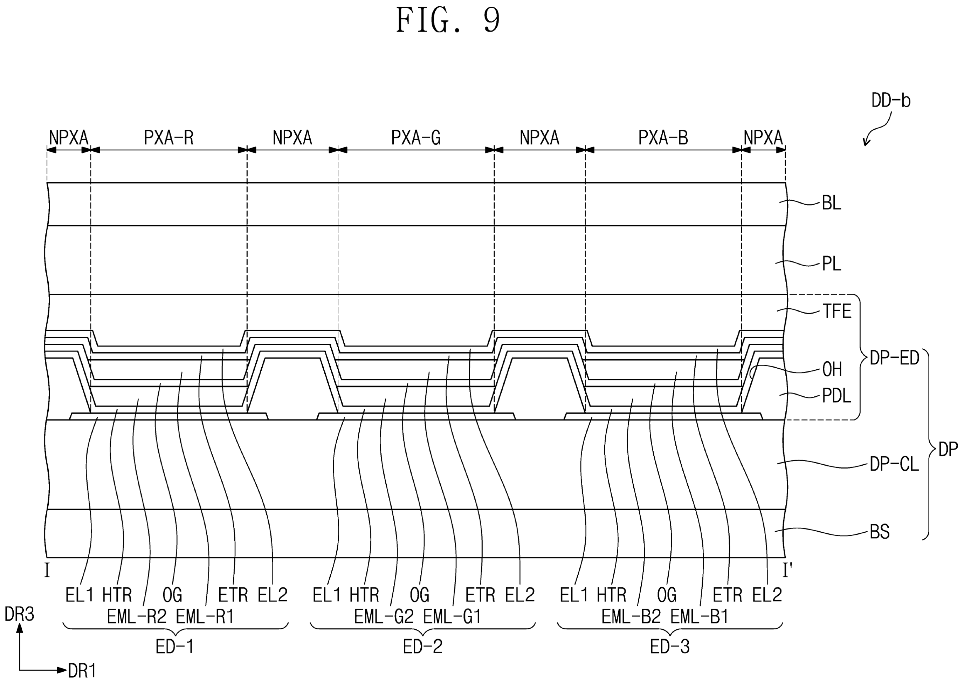

Referring to FIG. 9, a display device DD-b according to an aspect may include light emitting elements ED-1, ED-2, and ED-3 in which two emission layers are stacked. Compared to the display device DD according to an aspect shown in FIG. 2, the difference is that in an aspect shown in FIG. 9, the first to third light emitting elements ED-1, ED-2, and ED-3 each include two emission layers stacked in a thickness direction. In each of the first to third light emitting elements ED-1, ED-2, and ED-3, the two emission layers may emit light in the same wavelength range. At least one of the first to third light emitting elements ED-1, ED-2, or ED-3 includes the monoamine compound of an aspect. A light emitting element (at least one of ED-1, ED-2, or ED-3) including the monoamine compound of an aspect may exhibit low driving voltage, high luminous efficacy, and/or long life characteristics.

The first light emitting element ED-1 may include a first red emission layer EML-R1 and a second red emission layer EML-R2. The second light emitting element ED-2 may include a first green emission layer EML-G1 and a second green emission layer EML-G2. In addition, the third light emitting element ED-3 may include a first blue emission layer EML-B1 and a second blue emission layer EML-B2. A light emitting auxiliary portion OG may be disposed between the first red emission layer EML-R1 and the second red emission layer EML-R2, between the first green emission layer EML-G1 and the second green emission layer EML-G2, and between the first blue emission layer EML-B1 and the second blue emission layer EML-B2.

The light emitting auxiliary portion OG may include a single layer or multiple layers. The light emitting auxiliary portion OG may include a charge generation layer. To be more specific, the light emitting auxiliary portion OG may include an electron transport region, a charge generation layer, and a hole transport region that are sequentially stacked. The light emitting auxiliary portion OG may be provided as a common layer throughout the first to third light emitting elements ED-1, ED-2, and ED-3. However, an aspect is not limited thereto, and the light emitting auxiliary portion OG may be provided to be patterned inside the openings OH defined in the pixel defining film PDL.

The first red emission layer EML-R1, the first green emission layer EML-G1, and the first blue emission layer EML-B1 may be disposed between the light emitting auxiliary portion OG and the electron transport region ETR. The second red emission layer EML-R2, the second green emission layer EML-G2, and the second blue emission layer EML-B2 may be disposed between the hole transport region HTR and the light emitting auxiliary portion OG.

That is, the light emitting element ED-1 may include the first electrode EL1, the hole transport region HTR, the second red emission layer EML-R2, the emission auxiliary portion OG, the first red emission layer EML-R1, the electron transport region ETR, and the second electrode EL2, which are sequentially stacked. The second light emitting element ED-2 may include the first electrode EL1, the hole transport region HTR, the second green emission layer EML-G2, the emission auxiliary portion OG, the first green emission layer EML-G1, the electron transport region ETR, and the second electrode EL2, which are sequentially stacked. The third light emitting element ED-3 may include the first electrode EL1, the hole transport region HTR, the second blue emission layer EML-B2, the emission auxiliary portion OG, the first blue emission layer EML-B1, the electron transport region ETR, and the second electrode EL2, which are sequentially stacked.

Meanwhile, an optical auxiliary layer PL may be disposed on the display element layer DP-ED. The optical auxiliary layer PL may include a polarizing layer. The optical auxiliary layer PL may be disposed on the display panel DP to control reflected light in the display panel DP due to external light. Unlike what is shown, the optical auxiliary layer PL may be omitted in the display device according to an aspect.

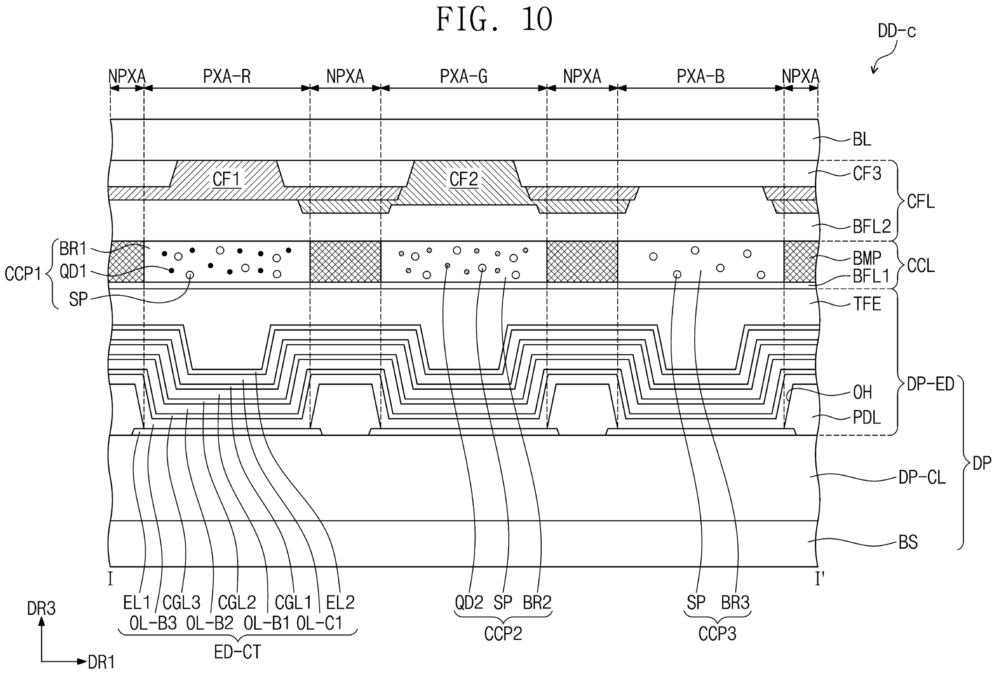

Unlike FIGS. 8 and 9, a display device DD-c of FIG. 10 is shown to include four light emitting structures OL-B1, OL-B2, OL-B3, and OL-C1. The light emitting element ED-CT may include the first electrode EL1 and the second electrode EL2 facing each other, and the first to fourth light emitting structures OL-B1, OL-B2, OL-B3, and OL-C1 sequentially stacked in a thickness direction between the first electrode EL1 and the second electrode EL2. Charge generation layers CGL1, CGL2, and CGL3 may be disposed between the first to fourth light emitting structures OL-B1, OL-B2, OL-B3, and OL-C1. The light emitting element ED-CT shown in FIG. 10 includes a monoamine compound of an aspect. The light emitting element ED-CT including the monoamine compound of an aspect may exhibit low driving voltage, high luminous efficacy, and/or long life characteristics.

Among the four light emitting structures, the first to third light emitting structures OL-B1, OL-B2, and OL-B3 may emit blue light, and the fourth light emitting structure OL-C 1 may emit green light. However, an aspect is not limited thereto, and the first to fourth light emitting structures OL-B1, OL-B2, OL-B3, and OL-C1 may emit light having different wavelength ranges.

The charge generation layers CGL1, CGL2 and CGL3 disposed between the neighboring light emitting structures OL-C1, OL-B1, OL-B2, and OL-B3 may include a p-type charge generation layer and/or an n-type charge generation layer.

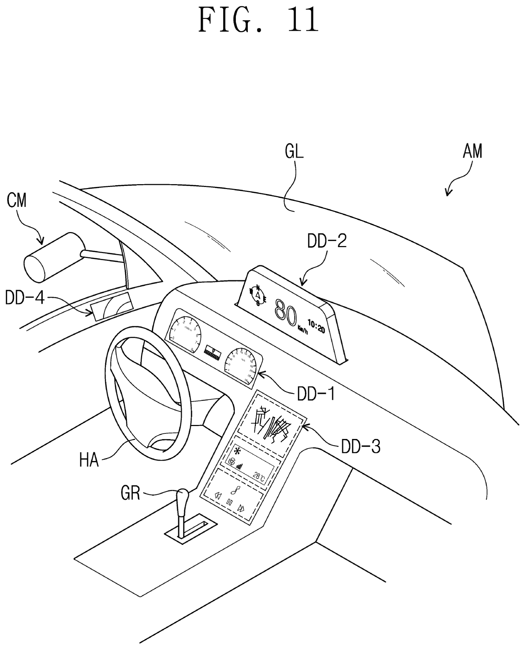

FIG. 11 is a view showing a vehicle AM in which first to fourth display devices DD-1, DD-2, DD-3, and DD-4 are disposed. At least one of the first to fourth display devices DD-1, DD-2, DD-3, and DD-4 may have the same configuration as the display devices DD, DD-TD, DD-a, DD-b, and DD-c of an aspect described with reference to FIGS. 1, 2, and 7 to 10.

FIG. 11 shows a car as the vehicle AM, but this is presented as an example, and the first to fourth display devices DD-1, DD-2, DD-3, and DD-4 may be disposed on other means of transportation, such as bicycles, motorcycles, trains, ships, and airplanes. In addition, at least one of the first to fourth display devices DD-1, DD-2, DD-3, and DD-4 having the same configuration as the display devices DD, DD-TD, DD-a, DD-b, and DD-c of an aspect may be adopted for personal computers, laptop computers, personal digital terminals, game consoles, portable electronic devices, an in- vehicle display device, a camera, televisions, monitors, outdoor billboards, and the like. In addition, these are merely presented as an aspect, and thus the display device may be adopted for other electronic apparatuses without departing from the present disclosure.

At least one of the first to fourth display devices DD-1, DD-2, DD-3, or DD-4 may include the light emitting element ED described with reference to FIGS. 3 to 6. At least one of the first to fourth display devices DD-1, DD-2, DD-3, or DD-4 includes a monoamine compound of an aspect. A display device (at least one of DD-1, DD-2, DD-3, or DD-4) including the monoamine compound of an aspect may exhibit excellent display efficiency and display lifespan.

Referring to FIG. 11, the vehicle AM may include a wheel HA and a gear GR for operating the vehicle AM, and have a front window GL disposed to face a driver.

The first display device DD-1 may be disposed in a first region overlapping the wheel HA. For example, the first display device DD-1 may be a digital cluster displaying first information of the vehicle AM. The first information may include a first scale indicating driving speed of the vehicle AM, a second scale indicating engine revolutions (i.e., revolutions per minute (RPM)), and an image indicating fuel gauge, and the like. The first scale and the second scale may be displayed as digital images. Unlike what is shown, the second information of the second display device DD-2 may be projected and displayed on the front window GL.

The second display device DD-2 may be disposed in a second region facing a driver seat and overlapping the front window GL. The driver seat may be a seat in which the wheel HA is disposed. For example, the second display device DD-2 may be a head up display HUD displaying second information of the vehicle AM. The second display device DD-2 may be optically transparent. The second information includes a digital number indicating driving speed of the vehicle AM and may further include information such as current time.

The third display device DD-3 may be disposed in a third region adjacent to the gear GR. For example, the third display device DD-3 may be a center information display (CID) for a vehicle, which is disposed between a driver seat and a front passenger seat, and displays third information. The passenger seat may be a seat spaced apart from the driver seat with the gear GR therebetween. The third information may include information about road conditions (e.g., navigation information), music or radio play, dynamic video play, temperature inside the vehicle AM, and the like.

The fourth display device DD-4 may be disposed in a fourth region spaced apart from the wheel HA and the gear GR and adjacent to a side of the vehicle AM. For example, the fourth display device DD-4 may be a digital side mirror displaying fourth information. The fourth display device DD-4 may display images of conditions outside the vehicle AM, which are taken by a camera module CM disposed outside the vehicle AM. The fourth information may include images of conditions outside the vehicle AM.

The first to fourth information described above are presented as an example, and the first to fourth display devices DD-1, DD-2, DD-3, and DD-4 may further display information about inside or outside a vehicle. The first to fourth information may include different information. However, an aspect is not limited thereto, and some of the first to fourth information may include the same information.

In an aspect, an electronic apparatus may include a display device including a plurality of light emitting elements, and a control portion configured to control the display device. The electronic apparatus of an aspect may be a device activated in response to electrical signals and may provide images. The electronic apparatus may include display devices of various aspects. For example, the display device may include large-sized display devices such as televisions, monitors, or outdoor billboards, as well as small- and medium-sized display devices such as personal computers, laptop computers, personal digital terminals, in-vehicle display devices, game consoles, portable electronic devices, or cameras.

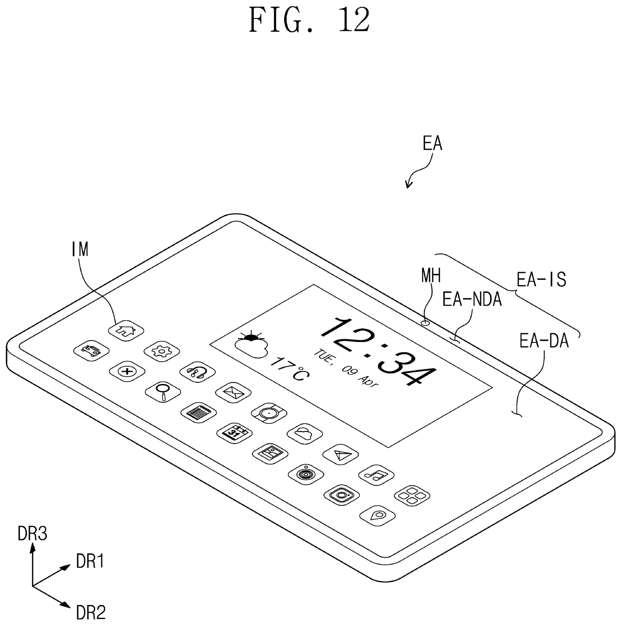

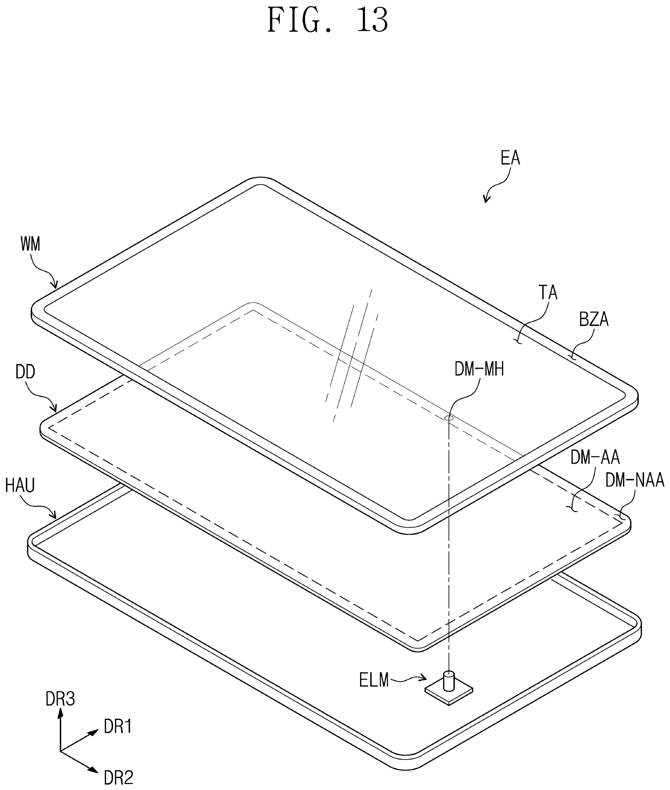

FIG. 12 is a perspective view showing an electronic apparatus of an aspect. FIG. 13 is an exploded perspective view showing an electronic apparatus of an aspect.

In FIG. 12, a portable electronic device is presented as an example of the electronic apparatus EA. The electronic apparatus EA may display an image IM through a display surface EA-IS. The image IM may include still images as well as dynamic images. The display surface EA-IS may be parallel to a plane defined by the first directional axis DR1 and the second directional axis DR2. FIG. 12 shows the electronic apparatus EA provided with the flat display surface EA-IS, but an aspect is not limited thereto. For example, the electronic apparatus EA may include a curved display surface or a three-dimensional display surface. The three-dimensional display surface may include a plurality of display regions indicating different directions.

The display surface EA-IS may include a display region EA-DA and a non-display region EA-NDA. The display surface EA-IS may further include a sub region MH. The electronic apparatus EA may display the image IM through the display region EA-DA.

The non-display region EA-NDA may have a predetermined color. The non-display region EA-NDA may be adjacent to the display region EA-DA. The non-display region EA-NDA may surround the display region EA-DA. Accordingly, the shape of the display region EA-DA may be defined substantially by the non-display region EA-NDA. However, FIG. 12 is presented as an example, and the non-display region EA-NDA may be disposed adjacent to only one side of the display region EA-DA, or may not be provided.

The sub region MH may detect an external subject received through the display surface EA-IS, or provide sound signals such as voice to the outside through the display surface EA-IS. A light signal such as visible light or infrared light may travel to the sub region MH.

The sub region MH may be disposed within the display region EA-DA. However, this is presented as an example, and the arrangement of the sub region MH is not limited to any one aspect. For example, the sub region MH may be surrounded by the non-display region EA-NDA, or may be surrounded by the display region EA-DA and the non-display region EA-NDA. Although one sub region MH is shown in FIG. 12, a plurality of sub regions MH may be provided.

Various electronic modules ELM (FIG. 13) may be disposed to correspond to the sub regions MH. For example, the electronic modules ELM (FIG. 13) may include at least one of a camera, a speaker, a light detection sensor, or a heat detection sensor. The electronic apparatus EA may include an electronic module ELM (FIG. 13) that captures external images via visible light passing through the sub regions MH or determines the accessibility of external objects via infrared light. The electronic modules ELM (FIG. 13) may include a plurality of components, and are not limited to any one aspect.

Referring to FIG. 13, the electronic apparatus EA includes a display device DD. In addition, the electronic apparatus EA may further include an electronic module ELM, a window member WM, and a housing HAU.

The window member WM may cover an entire outer portion of the electronic apparatus EA. The window member WM may include a transmission region TA and a bezel region BZA. A front surface of the window member WM including the transmission region TA and the bezel region BZA may serve as a front surface of the electronic apparatus EA. The transmission region TA may correspond to the display region EA-DA of the electronic apparatus EA shown in FIG. 12, and the bezel region BZA may correspond to the non-display region EA-NDA of the electronic apparatus EA shown in FIG. 12.

The transmission region TA may be an optically transparent region. The bezel region BZA may be a region having a relatively lower light transmittance than the transmission region TA. The bezel region BZA may have a predetermined color. The bezel region BZA may be adjacent to the transmission region TA and may surround the transmission region TA. The bezel region BZA may define a shape of the transmission region TA. However, an aspect is not limited to what is shown, and the bezel region BA may be disposed adjacent to only one side of the transmission region TA, and a portion thereof may not be provided.

The housing HAU may include a material having a relatively greater rigidity. For example, the housing HAU may include frames and/or plates formed of glass, plastic, or metal. The frames and/or plates may be provided in plurality. The housing HAU may provide a predetermined place for accommodation. The display device DD may be accommodated in the accommodation place to be protected from external shocks.

The display device DD may have the same configuration as at least one of the display devices DD, DD-TD, DD-a, DD-b, or DD-c of an aspect described with reference to FIGS. 1, 2, and 7 to 10. The display device DD may include the light emitting element ED described with reference to FIGS. 3 to 6. Accordingly, the electronic apparatus EA including the display device DD according to an aspect may exhibit excellent reliability.

The display device DD may define an active region DM-AA, a peripheral region DM-NAA, and a module region DM-MH. The active region DM-AA may overlap the display region EA-DA shown in FIG. 12, and the peripheral region DM-NAA may overlap the non-display region EA-NDA shown in FIG. 12. The module region DM-MH may overlap the sub region MH shown in FIG. 12.

The active region DM-AA may be a region activated according to electrical signals. The peripheral region DM-NAA may be a region adjacent to at least one side of the active region DM-AA. The active region DM-AA may include a non-light emitting region NPXA and light emitting regions PXA-R, PXA-G, and PXA-R shown in FIG. 1. The peripheral region DM-NAA may surround the active region DM-AA. However, an aspect is not limited thereto, and a portion of the peripheral region DM-NAA may be omitted unlike what is shown. A driving circuit, a driving line, or the like for driving the active region DM-AA may be disposed in the peripheral region DM-NAA.

Light signals such as visible light or infrared light may travel through the module region DM-MH. The module region DM-MH may be disposed in the active region DM-AA. Alternatively, the module region DM-MH may be surrounded by the peripheral region DM-NAA, or may be surrounded by the active region DM-AA and the peripheral region DM-NAA.

The electronic module ELM may be an electronic component that outputs or receives light signals. The electronic module ELM may include a camera module and/or a proximity sensor. The camera module may capture an external image through the module region DM-MH. However, an aspect is not limited thereto, and the electronic module ELM may further include a built-in module and/or an external module. The built-in module may include a sensor module, an antenna module, and an audio output module. The external module may include a light module and a communication module.

Display devices according to aspects DD, DD-TD, DD-a, DD-b, and DD-c (FIGS. 1, 2, and 7 to 10) may be applied to various electronic apparatuses. An electronic apparatus EA according to an aspect may include the display devices DD, DD-TD, DD-a, DD-b, and DD-c (FIGS. 1, 2, and 7 to 10) described above, and may further include modules or devices having additional functions in addition to the display devices DD, DD-TD, DD-a, DD-b, and DD-c (FIGS. 1, 2, and 7 to 10).

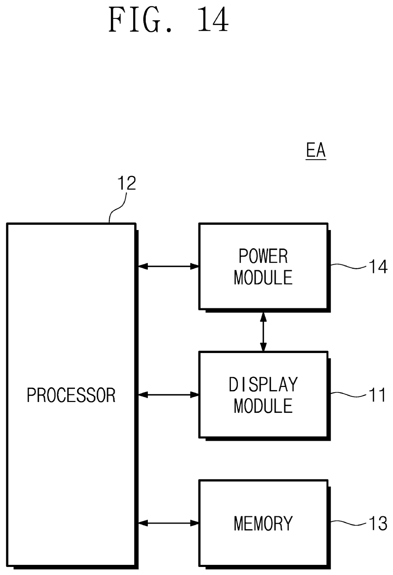

FIG. 14 is a block diagram of an electronic apparatus according to an aspect. Referring to FIG. 14, an electronic apparatus EA according to an aspect may include a display module 11, a processor 12, a memory 13, and a power module 14.

The processor 12 may include at least one of a central processing unit (CPU), an application processor (AP), a graphic processing unit (GPU), a communication processor (CP), an image signal processor (ISP), or a controller.

The memory 13 may store data information required for the operation of the processor 12 or the display module 11. When the processor 12 executes an application stored in the memory 13, image data signals and/or input control signals are transmitted to the display module 11, and the display module 11 may process the received signals and output image information through a display screen.

The power module 14 may include a power supply module such as a power adapter or a battery device, and a power conversion module that converts power supplied by the power supply module to generate power required for the operation of the electronic apparatus EA.

At least one of the components of the electronic apparatus EA described above may be included in the display device according to the above-described aspects. In addition, some of the individual modules functionally included in one module may be included in the display device, and others may be separately provided from the display device. For example, the display device may include a display module 11, and the processor 12, the memory 13 and the power module 14 may be provided in the form of other devices within the electronic apparatus EA, rather than the display device.

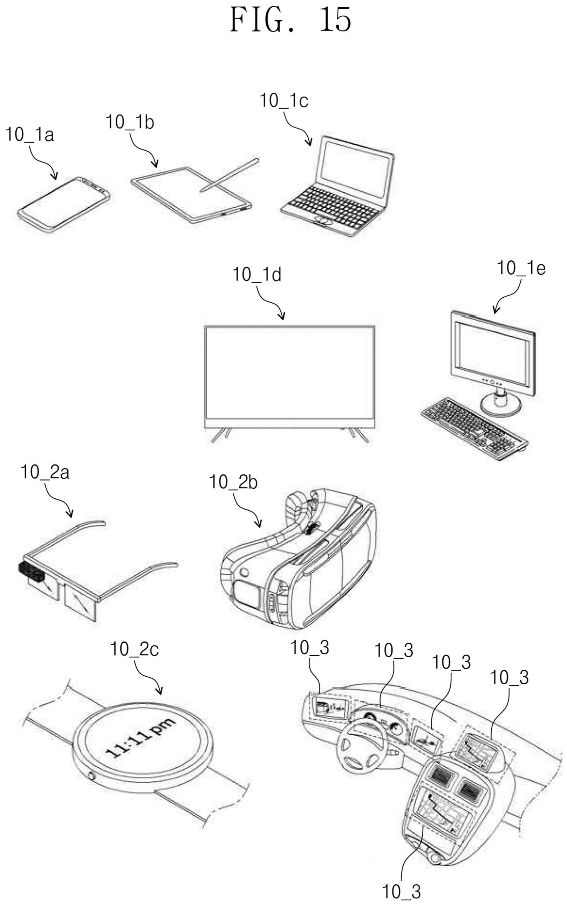

FIG. 15 shows schematic views of electronic apparatuses according to various aspects. Referring to FIG. 15, various electronic apparatuses to which a display device according to aspects is applied may include an electronic apparatus for displaying images, such as a smart phone 10_1a, a tablet PC 10_1b, a laptop 10_1c, a TV 10_1d, and a desk monitor 10_1e, a wearable electronic apparatus including a display module such as a smart glasses 10_2a, a head mounted display 10_2b, and a smart watch 10_2c, and a vehicle electronic apparatus 10_3 including a display module such as a center information display (CID) and a room mirror display disposed on an instrument panel, a center fascia, or a dashboard of a vehicle.

Hereinafter, with reference to Examples and Comparative Examples, a monoamine compound according to an aspect of the present disclosure and a light emitting element of an aspect will be specifically described. In addition, Examples below are shown only for the understanding of the present disclosure, and the scope of the present disclosure is not limited thereto.

### [Examples]

### 1. Synthesis of a monoamine compound of an aspect

A process of synthesizing monoamine compounds according to an aspect of the present disclosure will be described in detail by presenting a process of synthesizing monoamine compounds 3, 9, 21, 23, 29, 36, 45, 52, 4, 20, 26, 51, and 57 as an example. In addition, a process of synthesizing monoamine compounds, which will be described hereinafter, is provided as an example, and thus a process of synthesizing compounds according to an aspect of the present disclosure is not limited to Examples below.

### (1) Synthesis of monoamine Compound 3

Monoamine compound 3 according to an aspect may be synthesized by, for example, a process of Reaction Formula 1 below.

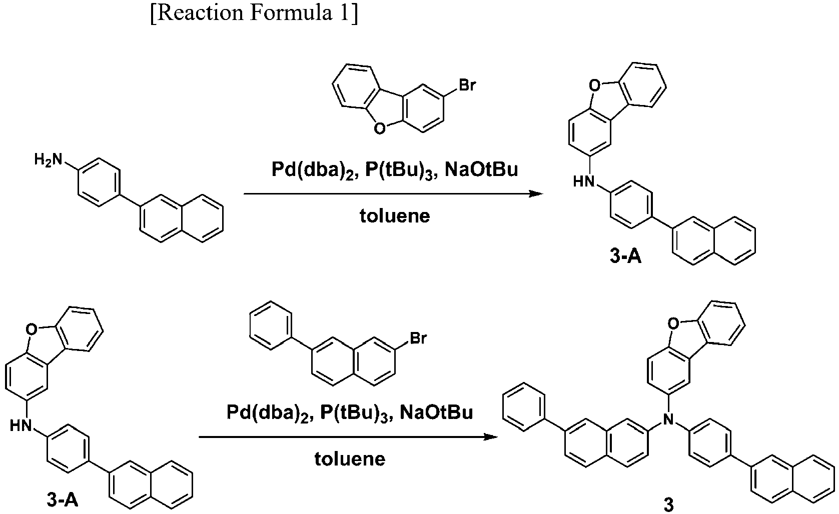

### <Synthesis of Intermediate Compound 3-A>

In an Ar atmosphere, 4-(naphthalen-2-yl)aniline (20.0 g), 2-bromodibenzofuran (22.5 g), bis(dibenzylideneacetone)palladium(0) (Pd(dba)₂, 0.5 g), and sodium tert-butoxide (NaOtBu, 13.1 g) were added to a 1 L 3-necked flask, and the mixture was dissolved in toluene (400 mL), and tri-tert-butylphosphine (P(*t*Bu)₃, 2.0 M in toluene, 0.9 mL) was added and stirred at room temperature for 8 hours. Water was added to the solution and an organic layer was obtained through extraction with dichloromethane (CH₂Cl₂). The obtained organic layer was dried over magnesium sulfate (MgSO₄) all at once, and then the solvent was distilled off under reduced pressure. The obtained crude product was purified through recrystallization to obtain 25.7 g of Intermediate Compound 3-A (yield: 73%). Intermediate Compound 3-A had a molecular weight of 385, as measured by FAB-MS.

### <Synthesis of Compound 3>

In an Ar atmosphere, Intermediate Compound 3-A (5.0 g), 2-bromo-7-phenylnaphthalene (3.7 g), Pd(dba)₂ (0.07 g), and NaOtBu (1.9 g) were added to a 300 mL 3-necked flask, and the mixture was dissolved in toluene (100 mL), and P(*t*Bu)₃ (2.0 M in toluene, 0.1 mL) was added, and heated and refluxed for 4 hours. Water was added to the solution and an organic layer was obtained through extraction with CH₂Cl₂. The obtained organic layer was dried over MgSO₄ all at once, and then the solvent was distilled off under reduced pressure. The obtained crude product was purified through silica gel column chromatography to obtain 6.2 g of Compound 3 (yield: 81%). Compound 3 had a molecular weight of 587, as measured by FAB-MS.

### (2) Synthesis of monoamine Compound 9

Monoamine Compound 9 according to an aspect may be synthesized by, for example, a process of Reaction Formula 2 below.

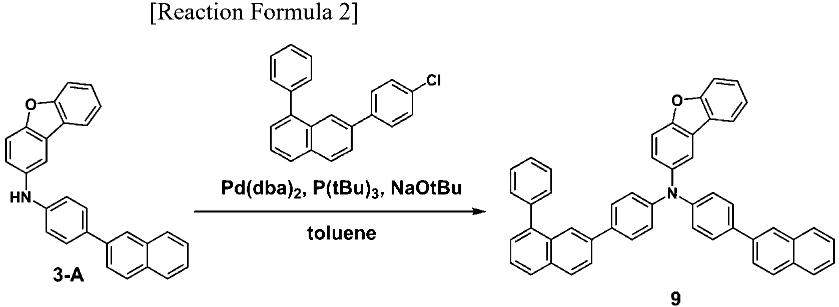

7.0 g of Compound 9 (yield: 81%) was obtained in the same manner as in the synthesis of Compound 3, except that 7-(4-chlorophenyl)-1-phenylnaphthalene (4.1 g) was used instead of 2-bromo-7-phenylnaphthalene (3.7 g). Compound 9 had a molecular weight of 663, as measured by FAB-MS.

### (3) Synthesis of monoamine Compound 21

Monoamine compound 21 according to an aspect may be synthesized by, for example, a process of Reaction Formula 3 below.

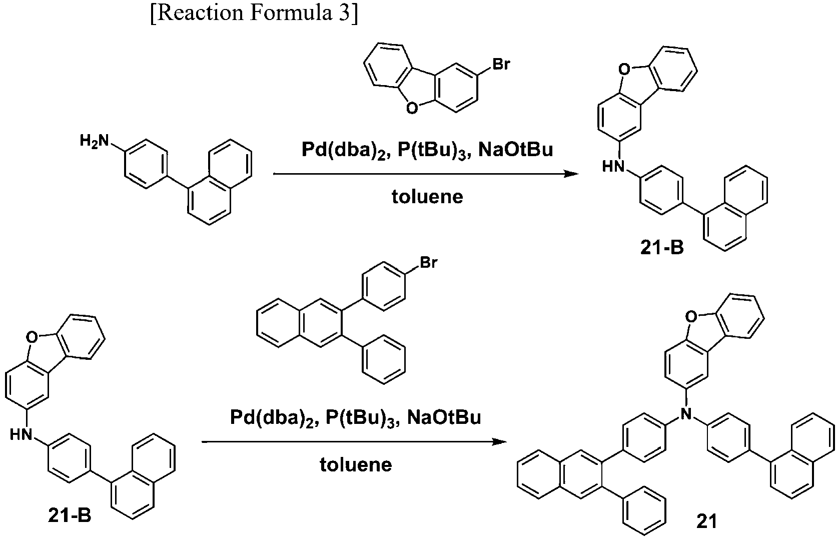

### <Synthesis of Intermediate Compound 21-B>

24.6 g of Intermediate Compound 21-B (yield: 70%) was obtained in the same manner as in the synthesis of Intermediate Compound 3-A, except that 4-(naphthalen-1-yl)aniline (20.0 g) was used instead of 4-(naphthalen-2-yl)aniline (20.0 g). Intermediate Compound 21-B had a molecular weight of 385, as measured by FAB-MS.

### <Synthesis of Compound 21>

6.5 g of Compound 21 (yield: 75%) was obtained in the same manner as in the synthesis of Compound 3, except that Intermediate Compound 21-B (5.0 g) was used instead of Intermediate Compound 3-A (5.0 g) and 2-(4-bromophenyl)-3-phenylnaphthalene (4.7 g) was used instead of 2-bromo-7-phenylnaphthalene (3.7 g). Compound 21 had a molecular weight of 663, as measured by FAB-MS.

### (4) Synthesis of monoamine Compound 23

Monoamine compound 23 according to an aspect may be synthesized by, for example, a process of Reaction Formula 4 below.

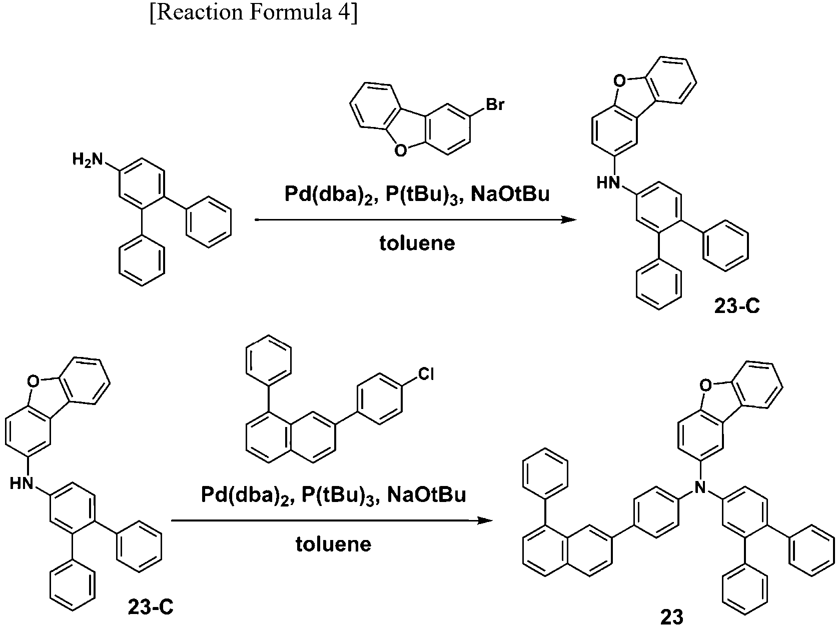

### <Synthesis of Intermediate Compound 23-C>

18.8 g of Intermediate Compound 23-C (yield: 56%) was obtained in the same manner as in the synthesis of Intermediate Compound 3-A, except that [1,1':2',1"-Terphenyl]-4'-amine (20.0 g) was used instead of 4-(naphthalen-2-yl)aniline (20.0 g) and 2-bromodibenzofuran (20.1 g) was used instead of 2-bromodibenzofuran (22.5 g). Intermediate Compound 23-C had a molecular weight of 411, as measured by FAB-MS.

### <Synthesis of Compound 23>

6.7 g of Compound 23 (yield: 80%) was obtained in the same manner as in the synthesis of Compound 3, except that Intermediate Compound 23-C (5.0 g) was used instead of Intermediate Compound 3-A (5.0 g) and 7-(4-chlorophenyl)-1-phenylnaphthalene (3.9 g) was used instead of 2-bromo-7-phenylnaphthalene (3.7 g). Compound 23 had a molecular weight of 689, as measured by FAB-MS.

### (5) Synthesis of monoamine Compound 29

Monoamine Compound 29 according to an aspect may be synthesized by, for example, a process of Reaction Formula 5 below.

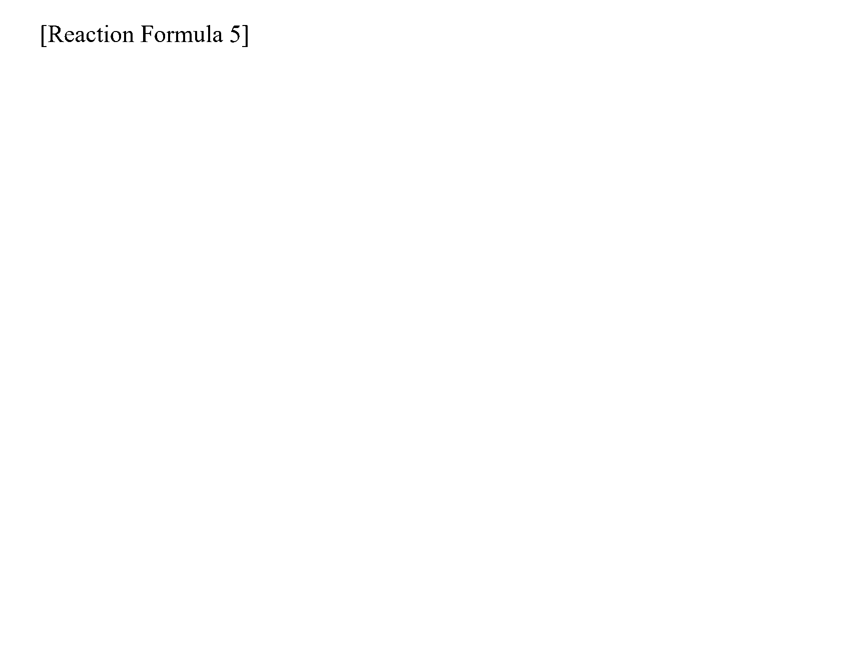

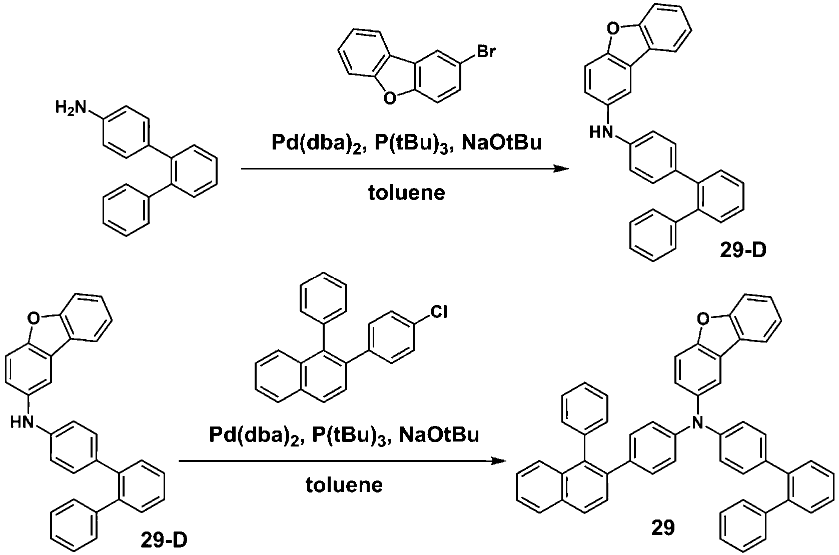

### <Synthesis of Intermediate Compound 29-D>

20.8 g of Intermediate Compound 29-D (yield: 62%) was obtained in the same manner as in the synthesis of Intermediate Compound 3-A, except that [1,1':2',1"-Terphenyl]-4-amine (20.0 g) was used instead of 4-(naphthalen-2-yl)aniline (20.0 g) and 2-bromodibenzofuran (20.1 g) was used instead of 2-bromodibenzofuran (22.5 g). Compound 29-D had a molecular weight of 411, as measured by FAB-MS.

### <Synthesis of Compound 29>

6.6 g of Compound 29 (yield: 79%) was obtained in the same manner as in the synthesis of Compound 3, except that Intermediate Compound 29-D (5.0 g) was used instead of Intermediate Compound 3-A (5.0 g) and 2-(4-chlorophenyl)-1-phenylnaphthalene (3.9 g) was used instead of 2-bromo-7-phenylnaphthalene (3.7 g). Compound 29 had a molecular weight of 689, as measured by FAB-MS.

### (6) Synthesis of monoamine Compound 36

Monoamine compound 36 according to an aspect may be synthesized by, for example, a process of Reaction Formula 6 below.

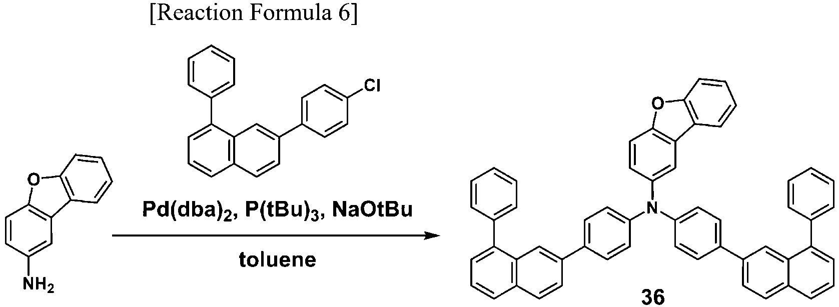

In an Ar atmosphere, dibenzofuran-2-amine (2.0 g), 7-(4-chlorophenyl)-1-phenylnaphthalene (6.9 g), Pd(dba)₂ (0.13 g), and NaOtBu (2.7 g) were added to a 300 mL 3-necked flask, and the mixture was dissolved in toluene (100 mL), and P(*t*Bu)₃ (2.0 M in toluene, 0.2 mL) was added, and heated and refluxed for 6 hours. Water was added to the solution and an organic layer was obtained through extraction with CH₂Cl₂. The obtained organic layer was dried over MgSO₄ all at once, and then the solvent was distilled off under reduced pressure. The obtained crude product was purified through silica gel column chromatography to obtain 6.1 g of Compound 36 (yield: 75%). Compound 36 had a molecular weight of 739, as measured by FAB-MS.

### (7) Synthesis of monoamine Compound 45

Monoamine Compound 45 according to an aspect may be synthesized by, for example, a process of Reaction Formula 7 below.

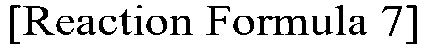

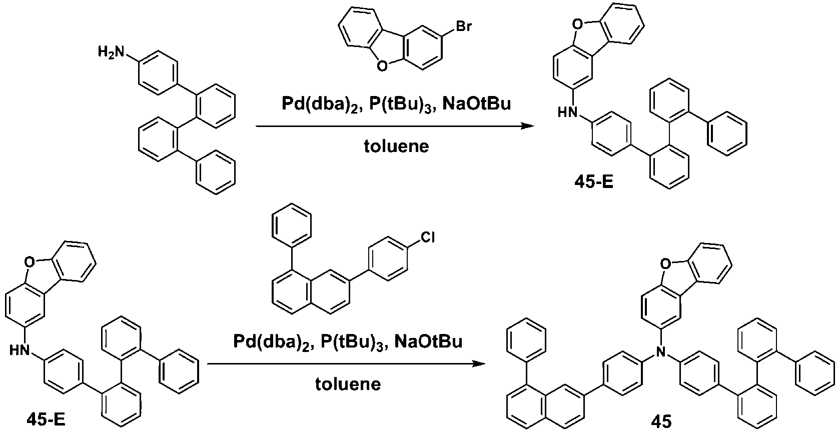

### <Synthesis of Intermediate Compound 45-E>

19.7 g of Intermediate Compound 45-E (yield: 65%) was obtained in the same manner as in the synthesis of Intermediate Compound 3-A, except that [1,1':2',1":2",1‴-Quaterphenyl]-4-amine (20.0 g) was used instead of 4-(naphthalen-2-yl)aniline (20.0 g) and 2-bromodibenzofuran (15.3 g) was used instead of 2-bromodibenzofuran (22.5 g). Intermediate Compound 45-E had a molecular weight of 487, as measured by FAB-MS.

### <Synthesis of Compound 45>

5.3 g of Compound 45 (yield: 68%) was obtained in the same manner as in the synthesis of Compound 3, except that Intermediate Compound 45-E (5.0 g) was used instead of Intermediate Compound 3-A (5.0 g) and 7-(4-chlorophenyl)-1-phenylnaphthalene (3.3 g) was used instead of 2-bromo-7-phenylnaphthalene (3.7 g). Compound 45 had a molecular weight of 765, as measured by FAB-MS.

### (8) Synthesis of monoamine Compound 52

Monoamine Compound 52 according to an aspect may be synthesized by, for example, a process of Reaction Formula 8 below.

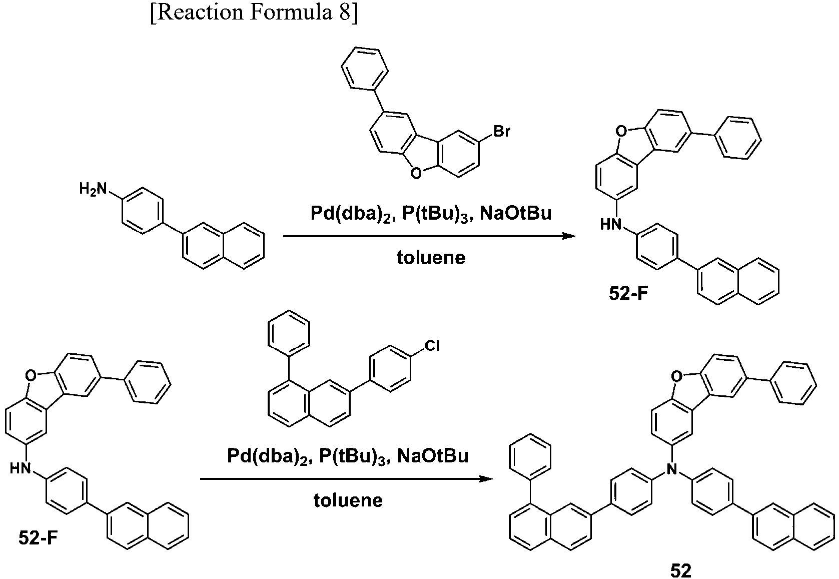

### <Synthesis of Intermediate Compound 52-F>

11.6 g of Intermediate Compound 52-F (yield: 55%) was obtained in the same manner as in the synthesis of Intermediate Compound 3-A, except that 4-(naphthalen-2-yl)aniline (10.0 g) was used instead of 4-(naphthalen-2-yl)aniline (20.0 g) and 2-bromo-8-phenyldibenzofuran (14.7 g) was used instead of 2-bromodibenzofuran (22.5 g). Intermediate Compound 52-F had a molecular weight of 461, as measured by FAB-MS.

### <Synthesis of Compound 52>

5.5 g of Compound 52 (yield: 68%) was obtained in the same manner as in the synthesis of Compound 3, except that Intermediate Compound 52-F (5.0 g) was used instead of Intermediate Compound 3-A (5.0 g) and 7-(4-chlorophenyl)-1-phenylnaphthalene (3.4 g) was used instead of 2-bromo-7-phenylnaphthalene (3.7 g). Compound 52 had a molecular weight of 739, as measured by FAB-MS.

### (9) Synthesis of monoamine Compound 4

Monoamine Compound 4 according to an aspect may be synthesized by, for example, a process of Reaction Formula 9 below.

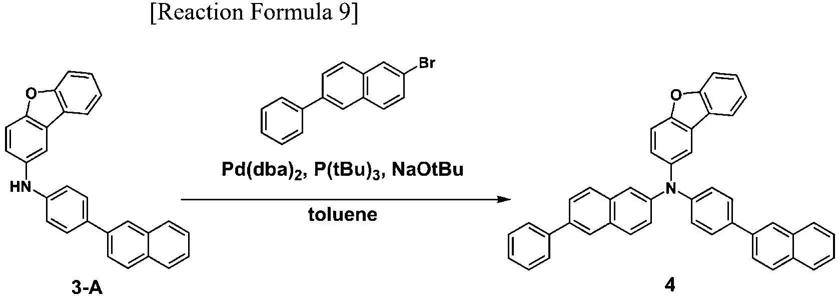

5.3 g of Compound 4 (yield: 69%) was obtained in the same manner as in the synthesis of Compound 3, except that 2-bromo-6-phenylnaphthalene (3.7 g) was used instead of 2-bromo-7-phenylnaphthalene (3.7 g). Compound 4 had a molecular weight of 587, as measured by FAB-MS.

### (10) Synthesis of monoamine Compound 20

Monoamine Compound 20 according to an aspect may be synthesized by, for example, a process of Reaction Formula 10 below.

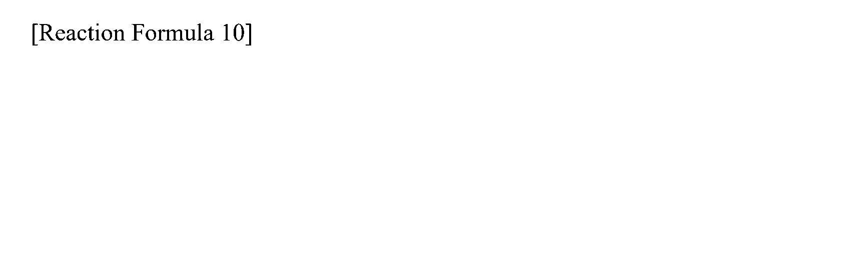

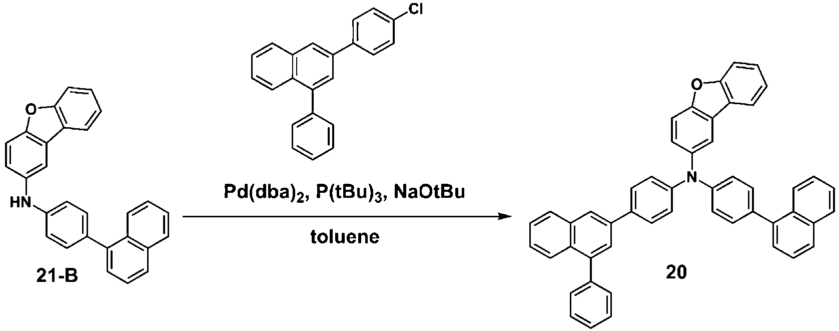

6.2 g of Compound 20 (yield: 72%) was obtained in the same manner as in the synthesis of Compound 3, except that Intermediate Compound 21-B (5.0 g) was used instead of Intermediate Compound 3-A (5.0 g) and 3-(4-chlorophenyl)-1-phenylnaphthalene (4.1 g) was used instead of 2-bromo-7-phenylnaphthalene (3.7 g). Compound 20 had a molecular weight of 663, as measured by FAB-MS.

### (11) Synthesis of monoamine Compound 26

Monoamine Compound 26 according to an aspect may be synthesized by, for example, a process of Reaction Formula 11 below.

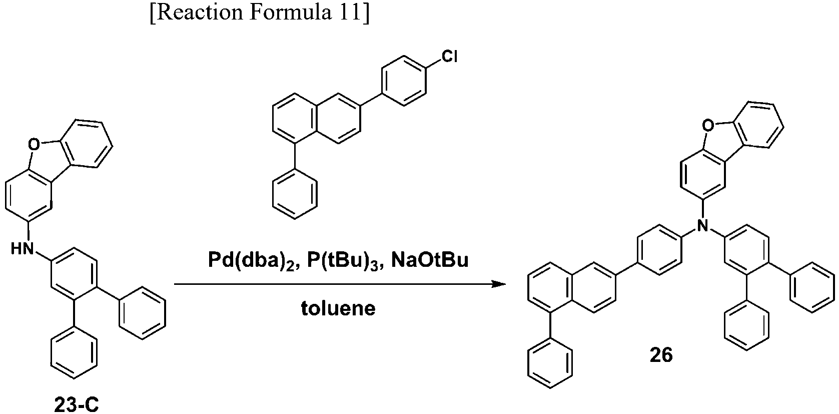

6.2 g of Compound 26 (yield: 74%) was obtained in the same manner as in the synthesis of Compound 3, except that Intermediate Compound 23-C (5.0 g) was used instead of Intermediate Compound 3-A (5.0 g) and 6-(4-chlorophenyl)-1-phenylnaphthalene (3.8 g) was used instead of 2-bromo-7-phenylnaphthalene (3.7 g). Compound 26 had a molecular weight of 689, as measured by FAB-MS.

### (12) Synthesis of monoamine Compound 51

Monoamine Compound 51 according to an aspect may be synthesized by, for example, a process of Reaction Formula 12 below.

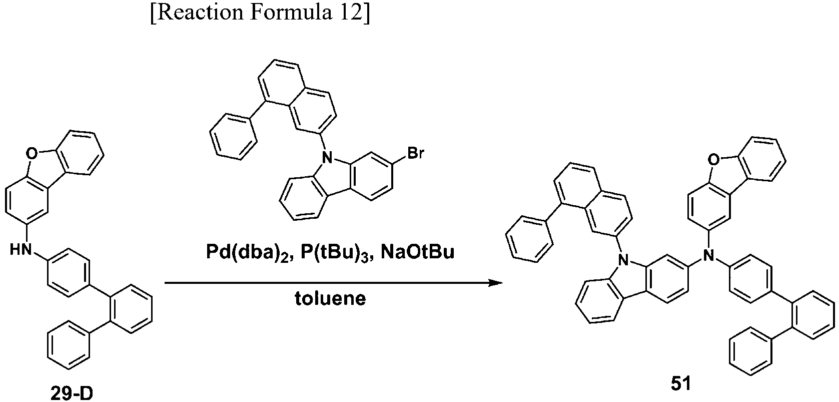

5.4 g of Compound 51 (yield: 57%) was obtained in the same manner as in the synthesis of Compound 3, except that Intermediate Compound 29-D (5.0 g) was used instead of Intermediate Compound 3-A (5.0 g) and 2-bromo-9-(8-phenylnaphthalen-2-yl)-9H-carbazole (5.5 g) was used instead of 2-bromo-7-phenylnaphthalene (3.7 g). Compound 51 had a molecular weight of 778, as measured by FAB-MS.

### (13) Synthesis of monoamine Compound 57

Monoamine Compound 57 according to an aspect may be synthesized by, for example, a process of Reaction Formula 13 below.

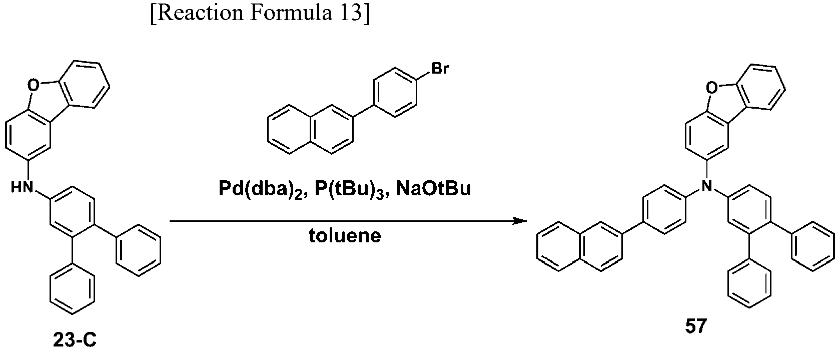

5.1 g of Compound 57 (yield: 68%) was obtained in the same manner as in the synthesis of Compound 3, except that Intermediate Compound 23-C (5.0 g) was used instead of Intermediate Compound 3-A (5.0 g) and 2-(4-bromophenyl)naphthalene (3.5 g) was used instead of 2-bromo-7-phenylnaphthalene (3.7 g). Compound 57 had a molecular weight of 613, as measured by FAB-MS.

### 2. Preparation and Evaluation of Light Emitting Element

### (1) Preparation of light emitting element

Light emitting elements including monoamine compounds according to an aspect or Comparative Example compounds in a hole transport layer were prepared through a method below. Light emitting elements of Examples 1 to 13 were prepared using monoamine Compounds of an aspect, 3, 9, 21, 23, 29, 36, 45, 52, 4, 20, 26, 51, and 57 as a material of the hole transport layer. Light emitting elements of Comparative Examples 1 to 40 were prepared using Comparative Example Compounds X-1 to X-40 as a material of the hole transport layer.

A glass substrate on which an ITO having a thickness of 150 nm was patterned as a first electrode was subjected to ultrasonic cleaning using isopropyl alcohol and pure water each for 5 minutes. The glass substrate was irradiated with UV for 30 minutes, and ozone-treated. Thereafter, a hole injection layer was formed through the deposition of 2-TNATA with a thickness of 60 nm. On the hole injection layer, a hole transport layer was formed through the deposition of Example compounds or Comparative Example compounds with a thickness of 30 nm.

On the hole transport layer, an emission layer was formed through the co-deposition of TBP and compound E15 with a thickness of 25 nm. TBP and compound E15 were subjected to the co-deposition at a weight ratio of 3:97. Thereafter, an electron transport region was formed through the sequential deposition of Alq₃ with a thickness of 25 nm and LiF with a thickness of 1 nm. Then, a second electrode was formed through the deposition of Al with a thickness of 100 nm. The hole injection layer, the hole transport layer, the emission layer, the electron transport region, and the second electrode were formed using a vacuum deposition device.

### <Materials used for preparation of light emitting elements>

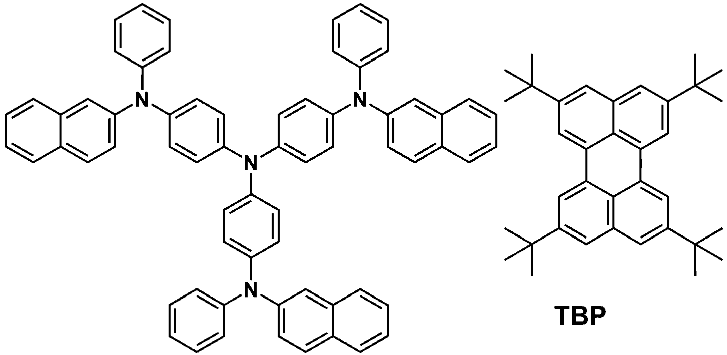

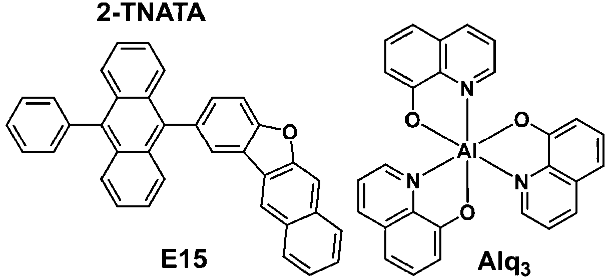

### <Example Compound>

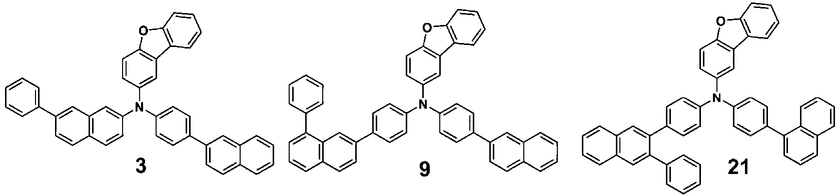

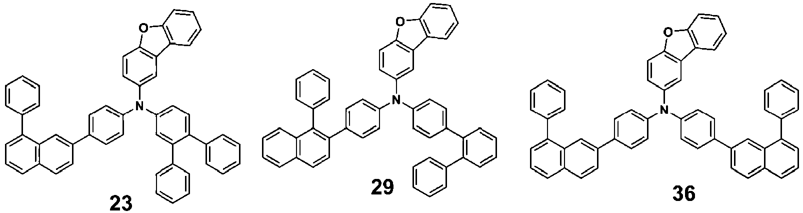

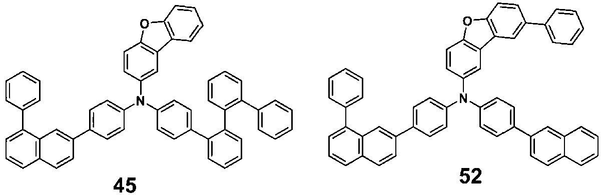

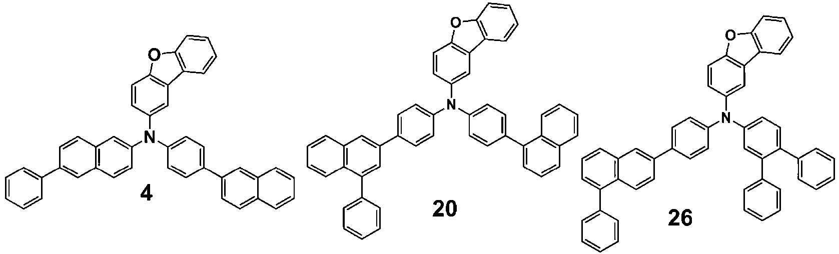

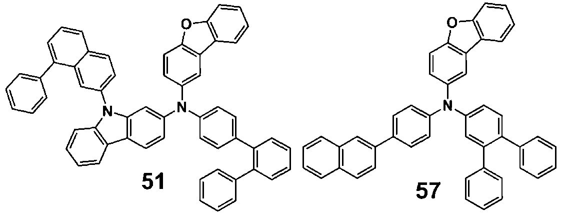

### <Comparative Example Compound>

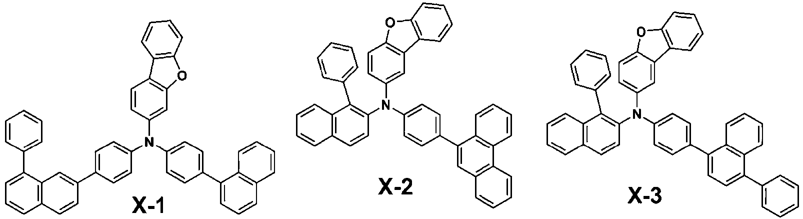

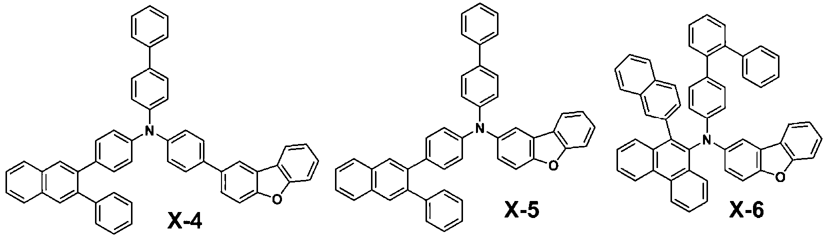

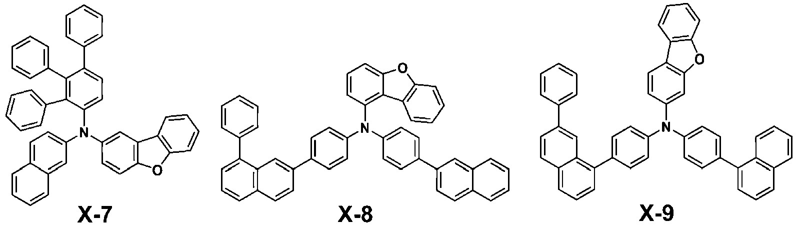

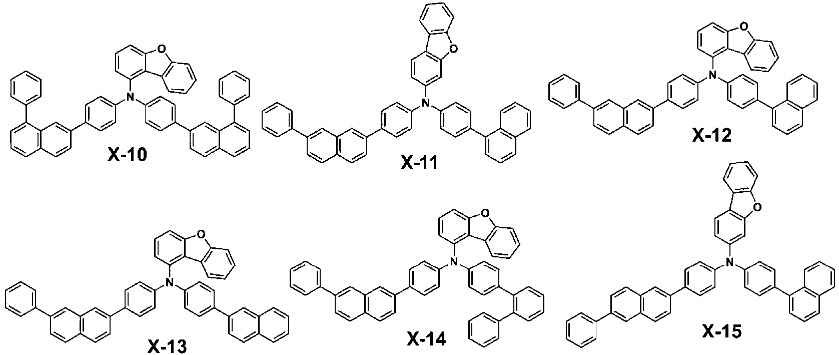

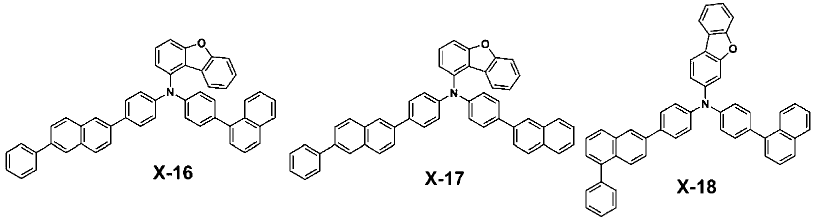

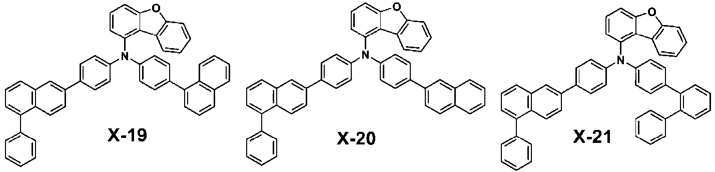

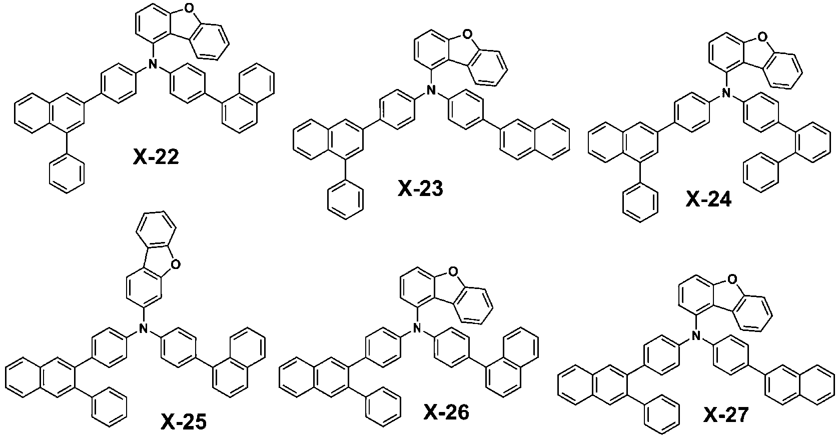

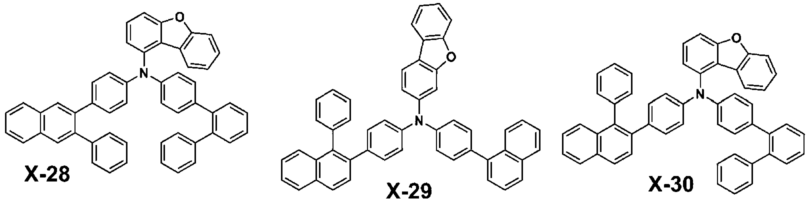

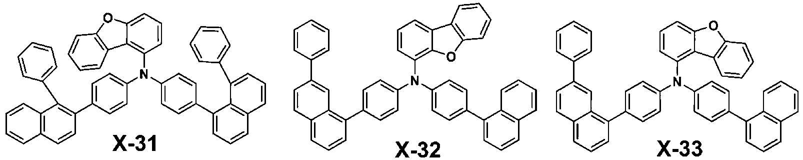

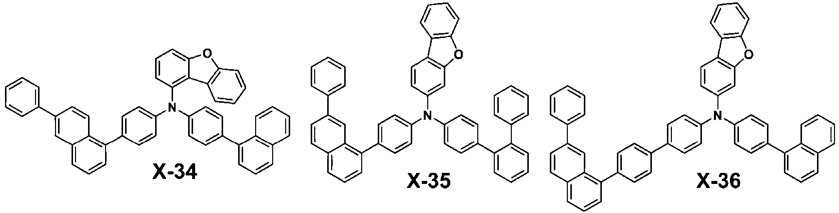

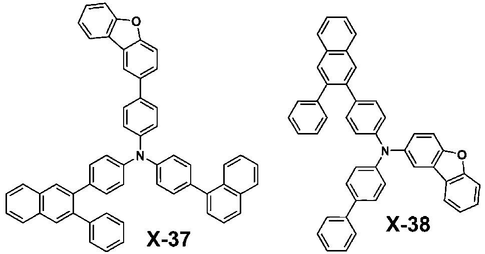

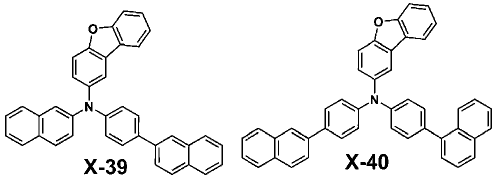

### (2) Evaluation of light emitting element

Table 1 below shows evaluation results of the light emitting elements of Examples and Comparative Examples. The driving voltage, luminous efficiency, and lifespan at a current density of 10 mA/cm² were evaluated using a C9920-11 luminance orientation characteristic measuring device from Hamamatsu Photonics. Lifespan (LT50) indicates the time taken to decrease from an initial luminance of 100% to a luminance of 50%. In Table 1, the driving voltage, luminous efficiency, and lifespan are relative values, with the value of Comparative Example 1 set as 100%.

**[Table 1]**

| Example of element preparation | Hole transport layer | Driving voltage | Luminous efficiency (@ 10 mA/cm²) | Lifespan (LT50) |
|---|---|---|---|---|
| Example 1 | Compound 3 | 95% | 102% | 150% |
| Example 2 | Compound 9 | 94% | 102% | 170% |
| Example 3 | Compound 21 | 97% | 105% | 130% |
| Example 4 | Compound 23 | 95% | 103% | 150% |
| Example 5 | Compound 29 | 96% | 103% | 140% |
| Example 6 | Compound 36 | 94% | 102% | 180% |
| Example 7 | Compound 45 | 95% | 103% | 140% |
| Example 8 | Compound 52 | 98% | 105% | 120% |
| Example 9 | Compound 4 | 95% | 102% | 140% |
| Example 10 | Compound 20 | 98% | 103% | 150% |
| Example 11 | Compound 26 | 96% | 104% | 130% |
| Example 12 | Compound 51 | 93% | 101% | 100% |
| Example 13 | Compound 57 | 94% | 102% | 160% |
| Comparative Example 1 | Comparative Example Compound X-1 | 100% | 100% | 100% |
| Comparative Example 2 | Comparative Example Compound X-2 | 98% | 101% | 65% |
| Comparative Example 3 | Comparative Example Compound X-3 | 98% | 101% | 70% |
| Comparative Example 4 | Comparative Example Compound X-4 | 100% | 98% | 80% |
| Comparative Example 5 | Comparative Example Compound X-5 | 95% | 100% | 70% |
| Comparative Example 6 | Comparative Example Compound X-6 | 102% | 100% | 50% |
| Comparative Example 7 | Comparative Example Compound X-7 | 100% | 100% | 40% |
| Comparative Example 8 | Comparative Example Compound X-8 | 105% | 100% | 70% |
| Comparative Example 9 | Comparative Example Compound X-9 | 103% | 101% | 60% |
| Comparative Example 10 | Comparative Example Compound X-10 | 105% | 101% | 80% |
| Comparative Example 11 | Comparative Example Compound X-11 | 100% | 100% | 100% |
| Comparative Example 12 | Comparative Example Compound X-12 | 105% | 100% | 70% |
| Comparative Example 13 | Comparative Example Compound X-13 | 104% | 100% | 75% |
| Comparative Example 14 | Comparative Example Compound X-14 | 104% | 100% | 60% |
| Comparative Example 15 | Comparative Example Compound X-15 | 100% | 100% | 90% |
| Comparative Example 16 | Comparative Example Compound X-16 | 106% | 100% | 60% |
| Comparative Example 17 | Comparative Example Compound X-17 | 106% | 99% | 60% |
| Comparative Example 18 | Comparative Example Compound X-18 | 101% | 100% | 80% |
| Comparative Example 19 | Comparative Example Compound X-19 | 105% | 100% | 50% |
| Comparative Example 20 | Comparative Example Compound X-20 | 105% | 99% | 55% |
| Comparative Example 21 | Comparative Example Compound X-21 | 105% | 100% | 50% |
| Comparative Example 22 | Comparative Example Compound X-22 | 105% | 100% | 50% |
| Comparative Example 23 | Comparative Example Compound X-23 | 105% | 100% | 60% |
| Comparative Example 24 | Comparative Example Compound X-24 | 105% | 100% | 50% |
| Comparative Example 25 | Comparative Example Compound X-25 | 101% | 101% | 80% |
| Comparative Example 26 | Comparative Example Compound X-26 | 105% | 100% | 60% |
| Comparative Example 27 | Comparative Example Compound X-27 | 105% | 100% | 60% |
| Comparative Example 28 | Comparative Example Compound X-28 | 104% | 101% | 50% |
| Comparative Example 29 | Comparative Example Compound X-29 | 101% | 101% | 90% |
| Comparative Example 30 | Comparative Example Compound X-30 | 105% | 101% | 45% |
| Comparative Example 31 | Comparative Example Compound X-31 | 106% | 98% | 30% |
| Comparative Example 32 | Comparative Example Compound X-32 | 102% | 101% | 70% |
| Comparative Example 33 | Comparative Example Compound X-33 | 107% | 101% | 40% |
| Comparative Example 34 | Comparative Example Compound X-34 | 106% | 100% | 50% |
| Comparative Example 35 | Comparative Example Compound X-35 | 102% | 100% | 70% |
| Comparative Example 36 | Comparative Example Compound X-36 | 100% | 99% | 60% |
| Comparative Example 37 | Comparative Example Compound X-37 | 99% | 100% | 90% |
| Comparative Example 38 | Comparative Example Compound X-38 | 97% | 99% | 50% |
| Comparative Example 39 | Comparative Example Compound X-39 | 97% | 97% | 50% |
| Comparative Example 40 | Comparative Example Compound X-40 | 96% | 99% | 80% |

Referring to Table 1, it is seen that, compared to the light emitting elements of Comparative Examples 1 to 40, the light emitting elements of Examples 1 to 13 exhibit high luminous efficiency and long life characteristics. It is seen that, compared to the light emitting elements of Comparative Examples 1, 4, and 6 to 37, the light emitting elements of Examples 1 to 13 exhibit low driving voltage. The light emitting elements of Examples 1 to 13 respectively include Compounds 3, 9, 21, 23, 29, 36, 45, 52, 4, 20, 26, 51, and 57 as a hole transport layer material, and Compounds 3, 9, 21, 23, 29, 36, 45, 52, 4, 20, 26, 51, and 57 are monoamine compounds of an aspect. Compounds 3, 9, 21, 23, 29, 36, 45, 52, 4, 20, 26, 51, and 57 each include the first to third substituents described above, and the first to third substituents are directly or indirectly bonded to an amine group. The first substituent is a 2-dibenzofuranyl group, and the second substituent is a phenylnaphthalenyl group or an unsubstituted naphthalenyl group. The third substituent is a substituted phenyl group. Accordingly, it is seen that the monoamine compound of an aspect contributes to reducing driving voltage, improving luminous efficiency, and/or improving lifespan with respect to a light emitting element. It is seen that the light emitting element including the monoamine compound of an aspect may exhibit low driving voltage, high luminous efficiency, and/or long life characteristics.

Referring to Table 1, it is seen that, compared to the light emitting elements of Comparative Examples 1 and 8, the light emitting elements of Examples 1 to 13 exhibit significantly reduced driving voltage. The light emitting elements of Comparative Examples 1 and 8 respectively include Comparative Example Compounds X-1 and X-8. Comparative Example Compounds X-1 and X-8 each include a phenylnaphthalenyl group (i.e., a second substituent) and a phenyl group substituted with a naphthyl group (i.e., a third substituent). However, Comparative Example Compound X-1 includes a 3-dibenzofuranyl group, differing from the monoamine compound of an aspect which includes a 2-dibenzofuranyl group. Comparative Example Compound X-8 includes a 1-dibenzofuranyl group, differing from the monoamine compound of an aspect which includes a 2-dibenzofuranyl group (i.e., a first substituent). Accordingly, the light emitting elements of Comparative Examples 1 and 8 exhibit relatively high driving voltage, low luminous efficiency, and short lifespan.

The light emitting elements of Comparative Examples 2 and 3 respectively include Comparative Example Compounds X-2 and X-3. Comparative Example Compound X-2 includes a phenyl group substituted with a phenanthrenyl group, differing from the monoamine compound of an aspect which includes the third substituent. Comparative Example Compound X-3 includes a naphthyl group substituted with a phenyl group as a substituent of the substituted phenyl group, differing from the monoamine compound of an aspect which includes the third substituent. Comparative Examples Compounds X-2 and X-3 differ from the monoamine compound of an aspect in that the position corresponding to a hydrogen atom of a naphthalenyl group in Formula 2-2 described above is a carbon atom. The phenyl group substituted with the phenanthrenyl group included in Comparative Example Compound X-2 corresponds to a bulky substituent. The substituted phenyl group including the naphthyl group substituted with the phenyl group included in Comparative Example Compound X-3 as a substituent corresponds to a bulky substituent. Accordingly, the light emitting elements of Comparative Examples 2 and 3 exhibit a relatively small effect of voltage reduction and relatively short lifespan.

The light emitting elements of Comparative Examples 4 and 5 respectively include Comparative Example Compounds X-4 and X-5. Comparative Example Compounds X-4 and X-5 include a biphenyl group, differing from the monoamine compound of an aspect which includes the third substituent. In addition, Comparative Example Compound X-4 differs from the monoamine compound of an aspect in that the first substituent (i.e., 2-dibenzofuranyl group) is not directly bonded to an amine group but is bonded via a phenyl group. The biphenyl group included in Comparative Example Compounds X-4 and X-5 includes one less phenyl group in a terphenyl moiety represented by Formulas 2-3 and 2-4 described above. It is seen that the monoamine compound of an aspect includes the terphenyl moiety represented by Formula 2-3 or Formula 2-4, and thus contributes to improving the lifespan of a light emitting element. In contrast, the light emitting elements of Comparative Examples 4 and 5, each including Comparative Example Compounds X-4 and X-5, exhibit relatively low luminous efficiency and short lifespan, as Comparative Example Compounds X-4 and X-5 show the differences as described above when compared to the monoamine compound of an aspect.

The light emitting element of Comparative Example 6 includes Comparative Example Compound X-6. Comparative Example Compound X-6 corresponds to the case where n1 is 0 and Ar¹ is represented by Formula 2-4 in Formula 1 described above, but differs from the monoamine compound of an aspect in that L¹ is a phenanthryl group. Comparative Example Compound X-6 includes a phenanthryl group, and thus exhibit a large steric hindrance within molecules and reduced material stability. Accordingly, the light emitting element of Comparative Example 6 exhibit relatively short lifespan.

The light emitting element of Comparative Example 7 includes Comparative Example Compound X-7. Comparative Example Compound X-7 corresponds to the case where n1 is 0 and Ar¹ is represented by Formula 2-3 in Formula 1 described above, but differs from the monoamine compound of an aspect in that y5 is 1. Comparative Example Compound X-7 includes 1,2,3-triphenylbenzene as the third substituent. Accordingly, Comparative Example Compound X-7 exhibits a large steric hindrance within molecules and reduced material stability. Accordingly, the light emitting element of Comparative Example 7 exhibit relatively short lifespan.

The light emitting element of Comparative Example 9 includes Comparative Example Compound X-9. Comparative Example Compound X-9 differs from the monoamine compound of an aspect in the bonding position of L¹ to the naphthalenyl group containing R^{a2} in Formula 1 described above. Comparative Example Compound X-9 exhibits reduced hole transport properties as L¹ is bonded to a carbon atom at position 1 of a naphthalenyl group. Accordingly, the light emitting element of Comparative Example 9 exhibits relatively high driving voltage.

The light emitting elements of Comparative Examples 10, 12 to 14, 16, 17, 19 to 24, 26 to 28, 30, 31, 33, and 34 respectively include Comparative Example Compounds X-10, X-12 to X-14, X-16, X-17, X-19 to X-24, X-26 to X-28, X-30, X-31, X-33, and X-34. Comparative Example Compounds X-10, X-12 to X-14, X-16, X-17, X-19 to X-24, X-26 to X-28, X-30, X-31, X-33, and X-34 include a 1-dibenzofuranyl group, differing from the monoamine compounds of an aspect which includes a 2-dibenzofuranyl group (i.e., a first substituent). Accordingly, the light emitting elements of Comparative Examples 10, 12 to 14, 16, 17, 19 to 24, 26 to 28, 30, 31, 33, and 34 exhibit relatively high driving voltages, low luminous efficiency, and short lifespan.

The light emitting elements of Comparative Examples 11, 15, 18, 25, 29, 35, and 36 respectively include Comparative Example Compounds X-11, X-15, X-18, X-25, X-29, X-35, and X-36. Comparative Example Compounds X-11, X-15, X-18, X-25, X-29, X-35, and X-36 include a 3-dibenzofuranyl group, differing from the monoamine compound of an aspect which includes a 2-dibenzofuranyl group (i.e., a first substituent). Accordingly, the light emitting elements of Comparative Examples 11, 15, 18, 25, 29, 35, and 36 exhibit relatively high driving voltage, low luminous efficiency, and short lifespan.

The light emitting element of Comparative Example 32 includes Comparative Example Compound X-32. Comparative Example Compound X-32 includes a 4-dibenzofuranyl group, differing from the monoamine compound of an aspect which includes a 2-dibenzofuranyl group (i.e., a first substituent). Accordingly, the light emitting element of Comparative Example 32 exhibits relatively high driving voltage, low luminous efficiency, and short lifespan.

The light emitting element of Comparative Example 37 includes Comparative Example Compound X-37. Comparative Example Compound X-37 differs from the monoamine compound of an aspect in that the first substituent (i.e., 2-dibenzofuranyl group) is not directly bonded to an amine group but is bonded via a phenyl group. Accordingly, the light emitting element of Comparative Example 37 exhibits relatively high driving voltage, low luminous efficiency, and short lifespan.

The light emitting element of Comparative Example 38 includes Comparative Example Compound X-38. Comparative Example Compound X-38 include a biphenyl group, differing from the monoamine compound of an aspect which includes the third substituent. Accordingly, the light emitting element of Comparative Example 38 exhibits relatively low luminous efficiency and short lifespan.

The light emitting element of Comparative Example 39 includes Comparative Example Compound X-39. Comparative Example Compound X-39 corresponds to the case where Ar¹ is represented by Formula 2-1 in Formula 1 described above, but differs from the monoamine compound of an aspect in that n1 is 0. Accordingly, the light emitting element of Comparative Example 39 exhibits relatively low luminous efficiency and short lifespan.

The light emitting element of Comparative Example 40 includes Comparative Example Compound X-40. Comparative Example Compound X-40 corresponds to the case where Ar¹ is represented by Formula 2-2 in Formula 1 described above, but differs from the monoamine compound of an aspect in that n1 is 0. Accordingly, the light emitting element of Comparative Example 40 exhibits relatively low luminous efficiency and short lifespan.

An electronic apparatus of an aspect includes a display device, and the display device includes a light emitting element. The light emitting element of an aspect includes a monoamine compound of an aspect. The monoamine compound of an aspect includes first to third substituents directly or indirectly bonded to an amine group. The first substituent is a 2-dibenzofuranyl group, and the second substituent is a phenylnaphthalenyl group or an unsubstituted naphthalenyl group. The third substituent is a substituted phenyl group. Accordingly, the monoamine compound of an aspect may contribute to reducing driving voltage, improving luminous efficiency, and/or improving lifespan with respect to the light emitting element. The light emitting element including the monoamine compound of an aspect may exhibit low driving voltage, high luminous efficiency, and/or long lifetime.

A light emitting element of an aspect and an electronic apparatus including the light emitting element include a monoamine compound of an aspect, and may thus exhibit high luminous efficiency and long life.

A monoamine compound of an aspect may contribute to high luminous efficiency and long life of a light emitting element.

In the above, description has been made with reference to aspects of the present disclosure, but those skilled or of ordinary skill in the art may understand that various modifications and changes may be made to the present disclosure insofar as such modifications and changes do not depart from the technical scope of the present disclosure.

Therefore, the technical scope of the present disclosure is not to be limited to the contents stated in the detailed description of the specification.

## Claims

1. A monoamine compound represented by Formula 1: 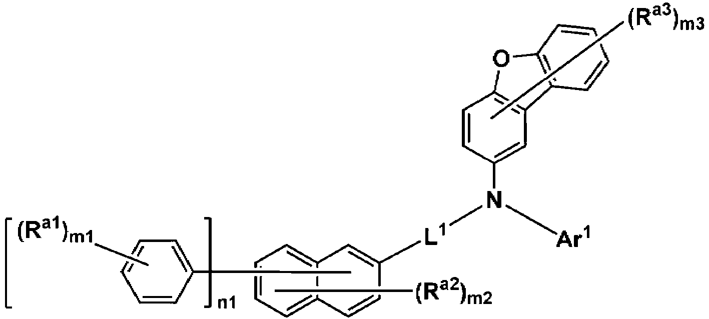
wherein in Formula 1,
m1 is an integer of 0 to 5,
m2 is an integer of 0 to 7,
m3 and n1 are each independently an integer of 0 to 7,
R^{a1} to R^{a3} are each independently a hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms,
L¹ is a direct linkage, a substituted or unsubstituted arylene group having 6 to 30 ring-forming carbon atoms, or a substituted or unsubstituted heteroarylene group having 5 to 30 ring-forming carbon atoms,
Ar¹ is represented by any one of Formulas 2-1 to 2-4, and
when Ar¹ is represented by Formula 2-1 or Formula 2-2, n1 is an integer of 1 to 7, 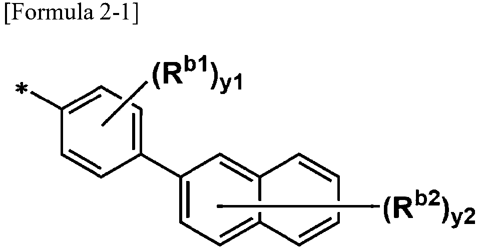 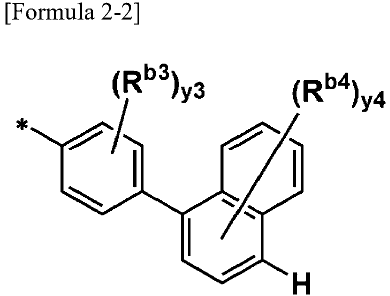 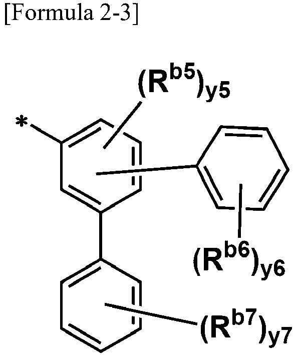 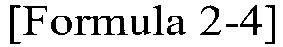 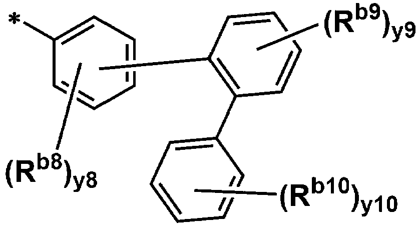
wherein in Formulas 2-1 to 2-4,
y1, y3, y8, and y9 are each independently an integer of 0 to 4,
y2 is an integer of 0 to 7,
y4 is an integer of 0 to 6,
y5 is an integer of 0 to 3,
y6, y7, and y10 are each independently an integer of 0 to 5,
R^{b1} to R^{b10} are each independently a hydrogen atom, a deuterium atom, a halogen atom, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms,
when n1 is 0 and Ar¹ is represented by Formula 2-4, L¹ is not a substituted or unsubstituted phenanthryl group,
when n1 is 0 and Ar¹ is represented by Formula 2-3, y5 is 0, and
any one or more hydrogen atoms in the monoamine compound is optionally substituted with a deuterium atom.

2. The monoamine compound of claim 1, wherein the monoamine compound is represented by any one of Formulas 1-1 to 1-8: 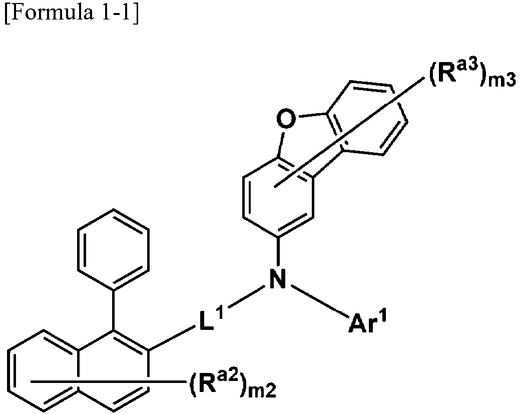 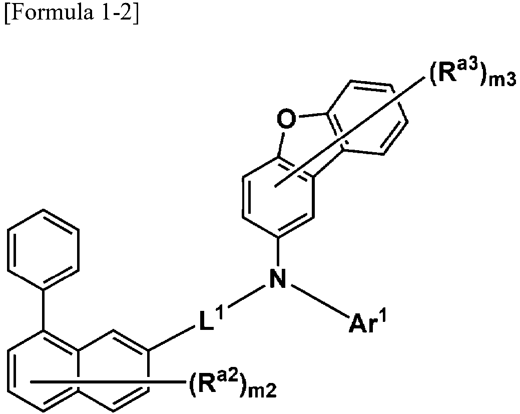 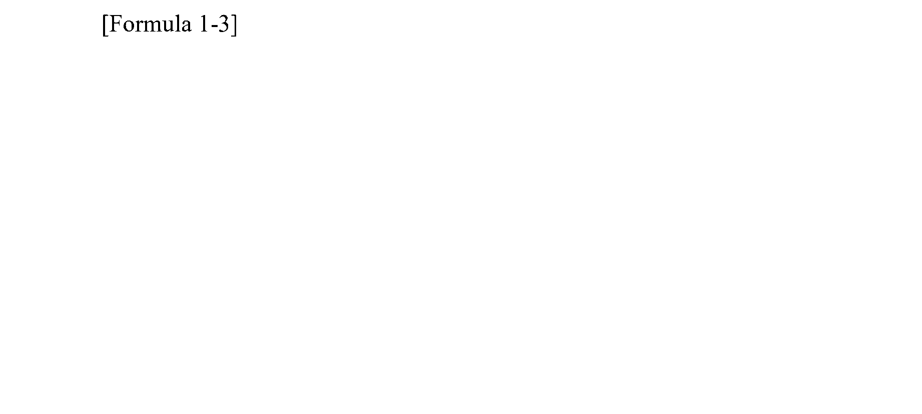 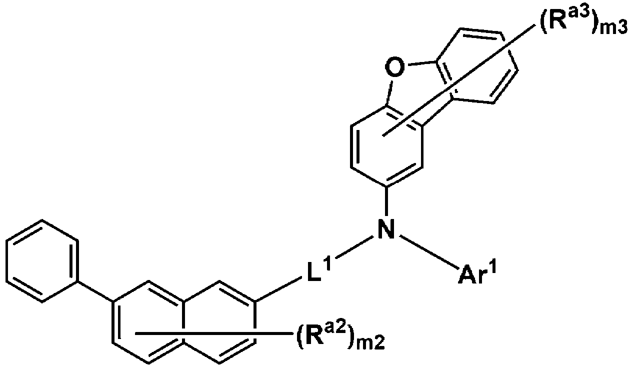 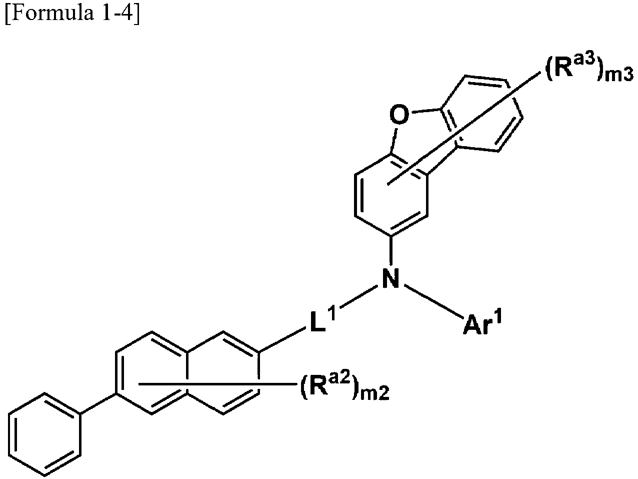 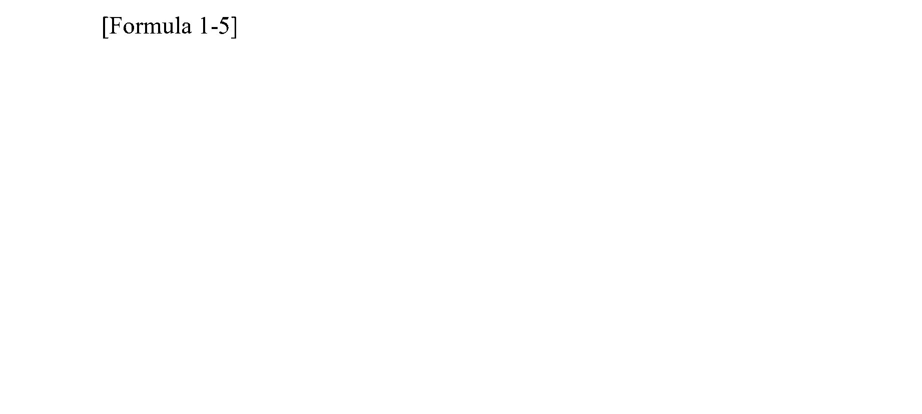 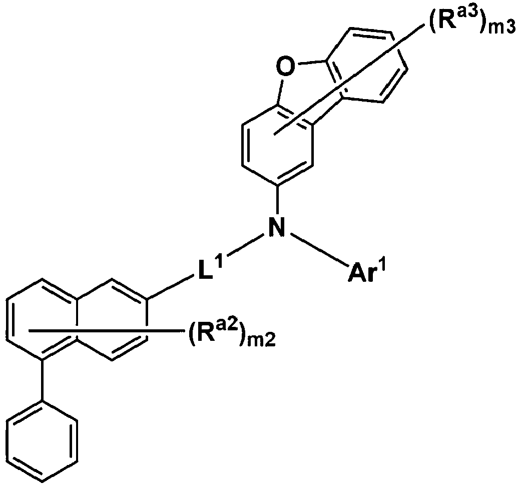 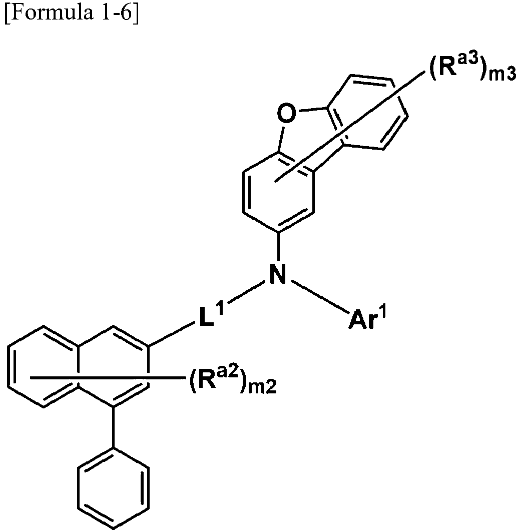 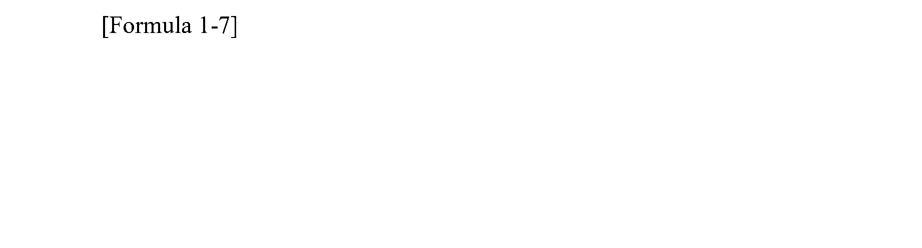 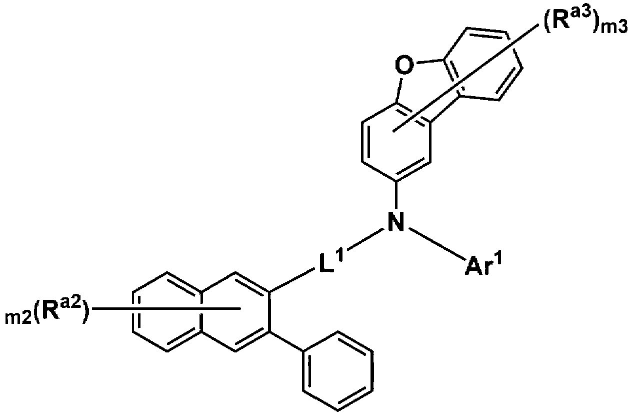 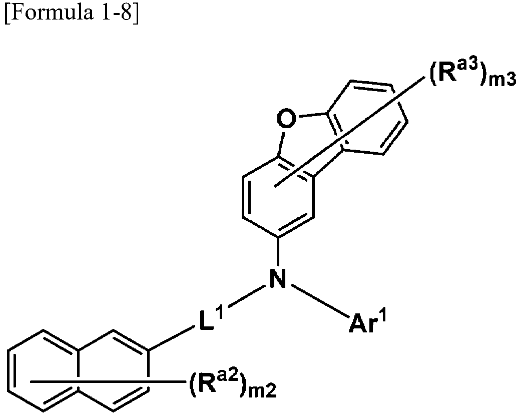
wherein in Formulas 1-1 to 1-8,
m2, m3, R^{a2}, R^{a3}, L¹, and Ar¹ are the same as defined in Formula 1.

3. The monoamine compound of claim 1 or claim 2, wherein Formula 2-1 is represented by Formula 2-1A or Formula 2-1B, and Formula 2-2 is represented by any one of Formulas 2-2A to 2-2C:

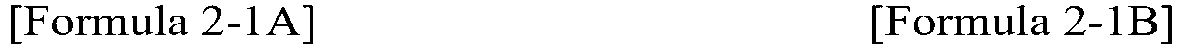

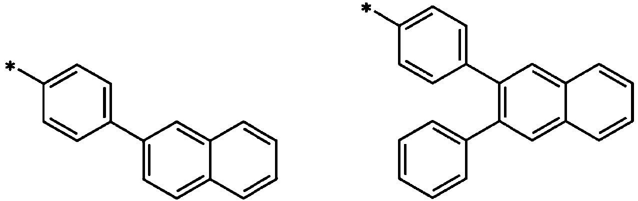

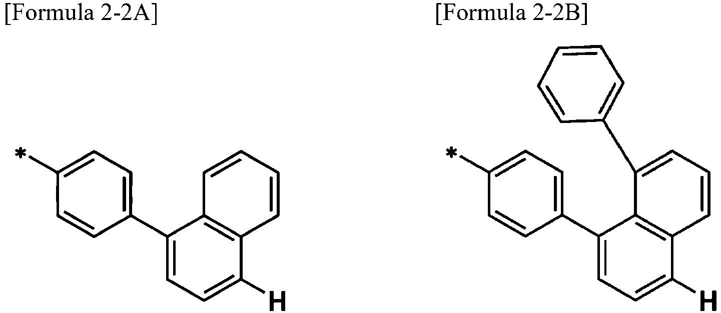

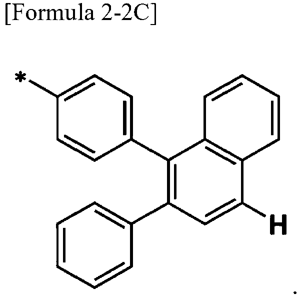

4. The monoamine compound of any one of claims 1 to 3, wherein Formula 2-3 is represented by any one of Formulas 2-3A to 2-3F, and Formula 2-4 is represented by any one of Formulas 2-4A to 2-4D:

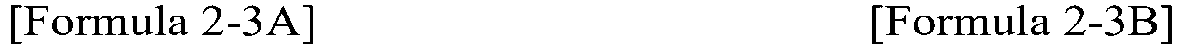

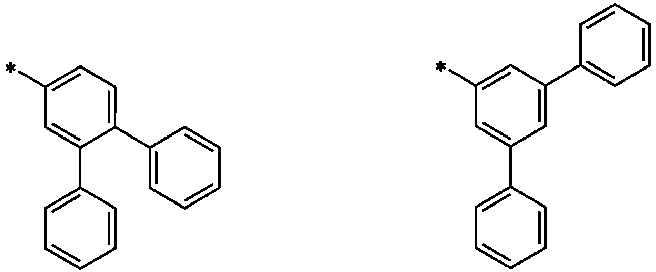

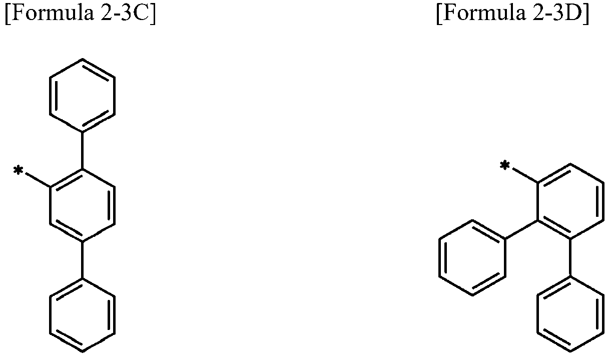

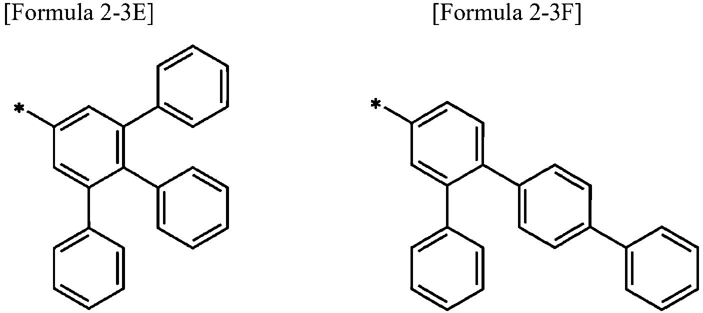

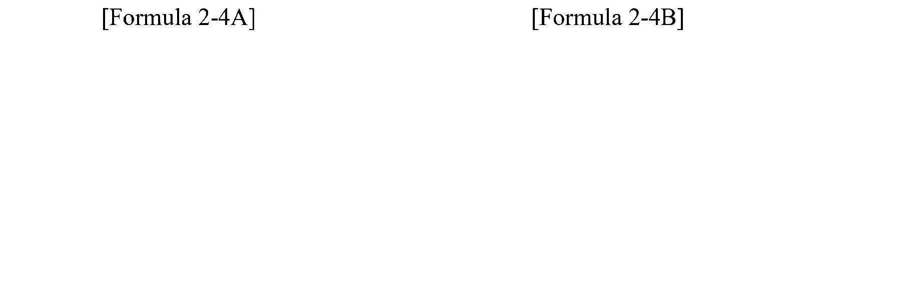

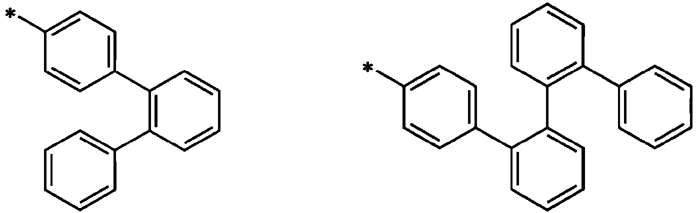

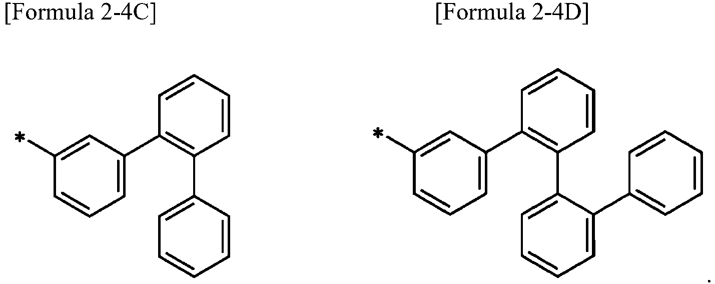

5. The monoamine compound of any one of claims 1 to 4, wherein in Formula 1, L¹ is a direct linkage, a substituted or unsubstituted phenylene group, a substituted or unsubstituted biphenylene group, a substituted or unsubstituted divalent dibenzofuran group, or a substituted or unsubstituted divalent carbazole group.

6. The monoamine compound of any one of claims 1 to 4, wherein in Formula 1, L¹ is a direct linkage or represented by any one of L1-1 to L1-5: 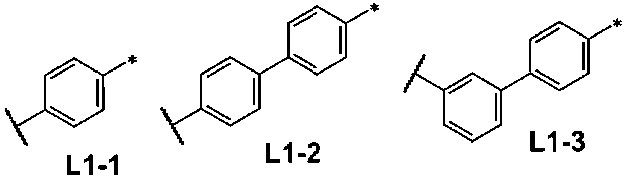 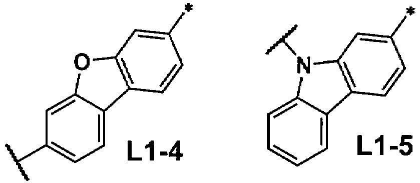
wherein in L1-1 to L1-5, 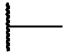
is a position bonded to a naphthalene group containing R^{a2} in Formula 1, and
-* is a position bonded to a nitrogen atom in Formula 1.

7. The monoamine compound of any one of claims 1 to 6, wherein in Formula 1, R^{a1} to R^{a3} are each independently a hydrogen atom, a deuterium atom, or a substituted or unsubstituted phenyl group.

8. The monoamine compound of claim 1, wherein the monoamine compound is represented by any one of compounds from Compound Group 1: 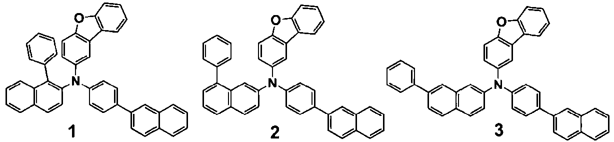 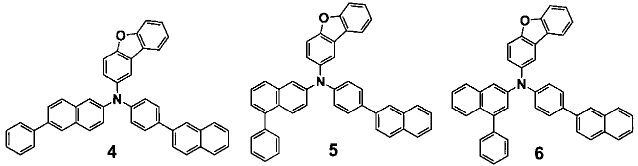 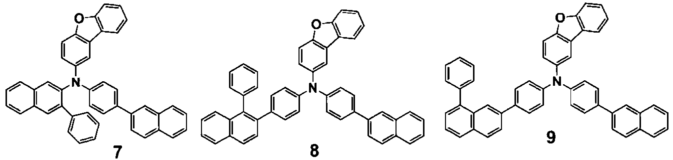 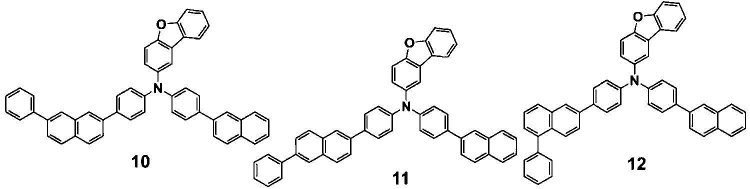 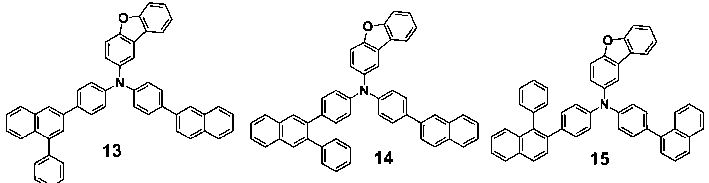 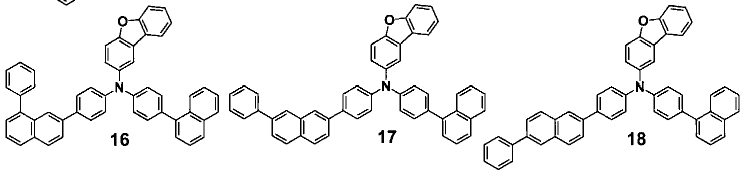 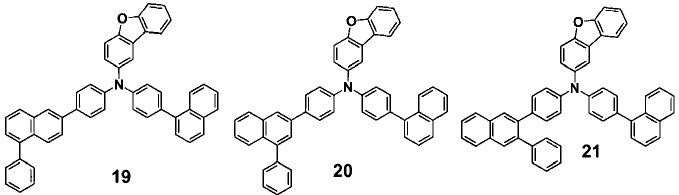 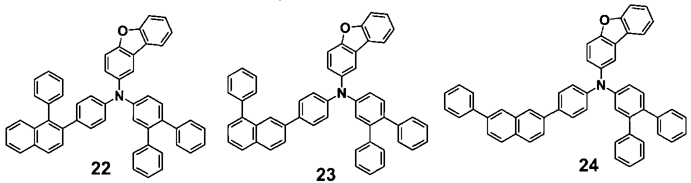 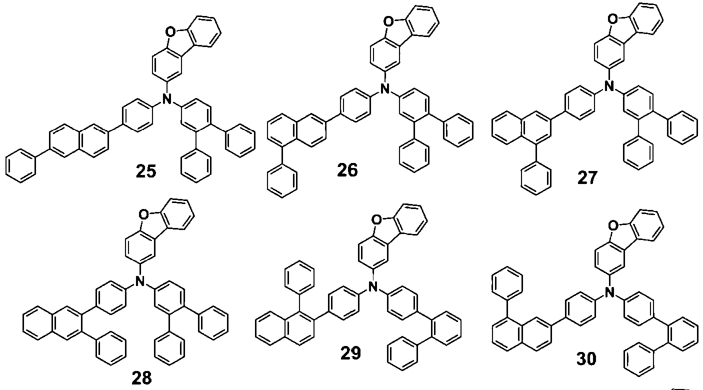 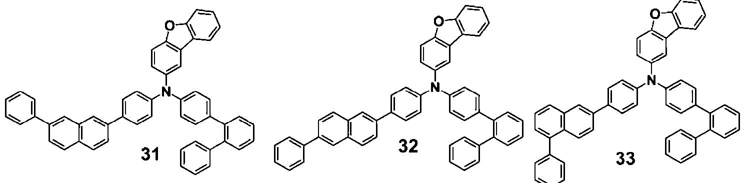 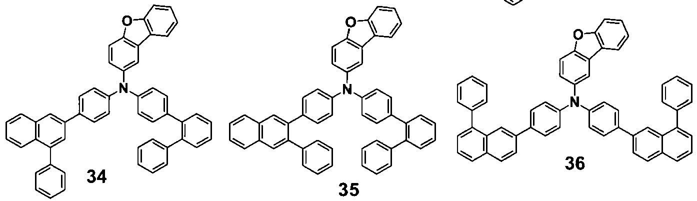 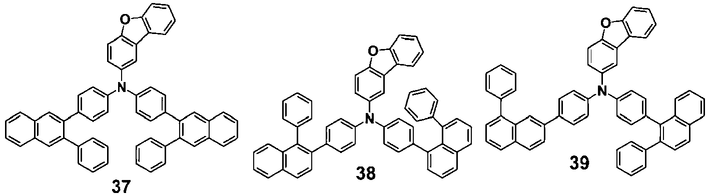 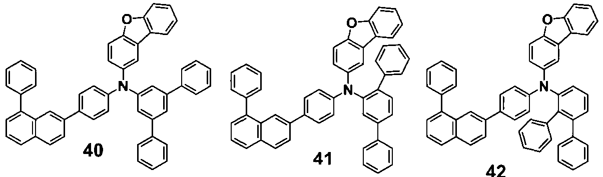 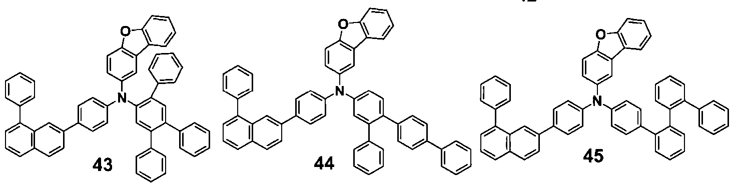 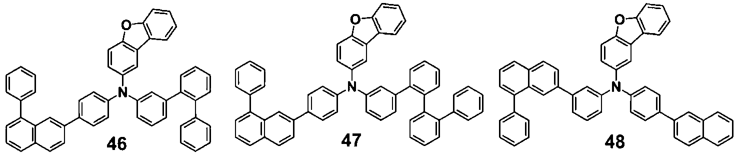 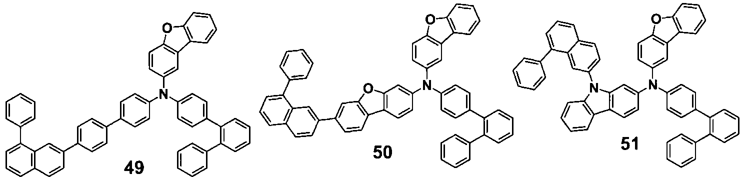 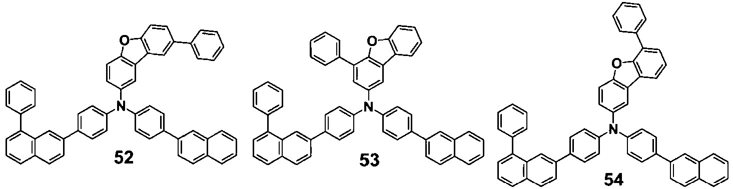 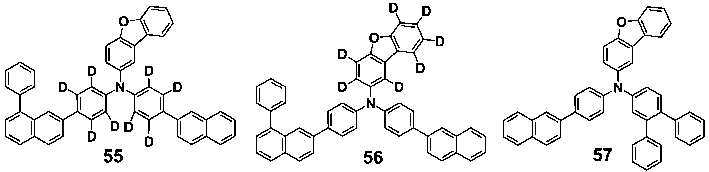 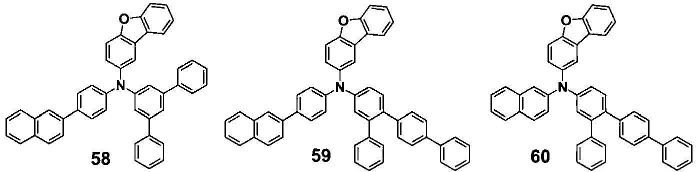 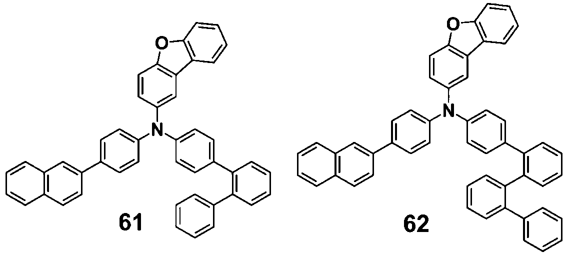 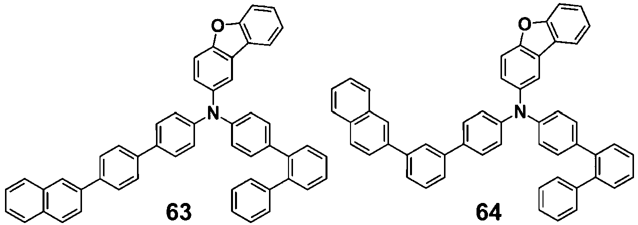 wherein in Compound Group 1, D is a deuterium atom.

9. A light emitting element (ED) comprising:
a first electrode (EL1);
a hole transport region (HTR) disposed on the first electrode (EL1);
an emission layer (EML) disposed on the hole transport region (HTR);
an electron transport region (ETR) disposed on the emission layer (EML); and
a second electrode (EL2) disposed on the electron transport region (ETR),
wherein the hole transport region (HTR) includes the monoamine compound according to any one of claims 1 to 8.

10. The light emitting element (ED) of claim 9, wherein the hole transport region (HTR) comprises a hole injection layer (HIL), a hole transport layer (HTL) disposed on the hole injection layer (HIL), and an electron blocking layer (EBL) disposed on the hole transport layer (HTL), and
at least one of the hole injection layer (HIL), the hole transport layer (HTL), or the electron blocking layer (EBL) comprises the monoamine compound.

11. An electronic apparatus (EA) providing images and comprising a display device (DD),
wherein the display device (DD) includes a base layer (BS), a circuit layer (DP-CL) disposed on the base layer (BS), and a display element layer (DP-ED) disposed on the circuit layer (DP-CL) and including a light emitting element (ED),
the light emitting element (ED) including:
a first electrode (EL1);
a hole transport region (HTR) disposed on the first electrode (EL1);
an emission layer (EML) disposed on the hole transport region (HTR);
an electron transport region (ETR) disposed on the emission layer (EML); and
a second electrode (EL2) disposed on the electron transport region (ETR),
wherein the hole transport region (HTR) includes the monoamine compound according to any one of claims 1 to 8.

12. The electronic apparatus (EA) of claim 11, further comprising at least one of a light control layer (CCL) or a color filter layer (CFL),
wherein the light control layer (CCL) includes quantum dots, and the color filter layer (CFL) includes pigment or dye.

13. The electronic apparatus (EA) of claim 11 or claim 12, wherein the display device (DD) comprises at least one of a television, a monitor, an outdoor billboard, a personal computer, a laptop, a personal digital terminal, an in-vehicle display device, a game console, a portable electronic device, or a camera.
